# FASCICULE DE BREVET EUROPEEN

(11) **EP 2 448 932 B1**
(45) Date de publication et mention de la délivrance du brevet: **05.03.2014**
(21) Numéro de dépôt: 10742015.0
(22) Date de dépôt: 01.07.2010
(51) Int. Cl.: C07D 403/06, C07D 413/06, A61K 31/513, A61P 35/00, A61K 31/5377

(54) **NOUVEAUX DERIVES DE 6-MORPHOLIN-4-YL-PYRIMIDIN-4-(3H)-ONE, LEUR PREPARATION PHARMACEUTIQUE COMME INHIBITEURS DE PHOSPHORYLATION D'AKT(PKB)**
NEUE 6-MORPHOLIN-4-YL-PYRIMIDIN-4-(3H)-ON-DERIVATE UND PHARMAZEUTISCHE ZUBEREITUNG DARAUS ALS AKT(PKB)-PHOSPHORYLIERUNGSHEMMER
NOVEL 6-MORPHOLIN-4-YL-PYRIMIDIN-4-(3H)-ONE DERIVATIVES, AND THE PHARMACEUTICAL PREPARATION THEREOF AS AKT(PKB) PHOSPHORYLATION INHIBITORS

(30) Priorité: 02.07.2009 FR 0903238; 10.09.2009 US 241101 P; 09.10.2009 FR 0957073
(43) Date de publication de la demande: 09.05.2012
(73) Titulaire: SANOFI, 75008 Paris (FR)
(72) Inventeur: CERTAL, Victor, F-75013 Paris (FR); FILOCHE-ROMMÉ, Bruno, F-75013 Paris (FR); HALLEY, Frank, F-75013 Paris (FR); KARLSSON, Karl Andreas, F-75013 Paris (FR); THOMPSON, Fabienne, F-75013 Paris (FR)
(74) Mandataire: Nony
(86) Numéro de dépôt international: PCT/FR2010/051376
(87) Numéro de publication internationale: WO 2011/001115

(56) Documents cités:
- WO-A1-03/024949
- WO-A1-2006/109081

## Description

La présente invention concerne de nouveaux composés chimiques 1H-pyrimidin-2-one, dérivés de pyrimidinones, leur procédé de préparation, les nouveaux intermédiaires obtenus, leur application à titre de médicaments, les compositions pharmaceutiques les renfermant et la nouvelle utilisation de tels dérivés.

La présente invention concerne ainsi également l'utilisation desdits dérivés pour la préparation d'un médicament destiné au traitement de l'homme. Plus particulièrement, l'invention concerne, de nouveaux dérivés de pyrimidinones et leur utilisation pharmaceutique pour la prévention et le traitement d'affections capables d'être modulées par l'inhibition de la voie PI3K/AKT/mTOR. AKT est un acteur clé dans cette voie de signalisation. Un niveau élevé de phosphorylation d'AKT est le marqueur de l'activation de la voie qui est retrouvée dans de nombreux cancers humains.

Les produits de la présente invention peuvent ainsi notamment être utilisés pour la prévention ou le traitement d'affections capables d'être modulées par l'inhibition de la phosphorylation d'AKT (P-AKT). L'inhibition de P-AKT peut être notamment obtenue par l'inhibition de la voie PI3K/AKT/mTOR, et en particulier par l'inhibition de kinases appartenant à cette voie comme les récepteurs à activité tyrosine kinase tels EGFR, IGFR, ErbB2, la 3'-phosphoinositide-dependent protein kinase-1 (PDK1), la phosphoinositide kinase PI3K, la serine-threonine kinase AKT, la kinase mTOR.

L'inhibition et la régulation de la voie PI3K/AKT/mTOR constitue notamment un nouveau et puissant mécanisme d'action pour le traitement d'un grand nombre de maladies cancéreuses incluant des tumeurs solides et liquides. De telles affections que peuvent traiter les produits de la présente demande sont les tumeurs humaines solides ou liquides.

### Rôle de la voie PI3K/AKT/mTOR

La voie de signalisation PI3K/AKT/mTOR est un réseau complexe qui régule de multiples fonctions cellulaires, comme la croissance, la survie, la prolifération et la motilité cellulaire, qui sont des processus clés de la tumorigénèse.

Cette voie de signalisation est une cible importante dans le traitement du cancer car la plupart de ses effecteurs sont altérés dans les tumeurs humaines. Les principaux effecteurs contribuant à l'activation de la voie sont i) les oncogènes tels que ErbB1 (EGFR), ErbB2 (HER2), PIK3CA et AKT activés par mutation, amplification ou surexpression ; ii) la déficience des gènes suppresseurs de tumeurs tels que PTEN, TSC1/2, LKB et PML qui sont inactivés suite à des mutations ou à des délétions (Jiang L-Z & Liu L-Z, Biochim Biophys Acta, 2008, 1784 :150 ; Vivanco I & Sawyers CL, 2002, Nat Rev Cancer, 2 :489 ; Cully M et al., Nature Rev. Cancer, 2006, 6 :184).

L'activation des oncogènes de cette voie de signalisation est retrouvée dans de nombreuses maladies cancéreuses humaines.
- les mutations activatrices de PIK3CA sont présentes dans 15-30% des cancers du colon, du sein, de l'endomètre, du foie, de l'ovaire et de la prostate (TL Yuan and LC Cantley, Oncogene, 2008, 27:5497; Y. Samuels et al. Science, 2004, 304:554; KE. Bachman et al. Cancer Biol Ther, 2004, 3:772; DA Levine et al. Clin Canc Res. 2005, 11:2875; C. Hartmann et al. Acta Neuropathol. 2005, 109:639).
- les amplifications, mutations activatrices et surexpressions des RTKs tels qu'EGFR et HER2 dans les cancers du cerveau, du sein et du poumon (NSCLC)
- l'amplification et la surexpression activatrice d'AKT dans les cancers du cerveau, du poumon (NSCLC), du sein, du rein, de l'ovaire et du pancréas (Testa JR. and Bellacosa A., Proct. Natl. Acad. Sci. USA 2001, 98:10983 ; Cheng et al., Proct. Natl. Acad. Sci. USA 1992, 89: 9267 ; Bellacosa et al., Int. J. Cancer, 1995, 64:280 ; Cheng et al., Proct. Natl. Acad. Sci. USA 1996, 93 :3636 ; Yuan et al., Oncogene, 2000, 19 :2324).

La déficience des gènes suppresseurs de tumeurs de cette voie de signalisation est également retouvée dans de nombreuses maladies cancéreuses humaines:
○ la délétion de *PTEN* dans 50% des cancers du poumon (NSCLC), du foie, du rein, de la prostate, du sein, du cerveau, du pancréas, de l'endomètre et du colon (Maxwell GL et al. Canc. Res. 1998, 58 :2500 ; Zhou X-P et al. Amer. J. Pathol., 2002, 161 :439 ; Endersby R & Baker SJ, Oncogene, 2008, 27 :5416 ; Li et al. Science, 1997, 275:1943; Steack PA et al., Nat. Genet., 1997, 15 :356)
○ les mutations de *TSC1*/*2* dans plus de 50% des scléroses tubéreuses
○ les mutations ou délétions de *LKB1* (or *STK11)* qui prédisposent aux cancers du tractus gastro-intestinal et au cancer du pancreas et qui sont retouvées en particulier dans 10-38% des adenocarcinomes du poumon (Shah U. et al. Cancer Res. 2008, 68 :3562)
○ les modification de *PML* notament par translocation dans les tumeurs humaines (Gurrieri C et al, J. NAtI Cancer Inst. 2004, 96 :269).

De plus cette voie de signalisation est un facteur majeur de résistance à la chimiothérapie, la radiothérapie et à des thérapies ciblées tels que les inhibiteurs d'EGFR et HER2 par exemple (C. Sawyers et al. Nat Rev 2002).

### Role d'AKT

AKT (protéine kinase B ; PKB) est une sérine-thréonine kinase qui occupe une place centrale dans une des voies majeures de signalisation cellulaire, la voie PI3K/AKT. AKT est notamment impliquée dans la croissance, la prolifération et la survie des cellules tumorales. L'activation d'AKT se fait en deux étapes (i) par phosphorylation de la thréonine 308 (P-T308) par PDK1 et (2) par phosphorylation de la sérine 473 (P-S473) par mTORC2 (ou complexe mTOR-Rictor), résultant en une activation totale. AKT à son tour régule un grand nombre de protéines dont mTOR (mammalian target of Rapamycin), BAD, GSK3, p21, p27, FOXO ou FKHRL1 (Manning BD & Cantley LC, Cell, 2007 129 :1261). L'activation d'AKT promeut l'internalisation des nutriments, ce qui déclenche un processus de métabolisation anabolisante soutenant la croissance et la prolifération cellulaire. En particulier, AKT contrôle l'initiation de la synthèse protéique à travers une cascade d'interactions qui procède par l'intermédiaire de TSC1/2 (complexe de sclérose tubéreuse), Rheb, et TOR pour aboutir à deux cibles critiques de la voie de signalisation, p70S6K et 4EBP. AKT induit également une phosphorylation inhibitrice du facteur de transcription Forkhead et l'inactivation de GSK3β qui conduisent à l'inhibition de l'apoptose et à la progression du cycle cellulaire (Franke TF, Oncogene, 2008, 27 :6473). AKT est donc une cible pour la thérapie anti-cancéreuse et l'inhibition de l'activation d'AKT par l'inhibition de sa phosphorylation peut induire l'apoptose des cellules malignes et par la même, présenter un traitement pour le cancer.

### Les récepteurs à activité tyrosyne kinase comme IGF1R

Des niveaux anormalement élevés d'activité protéine kinase ont été impliqués dans de nombreuses maladies résultant de fonctions cellulaires anormales. Ceci peut provenir soit directement soit indirectement, d'un disfonctionnement dans les mécanismes de contrôle de l'activité kinase, lié par exemple à une mutation, une sur-expression ou une activation inappropriée de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc). Le récepteur activé par son ligand induit une activité mitogènique dans les cellules normales. Cependant IGF-I-R joue un rôle important dans la croissance dite anormale.

Plusieurs rapports cliniques soulignent le rôle important de la voie IGF-I dans le développement des cancers humains :
IGF-I-R est souvent trouvé sur-exprimé dans de nombreux types tumoraux (sein, colon, poumon, sarcome, prostate, myelome multiple) et sa présence est souvent associée à un phénotype plus agressif.

De fortes concentrations d'IGF1 circulant sont fortement corrélées à un risque de cancer de la prostate, poumon et sein.

De plus, il a été largement documenté que IGF-I-R est nécessaire à l'établissement et au maintient du phénotype transformé in vitro comme in vivo [Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie, radiation, et l'apoptose induite par des cytokines. De plus, l'inhibition d'IGF-I-R endogène par un dominant négatif, la formation de triple hélice ou l'expression d'un antisens provoque une suppression de l'activité transformante in vitro et la diminution de la croissance de tumeurs dans les modèles animaux.

### PDK1

La 3'-phosphoinositide-dependent protein kinase-1 (PDK1) est une des composantes essentielles de la voie de signalisation PI3K-AKT. C'est une sérine-thréonine (Ser/Thr) kinase dont le rôle est de phosphoryler et d'activer d'autres Ser/Thr kinases de la famille des AGC impliquées dans le contrôle de la croissance, la prolifération, la survie cellulaire et dans la régulation du métabolisme. Ces kinases incluent la protéine kinase B (PKB ou AKT), SGK (ou sérum and glucocorticoïd regulated kinase), RSK (ou p90 ribosomal S6 kinase), p70S6K (ou p70 ribosomal S6 kinase) ainsi que diverses isoformes de la protéine kinase C (PKC) (Vanhaesebroeck B. & Alessi DR., Biochem J, 2000, 346:561). Un des rôles clés de PDK1 est donc l'activation d'AKT : en présence de PIP3, le second messager généré par PI3K, PDK-1 est recruté à la membrane plasmique via son domaine PH (plekstrin homology) et phosphoryle AKT sur la thréonine 308 situé dans la boucle d'activation, une modification essentielle de l'activation d'AKT. PDK1 est exprimée de façon ubiquitaire et est une kinase constitutivement active. PDK1 est un élément clé dans la voie de signalisation PI3K/AKT pour la régulation de processus clés dans la tumorigénèse comme la prolifération et la survie cellulaire. Cette voie étant activée dans plus de 50% des cancers humains, PDK1 représente une cible pour la thérapie anticancéreuse. L'inhibition de PDK1 devrait résulter en une inhibition effective de la proliferation et de la survie des cellules cancéreuses et donc apporter un bénéfice thérapeutique pour les cancers humains (Bayascas JR, Cell cycle, 2008, 7 :2978 ; Peifer C. & Alessi DR, ChemMedChem, 2008, 3 :1810).

### Les phosphoinositides-3 kinases (PI3Ks)

La lipide kinase PI3K est une cible importante dans cette voie de signalisation pour l'oncologie. Les PI3Ks de la classe I sont réparties en classe Ia (PI3Kα,β,δ) activée par les récepteurs à activité tyrosine kinase (RTKs), les récepteurs couplés aux protéines G (GPCRs), les GTPases de la famille Rho, p21-Ras et en classe Ib (PI3Kγ) activé par les GPCRs et par p21-Ras. Les PI3Ks de la classe Ia sont des hétérodimères qui consistent en une sous unité catalytique p110α, β ou δ et une sous unité régulatrice p85 ou p55. La classe Ib (p110γ) est monomérique. Les PI3Ks de la classe I sont des lipides/protéines kinases qui sont activées par les RTKs, les GPCRs ou Ras après recrutement à la membrane. Ces PI3Ks de la classe I phosphorylent le phosphatidylinositol 4,5 diphosphate (PIP2) sur la position 3 de l'inositol pour donner le phosphatidylinositol 3,4,5 triphosphate (PIP3), messager secondaire clé de cette voie de signalisation. A son tour, PIP3 recrute AKT et PDK1 à la membrane où ils se fixent par leur domaine homologue à la pleckstrine (domaine PH), conduisant à l'activation d'AKT par phosphorylation de PDK1 sur la thréonine 308. AKT phosphoryle de nombreux substrats, jouant ainsi un rôle clé dans de nombreux processus aboutissant à la transformation cellulaire comme la prolifération, la croissance et la survie cellulaire ainsi que l'angiogénèse.

Les PI3Ks de classe I sont impliquées dans les cancers humains : des mutations somatiques du gène PIK3CA qui code pour PI3Kα se retrouvent dans 15-35% des tumeurs humaines avec notamment deux mutations oncogéniques principales H1047R (dans le domaine kinase) et E545K/E542K (dans le domaine hélical) (Y. Samuels et al. Science, 2004, 304:554; TL Yuan and LC Cantley, Oncogene, 2008, 27:5497). Des inhibiteurs de PI3K sont attendus efficaces pour le traitement de nombreux cancers humains présentant des altérations génétiques aboutissant à l'activation de la voie PI3K/AKT/mTOR (Vogt P. et al., Virology, 2006, 344 :131 ; Zhao L & Vogt PK, Oncogene, 2008, 27 :5486).

Des dérivés Morpholino pyrimidinones inhibiteurs de kinases sont connus de l'homme de l'art.

L'application WO2008/148074 décrit des produits qui possèdent une activité inhibitrice de mTOR. Ces produits sont des pyrido[1,2-a]pyrimidin-4-ones qui diffèrent des produits de la présente invention en raison de leur caractère entièrement aromatique et de leurs substitutions.

L'application WO2008/064244 décrit l'application des produits TGX-221 et TGX-155 inhibiteurs de PI3Kβ utiles dans le traitement du cancer et notamment dans le cancer du sein. Ces produits sont des pyrido[1,2-a]pyrimidin-4-ones précédemment décrits dans les applications WO2004/016607 et WO2001/053266 qui diffèrent des produits de la présente invention en raison de leur caractère entièrement aromatique et de leurs substitutions.

Les applications WO2006/109081, WO2006/109084 et WO2006/126010 décrivent des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

L'application WO2003/024949 décrit des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

Des dérivés morpholino pyrimidine inhibiteurs de kinase sont également connus de l'homme de l'art.

Les applications WO2009/007748, WO2009/007749, WO2009/007750 et WO2009/007751 décrivent des produits qui possèdent une activité inhibitrice de mTOR et/ou de PI3K pour le traitement des cancers. Ces produits sont des pyrimidines substituées en 2, 4 et 6 et les produits de la présente invention en différent en raison de la présence du groupement carbonyle sur la pyrimidinone ainsi que par les différent substituants
La présente invention a pour objet les produits de formule (I): 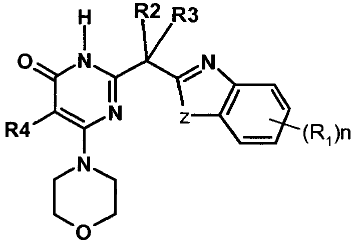 dans laquelle :
Z représente -O-, -NH, Ncycloalkyle, Nalk, ou N-phényl, avec alk et phényle éventuellement substitués par un ou plusieurs radicaux choisis parmi les atomes d'halogène, et les radicaux alkyle, alcoxy, hydroxyle, cyano et phényle lui-même éventuellement substitué par un ou plusieurs radicaux choisis parmi les atomes d'halogène et les radicaux hydroxyle, alcoxy et alkyle ;
n représente un entier de 0 à 4 ;
R1 représente un atome d'hydrogène, d'halogène ou un radical hydroxyle, alkyle ou alcoxy ; les radicaux alkyle et alcoxy étant éventuellement substitués par un groupe NRxRy ou par un ou plusieurs atomes d'halogène ;
étant entendu que R1 peut représenter un reste phényle fusionné avec le phényl qui porte R1 pour former un naphtyle;
R2 et R3 identiques ou différents représentent un atome d'hydrogène, un atome d'halogène ou un radical alkyle éventuellement substitué par un ou plusieurs atomes d'halogène ;
R4 représente un atome d'hydrogène;
NRxRy étant tel que Rx représente un atome d'hydrogène ou un radical alkyle et Ry représente un atome d'hydrogène ou un radical alkyle; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué par un ou plusieurs radicaux identiques ou différents choisis parmi les atomes d'halogène et les radicaux alkyle, hydroxyle, oxo, alcoxy, NH2; NHalk et N(alk)2 ;
tous les radicaux alkyle (alk) et alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

La présente invention a ainsi pour objet les produits de formule (I) tels que définis ci-dessus dans laquelle :
Z représente -O-, -NH, Ncycloalkyle, Nalk ou N-phényl, avec alk éventuellement substitué par un ou plusieurs radicaux choisis parmi alcoxy, hydroxyle, cyano et phényle;
n représente un entier de 0 à 4 ;
R1 représente un atome d'hydrogène, d'halogène ou un radical alkyle ou alcoxy ; les radicaux alkyle et alcoxy étant éventuellement substitués par un groupe NRxRy ou par un ou plusieurs atomes d'halogène ;
étant entendu que R1 peut représenter un reste phényle fusionné avec le phényl qui porte R1 pour former un radical naphtyle;
R2 et R3 représentent un atome d'hydrogène ;
R4 représente un atome d'hydrogène;
NRxRy étant tel que Rx représente un atome d'hydrogène ou un radical alkyle et Ry représente un atome d'hydrogène ou un radical alkyle; soit soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
tous les radicaux alkyle (alk) et alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

La présente invention a pour objet les produits de formule (I): 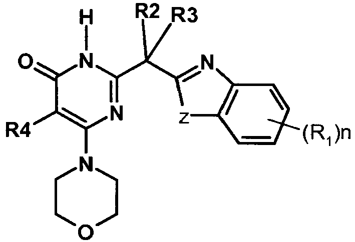 dans laquelle :
Z représente -O-, -NH, Nalk ou N-phényl éventuellement substitué par un ou plusieurs radicaux choisis parmi les atomes d'halogène, et les radicaux alkyle, alcoxy et hydroxyle;
n représente un entier de 0 à 4 ;
R1 représente un atome d'hydrogène, d'halogène ou un radical hydroxyle, alkyle ou alcoxy ; les radicaux alkyle et alcoxy étant éventuellement substitués par un groupe NRxRy ;
R2 et R3 identiques ou différents représentent un atome d'hydrogène, un atome d'halogène ou un radical alkyle éventuellement substitué par un ou plusieurs atomes d'halogène ;
R4 représente un atome d'hydrogène;
NRxRy étant tel que Rx représente un atome d'hydrogène ou un radical alkyle et Ry représente un atome d'hydrogène ou un radical alkyle; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué par un ou plusieurs radicaux identiques ou différents choisis parmi les atomes d'halogène et les radicaux alkyle, hydroxyle, oxo, alcoxy, NH2; NHalk et N(alk)2 ;
tous les radicaux alkyle (alk) et alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

La présente invention a pour objet les produits de formule (I):tels que définis ci-dessus 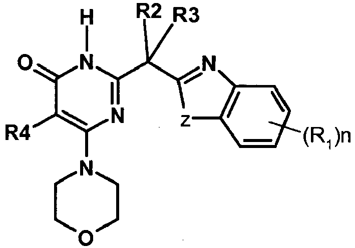 dans laquelle :
Z représente -O-, -NH ou Nalk,
n représente un entier de 0 à 4 ;
R1 représente un atome d'halogène ou un radical hydroxyle, alkyle ou alcoxy ; les radicaux alkyle et alcoxy étant éventuellement substitués par un groupe NRxRy ;
R2 et R3 identiques ou différents représentent un atome d'hydrogène, un atome d'halogène ou un radical alkyle éventuellement substitué par un ou plusieurs atomes d'halogène ;
R4 représente un atome d'hydrogène;
NRxRy étant tel que Rx représente un atome d'hydrogène ou un radical alkyle et Ry représente un atome d'hydrogène ou un radical alkyle; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué par un ou plusieurs radicaux identiques ou différents choisis parmi les atomes d'halogène et les radicaux alkyle, hydroxyle, oxo, alcoxy, NH2; NHalk et N(alk)2 ;
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) :
- le terme radical alkyle (ou alk) désigne les radicaux, linéaires et le cas échéant ramifiés, méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : on préfère les radicaux alkyles renfermant de 1 à 6 atomes de carbone et plus particulièrement les radicaux alkyle renfermant de 1 à 4 atomes de carbone de la liste ci-dessus ;
- le terme radical alcoxy désigne les radicaux linéaires et le cas échéant ramifiés, méthoxy, éthoxy, propoxy, isopropoxy, butoxy linéaire, secondaire ou tertiaire, pentoxy ou hexoxy ainsi que leurs isomères de position linéaires ou ramifiés : on préfère les radicaux alkoxy renfermant de 1 à 4 atomes de carbone de la liste ci-dessus ;
- le terme radical alkylthio désigne les radicaux linéaires et le cas échéant ramifiés, méthylthio, éthylthio, propylthio, isopropylthio, butylthio linéaire, secondaire ou tertiaire, pentylthio ou hexylthio ainsi que leurs isomères de position linéaires ou ramifiés : on préfère les radicaux alkylthio renfermant de 1 à 4 atomes de carbone de la liste ci-dessus ;
- le terme atome d'halogène désigne les atomes de chlore, de brome, d'iode ou de fluor et de préférence l'atome de chlore, de brome ou de fluor.
- le terme radical cycloalkyle désigne un radical carbocyclique saturé renfermant 3 à 10 atomes de carbone et désigne ainsi notamment les radicaux cyclopropylee, cyclobutyle, cyclopentyle et cyclohexyle et tout particulièrement les radicaux cyclopropyle, cyclopentyle et cyclohexyle ;
- dans le radical -O-cycloalkyle, cycloalkyle est tel que défini ci-dessus ;
- le terme radical hétérocycloalkyle désigne ainsi un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre : on peut citer par exemple les radicaux morpholinyle, thiomorpholinyle, homomorpholinyle, aziridyle, azétidyle, pipérazinyle, pipéridyle, homopipérazinyle, pyrrolidinyle, imidazolidinyle, pyrazolidinyle, tétrahydrofuryle, tétrahydrothiényle, tétrahydropyranne, oxodihydropyridazinyle, ou encore oxétanyle tous ces radicaux étant éventuellement substitués ; on peut citer notamment les radicaux morpholinyle, thiomorpholinyle, homomorpholinyle, pipérazinyle, pipéridyle, homopipérazinyle ou encore pyrrolidinyle,
- les termes aryle et hétéroaryle désignent des radicaux insaturés ou partiellement insaturés, respectivement carbocycliques et hétérocycliques, monocycliques ou bicycliques, renfermant au plus 12 chaînons, pouvant éventuellement contenir un chaînon -C(O), les radicaux hétérocycliques contenant un ou plusieurs hétéroatomes identiques ou différents choisis parmi O, N, ou S avec N, le cas échéant, éventuellement substitué ;
- le terme radical aryle désigne ainsi des radicaux monocycliques ou bicycliques renfermant 6 à 12 chaînons tels que par exemple les radicaux phényle, naphtyle, biphényle, indényle, fluorényle et anthracényle, plus particulièrement les radicaux phényle et naphtyle et encore plus particulièrement le radical phényle. On peut noter qu'un radical carbocyclique contenant un chaînon -C(O) est par exemple le radical tétralone ;
- le terme radical hétéroaryle désigne ainsi des radicaux monocycliques ou bicycliques renfermant 5 à 12 chaînons : des radicaux hétéroaryles monocycliques tels que par exemple les radicaux thiényle tel que 2-thiényle et 3-thiényle, furyle tel que 2-furyle, 3-furyle, pyrannyle, pyrrolyle, pyrrolinyle, pyrazolinyle, imidazolyle, pyrazolyle, pyridyle tel que 2-pyridyle, 3-pyridyle et 4-pyridyle, pyrazinyle, pyrimidinyle, pyridazinyle, oxazolyle, thiazolyle, isothiazolyle, diazolyle, thiadiazolyle, thiatriazolyle, oxadiazolyle, isoxazolyle tel que 3- ou 4-isoxazolyle, furazannyle, tétrazolyle libre ou salifié, tous ces radicaux étant éventuellement substitués parmi lesquels plus particulièrement les radicaux thiényle tel que 2-thiényle et 3-thiényle, furyle tel que 2-furyle, pyrrolyle, pyrrolinyle, pyrazolinyle, imidazolyle, pyrazolyle, oxazolyle, isoxazolyle, pyridyle, pyridazinyle, ces radicaux étant éventuellement substitués ; des radicaux hétéroaryles bicycliques tels que par exemple les radicaux benzothiényle tel que 3-benzothiényle, benzothiazolyle, quinolyle, isoquinolyle, dihydroquinolyle, quinolone, tétralone, adamentyl, benzofuryle, isobenzofuryle, dihydrobenzofuranne, éthylènedioxyphényle, thianthrényle, benzopyrrolyle, benzimidazolyle, benzoxazolyle, thionaphtyle, indolyle, azaindolyle, indazolyle, purinyle, thiénopyrazolyle, tétrahydroindazolyle, tétrahydrocyclopentapyrazolyle, dihydrofuropyrazolyle, tétrahydropyrrolopyrazolyle, oxotétrahydropyrrolopyrazolyle, tétrahydropyranopyrazolyle, tétrahydropyridinopyrazolyle ou oxodihydropyridino-pyrazolyle, tous ces radicaux étant éventuellement substitués ;

Comme exemples de radicaux hétéroaryles ou bicycliques, on peut citer plus particulièrement les radicaux pyrimidinyle, pyridyle, pyrrolyle, azaindolyle, indazolyle ou pyrazolyle, benzothiazolyle ou benzimidazolyle éventuellement substitués par un ou plusieurs substituants identiques ou différents comme indiqué ci-dessus.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a pour objet les produits de formule (I) tels que définis ci-dessus dans laquelle ::
Z représente -O-, -NH, Nalk ou N-phényl ;
n représente un entier de 0 à 4 ;
R1 représente un atome d'hydrogène, d'halogène ou un radical alkyle ou alcoxy ;
R2 et R3 représentent un atome d'hydrogène ;
R4 représente un atome d'hydrogène;
NRxRy étant tel que Rx représente un atome d'hydrogène ou un radical alkyle et Ry représente un atome d'hydrogène ou un radical alkyle; soit soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
tous les radicaux alkyle (alk) et alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

La présente invention a pour objet les produits de formule (I) tels que définis ci-dessus dans laquelle :
Z représente -O-, -NH ou Nalk ;
n représente un entier de 0 à 4 ;
R1 représente un atome d'halogène ou un radical alkyle ou alcoxy ;
R2 et R3 représentent un atome d'hydrogène ;
R4 représente un atome d'hydrogène;
NRxRy étant tel que Rx représente un atome d'hydrogène ou un radical alkyle et Ry représente un atome d'hydrogène ou un radical alkyle; soit soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
tous les radicaux alkyle (alk) et alcoxy ci-dessus étant linéaires ou ramifiés et renfermant de 1 à 6 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Notamment, lorsque NRxRy forme un cycle comme défini ci-dessus, un tel cycle aminé peut être choisi notamment parmi les radicaux pyrrolidinyle, pyrazolidinyle, pyrazolinyle, pipéridyle, azépinyle, morpholinyle, homomorpholinyle, pipérazinyle ou homopipérazinyle, ces radicaux étant eux-mêmes éventuellement substitués comme indiqué ci-dessus ou ci-après.

Le cycle NRxRy peut plus particulièrement être choisi parmi les radicaux pyrrolidinyle, morpholinyle éventuellement substitué par un ou deux radicaux alkyle ou pipérazinyle éventuellement substitué sur le second atome d'azote par un radical alkyle, phényle, ou et CH2-phényle, eux-mêmes éventuellement substitués par un ou plusieurs radicaux identiques ou différents choisis parmi les atomes d'halogène et les radicaux alkyle, hydroxyle et alcoxy.

La présente invention a ainsi tout articulièrement pour objet les produits de formule (I) tels que définis ci-dessus répondnat aux formules suivantes :
- 2-[(5-fluoro-1H-benzimidazol-2-yl)méthyl]-6-(morpholin-4-yl)pyrimidin-4(3H)-one
- 2-(1,3-benzoxazol-2-ylméthyl)-6-(morpholin-4-yl)pyrimidin-4(3H)-one
- 2-[(6-bromo-1H-benzimidazol-2-yl)méthyl]-6-(morpholin-4-yl)pyrimidin-4(3H)-one
- 2-[(6-méthoxy-1H-benzimidazol-2-yl)méthyl]-6-(morpholin-4-yl)pyrimidin-4(3H)-one
- 2-[(5,6-difluoro-1H-benzimidazol-2-yl)méthyl]-6-(morpholin-4-yl)pyrimidin-4(3H)-one
- 2-[(6-fluoro-1,3-benzoxazol-2-yl)méthyl]-6-(morpholin-4-yl)pyrimidin-4(3H)-one
- 2-[(5-fluoro-1,3-benzoxazol-2-yl)méthyl]-6-(morpholin-4-yl)pyrimidin-4(3H)-one
- 2-[(6-méthyl-1,3-benzoxazol-2-yl)méthyl]-6-(morpholin-4-yl)pyrimidin-4(3H)-one
- 2-[(6-bromo-1,3-benzoxazol-2-yl)méthyl]-6-(morpholin-4-yl)pyrimidin-4(3H)-one
- 2-[(1-méthyl-1H-benzimidazol-2-yl)méthyl]-6-(morpholin-4-yl)pyrimidin-4(3H)-one
- 2-[(5-fluoro-1-méthyl-1H-benzimidazol-2-yl)méthyl]-6-(morpholin-4-yl)pyrimidin-4(3H)-one
- 2-[(7-fluoro-1,3-benzoxazol-2-yl)méthyl]-6-(morpholin-4-yl)pyrimidin-4(3H)-one
- 6-(morpholin-4-yl)-2-[(1-phényl-1H-benzimidazol-2-yl)méthyl]pyrimidin-4(3H)-one
- 2-[(1-benzyl-1H-benzimidazol-2-yl)méthyl]-6-(morpholin-4-yl)pyrimidin-4(3H)-one
- 2-[(1-éthyl-1H-benzimidazol-2-yl)méthyl]-6-(morpholin-4-yl)pyrimidin-4(3H)-one
- 2-[(5,6-difluoro-1,3-benzoxazol-2-yl)méthyl]-6-(morpholin-4-yl)pyrimidin-4(3H)-one
- 2-[(6-chloro-1H-benzimidazol-2-yl)méthyl]-6-(morpholin-4-yl)pyrimidin-4(3H)-one
- 2-[(1-cyclopropyl-5-fluoro-1H-benzimidazol-2-yl)méthyl]-6-(morpholin-4-yl)pyrimidin-4(3H)-one
- 2-{[5-fluoro-1-(2-méthoxyéthyl)-1H-benzimidazol-2-yl]méthyl}-6-(morpholin-4-yl)pyrimidin-4(3H)-one
- 6-(morpholin-4-yl)-2-(naphtho[2,1-d][1,3]oxazol-2-ylmethyl)pyrimidin-4(3H)-one
- 2-[(6-methoxy-1,3-benzoxazol-2-yl)methyl]-6-(morpholin-4-yl)pyrimidin-4(3H)-one
- 2-[(6,7-difluoro-1,3-benzoxazol-2-yl)methyl]-6-(morpholin-4-yl)pyrimidin-4(3H)-one
- 2-[(5,6-dichloro-1 H-benzimidazol-2-yl)methyl]-6-(morpholin-4-yl)pyrimidin-4(3H)-one
- 2-[(6-methyl-1 H-benzimidazol-2-yl)methyl]-6-(morpholin-4-yl)pyrimidin-4(3H)-one
- 2-{[4-chloro-6-(trifluoromethyl)-1H-benzimidazol-2-yl]methyl}-6-(morpholin-4-yl)pyrimidin-4(3H)-one
- 6-(morpholin-4-yl)-2-{[6-(trifluoromethyl)-1H-benzimidazol-2-yl]methyl}pyrimidin-4(3H)-one
- 2-[(5-chloro-6-fluoro-1 H-benzimidazol-2-yl)methyl]-6-(morpholin-4-yl)pyrimidin-4(3H)-one
- 2-[(6,7-difluoro-1 H-benzimidazol-2-yl)methyl]-6-(morpholin-4-yl)pyrimidin-4(3H)-one
- 2-[(5-chloro-6-methyl-1H-benzimidazol-2-yl)methyl]-6-(morpholin-4-yl)pyrimidin-4(3H)-one
- (2-{[4-(morpholin-4-yl)-6-oxo-1,6-dihydropyrimidin-2-yl]methyl}-1H-benzimidazol-1-yl)acetonitrile
- 2-[(5,7-difluoro-1 H-benzimidazol-2-yl)methyl]-6-(morpholin-4-yl)pyrimidin-4(3H)-one
- 2-{[5-chloro-1-(1-phenylethyl)-1H-benzimidazol-2-yl]methyl}-6-(morpholin-4-yl)pyrimidin-4(3H)-one
- 2-[(1-cyclohexyl-1H-benzimidazol-2-yl)methyl]-6-(morpholin-4-yl)pyrimidin-4(3H)-one
- 6-(morpholin-4-yl)-2-[(5,6,7-trifluoro-1H-benzimidazol-2-yl)methyl]pyrimidin-4(3H)-one
- 2-[(4-bromo-6-fluoro-1 H-benzimidazol-2-yl)methyl]-6-(morpholin-4-yl)pyrimidin-4(3H)-one
- 2-[(6-bromo-1-methyl-1 H-benzimidazol-2-yl)methyl]-6-(morpholin-4-yl)pyrimidin-4(3H)-one
- 2-[(6,7-difluoro-1-methyl-1H-benzimidazol-2-yl)methyl]-6-(morpholin-4-yl)pyrimidin-4(3H)-one
- 2-[(5,6-dichloro-1-methyl-1 H-benzimidazol-2-yl)methyl]-6-(morpholin-4-yl)pyrimidin-4(3H)-one
- 6-(morpholin-4-yl)-2-[(5,6,7-trifluoro-1-methyl-1H-benzimidazol-2-yl)methyl]pyrimidin-4(3H)-one
- 2-[(4-hydroxy-1,3-benzoxazol-2-yl)methyl]-6-(morpholin-4-yl)pyrimidin-4(3H)-one
- 2-[(7-bromo-1-methyl-1H-benzimidazol-2-yl)methyl]-6-(morpholin-4-yl)pyrimidin-4(3H)-one
ainsi que les sels d'addition avec les acides minéraux et organiques ou avec les bases minérales et organiques desdits produits de formule (I).

La présente invention a tout particulièrement pour objet les produits de formule (I) tels que définis ci-dessus répondant aux formules suivantes :
- 2-[(5-fluoro-1 H-benzimidazol-2-yl)méthyl]-6-(morpholin-4-yl)pyrimidin-4(3H)-one
- 2-(1,3-benzoxazol-2-ylméthyl)-6-(morpholin-4-yl)pyrimidin-4(3H)-one
- 2-[(6-bromo-1H-benzimidazol-2-yl)méthyl]-6-(morpholin-4-yl)pyrimidin-4(3H)-one
- 2-[(6-méthoxy-1H-benzimidazol-2-yl)méthyl]-6-(morpholin-4-yl)pyrimidin-4(3H)-one
- 2-[(5,6-difluoro-1H-benzimidazol-2-yl)méthyl]-6-(morpholin-4-yl)pyrimidin-4(3H)-one
- 2-[(6-fluoro-1,3-benzoxazol-2-yl)méthyl]-6-(morpholin-4-yl)pyrimidin-4(3H)-one
- 2-[(5-fluoro-1,3-benzoxazol-2-yl)méthyl]-6-(morpholin-4-yl)pyrimidin-4(3H)-one
- 2-[(6-méthyl-1,3-benzoxazol-2-yl)méthyl]-6-(morpholin-4-yl)pyrimidin-4(3H)-one
- 2-[(6-bromo-1,3-benzoxazol-2-yl)méthyl]-6-(morpholin-4-yl)pyrimidin-4(3H)-one
- 2-[(1-méthyl-1H-benzimidazol-2-yl)méthyl]-6-(morpholin-4-yl)pyrimidin-4(3H)-one
- 2-[(5-fluoro-1-méthyl-1 H-benzimidazol-2-yl)méthyl]-6-(morpholin-4-yl)pyrimidin-4(3H)-one
- 2-[(7-fluoro-1,3-benzoxazol-2-yl)méthyl]-6-(morpholin-4-yl)pyrimidin-4(3H)-one
- 6-(morpholin-4-yl)-2-[(1-phényle-1H-benzimidazol-2-yl)méthyl]pyrimidin-4(3H)-one
ainsi que les sels d'addition avec les acides minéraux et organiques ou avec les bases minérales et organiques desdits produits de formule (I).

La présente invention a notamment pour objet les produits de formule (I) tels que définis ci-dessus répondant aux formules suivantes :
2-[(1-méthyl-1H-benzimidazol-2-yl)méthyl]-6-(morpholin-4-yl)pyrimidin-4(3H)-one
ainsi que les sels d'addition avec les acides minéraux et organiques ou avec les bases minérales et organiques desdits produits de formule (I).

La présente invention a encore pour objet tout procédé de préparation des produits de formule (I) tels que définis ci-dessus.

Les produits selon l'invention peuvent être préparés à partir de méthodes conventionnelles de chimie organique.

### Préparation de composés de formule (I)

Les produits de formule générale (I) selon la présente invention peuvent notamment être préparés comme indiqué dans les Schémas Généraux 1A-1 B ci-dessous. A ce titre, les méthodes décrites ne sauraient constituer une limitation de la portée de l'invention, en ce qui concerne les méthodes de préparation des composés revendiqués.

Les préparations des exemples de la présente invention donnent des illustrations des Schémas ci-dessous.

De tels schémas de synthèse font partie de la présente invention : la présente invention a ainsi également pour objet les procédés de préparation des produits de formule C à (I)-b, tels que définis dans les Schémas Généraux 1 A-1 B ci-dessous.

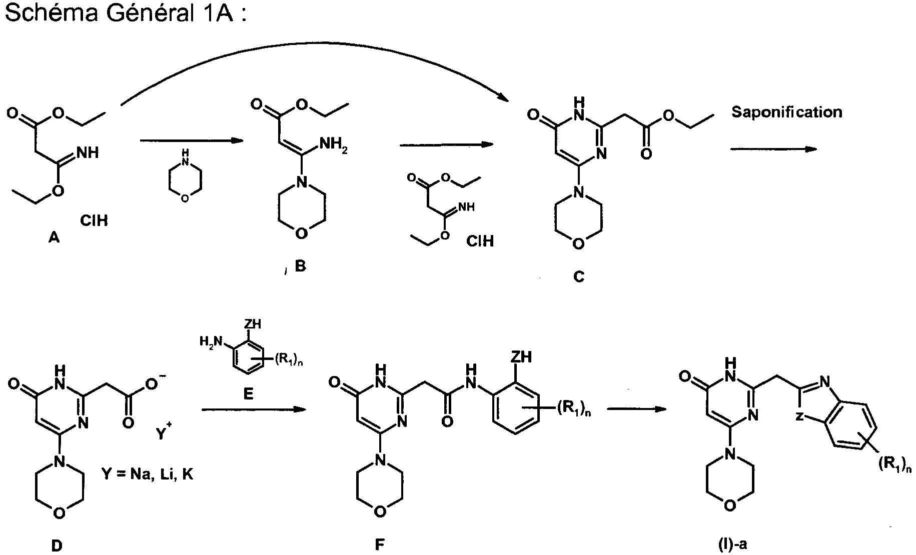

Dans le Schéma Général 1A, les substituants R1 et Z prennent les valeurs définis ci-dessus et en particulier Z= NH et O.

Le cétène aminal B peut être obtenu à partir de l'imino-éther A ou de son tautomère amino-acrylate commercial, par réaction avec la morpholine dans un solvant tel que l'éthanol, à une température comprise entre 0°C et la température d'ébullition du solavant selon le procédé décrit par Landwehr J. et coll. dans J. Med. Chem. 2006, 49, 4327-4332.

L'ester C peut être obtenu par réaction du cétène aminal B avec l'imino-éther A, ou son tautomère amino-acrylate, dans un solvant tel que l'éthanol, à une température comprise entre 20°C et le point d'ébullition du solvant.

Alternativement, l'ester C peut être obtenu par réaction « one-pot » entre la morpholine et un excès (par exemple 3 équivalents) d'imino-éther A (ou de son tautomère amino-acrylate), dans un solvant tel que l'éthanol, à une température comprise entre 20°C et le point d'ébullition du solvant.

Le carboxylate D peut être obtenu par hydrolyse de l'ester C en présence d'une base telle que la soude ou la lithine, dans un solvant tel que le tétrahydrofuranne ou le méthanol, à une température comprise entre 0°C et 30°C.

Les amides F peuvent être obtenus à partir du carboxylate D par condensation d'une aniline E en présence d'un agent de couplage peptidique tel que l'éthyl diméthylaminopropyle carbodiimide (EDCI), le chlorure de 4-(4,6-diméthoxy-1,2,3-triazin-2-yl) 4-méthylmorpholinium (DMT-MM), l'hexafluorophosphate de benzotriazol-1-yloxy tris-diméthylamino phosphonium (BOP), l'hexafluorophosphate de benzotriazol-1-yloxy trispyrrolidino phosphonium (PyBOP), l'hexafluorophosphate de bromo trispyrrolidino phosphonium (PyBROP), hexafluorophosphate de O-(7-azabenzotriazol-1-yl)-1,1,3,3-tétraméthyluronium) (HATU), ou un mélange hydroxybenzotriazole/éthyl diméthylaminopropyle carbodiimide HOBT/EDCI dans un solvant tel que le diméthylformamide, la pyridine, l'éthanol, l'eau ou le méthanol à une température comprise entre 20°C et 50°C comme par exemple dans les conditions décrites par Kunishima M. et Coll. dans Tetrahedron 2001, 57, 1551-1558.

Les produits (I)-a peuvent être obtenus à partir des amides F par cyclodéshydratation dans un solvant tel que l'acide acétique, ou en présence d'un acide tel que l'acide paratoluène sulfonique, dans un solvant tel que le toluène, le xylène ou le n-butanol, à la température d'ébullition du solvant, selon par exemple, le procédé décrit par Arakawa.K et coll. Dans Chemical and Pharmaceutical Bulletin, 45 (12), 1984-1993, 1997.

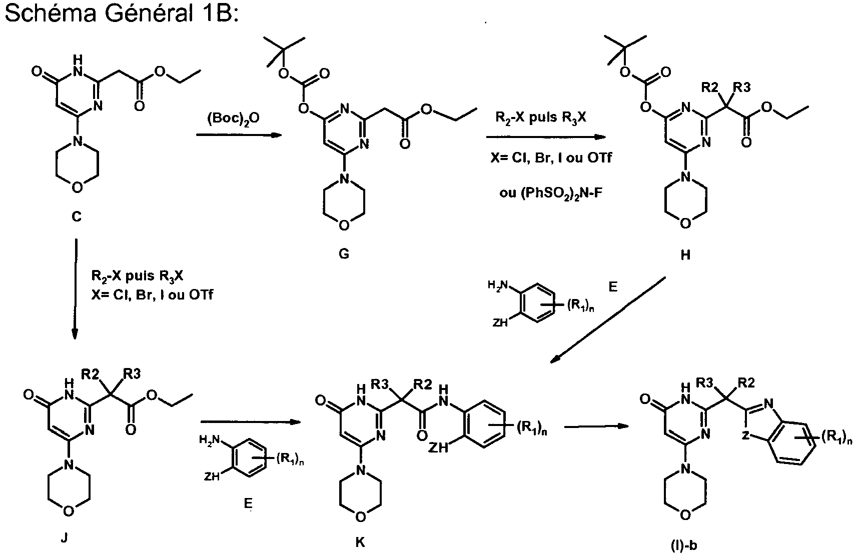

Dans le Schéma Général 1 B, les substituants R1, R2, R3 et Z prennent les valeurs définis ci-dessus et en particulier Z= NH et O.

l'ester G peut être obtenu à partir de l'ester C par réaction avec le (Boc)2O (dicarbonate de tertiobutyle), dans un solvant tel que la diméthylformamide, le dioxanne, l'acétonitrile ou le dichlorométhane, en présence d'une base comme par exemple, l'hydrure de sodium, la triéthylamine, la N,N-diisopropyléthylamine ou la pyridine, à une température comprise entre 0°C et 60°C, selon par exemple le procédé décrit par Hioki K. et Coll. Synthesis 2006, 12, 1931-1933.

Les produits H peuvent être obtenus à partir de l'ester G par réaction avec R2-X puis éventuellement avec R3-X (X = Cl, Br, I ou OTf et R2 et R3 sont des groupes alkyles), en présence d'une base telle que la soude, le tertiobutylate de potassium ou le carbonate de césium, dans un solvant tel que le méthanol, l'éthanol ou le dioxanne, à une température comprise entre 0°C et 100°C, selon par exemple le procédé décrit par Noël D. D'Angelo et coll. dans J. Med. Chem. 2008, 51, 5766-5779.

Le produit H où R2 = R3 = F peut être obtenu par réaction du produit G avec la N-fluorobenzènesulfonimide, en présence d'une base telle que le sel de potassium de l'héxaméthyl-disilylazane, dans un solvant tel que le tétrahydrofuranne, à une température comprise entre -78°C et 20°C, selon par exemple le procédé décrit par Christopher S. Burgey et Coll. dans J. Med. Chem. 2003, 46, 461-473.

Les esters J où les groupes R2 et R3 sont des radicaux alkyles peuvent être obtenus à partir de l'ester C de la même façon que les produits H, en présence d'une base telle que le butyl lithium, l'hydrure de sodium, le tertiobutylate de potassium ou le carbonate de césium dans un solvant tel que le méthanol, l'éthanol, le tétrahydrofuranne, le diméthylformamide ou le dioxanne, à une température comprise entre 0°C et 100°C.

Les amides K peuvent être obtenus à partir des esters H ou J, par réaction d'une aniline E, en présence d'un agent tel le triméthylaluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197.

Les produits (I)-b peuvent être obtenus à partir des amides K par cyclodéshydratation, en présence d'un acide tel que l'acide para-toluène sulfonique, dans un solvant tel que l'acide acétique, le toluène, le xylène ou le n-butanol, à la température d'ébullition du solvant selon, par exemple, le procédé décrit par Arakawa K. et coll. Dans Chemical and Pharmaceutical Bulletin (1997) 45 (12), 1984-1993.

Parmi les produits de départ de formule A ou B certains sont connus et peuvent être obtenus soit commercialement, soit selon les méthodes usuelles connues de l'homme du métier, par exemple à partir de produits commerciaux

Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyles, frityle, benzyle, tert-butoxycarbonyle, BOC, benzyloxycarbonyle, phtalimido ou d'autres radicaux connus dans la chimie des peptides,

Les fonctions acide peuvent être protégées par exemple sous forme d'esters formés avec les esters facilement clivables tels que les esters benzyliques ou ter butyliques ou des esters connus dans la chimie des peptides.

On trouvera une liste de différents groupements protecteurs utilisables dans les manuels connus de l'homme du métier et par exemple dans le brevet FR 2 499 995.

On peut noter que l'on peut soumettre, si désiré et si nécessaire, des produits intermédiaires ou des produits de formule (I) ainsi obtenus par les procédés indiqués ci-dessus, pour obtenir d'autres intermédiaires ou d'autres produits de formule (I), à une ou plusieurs réactions de transformations connues de l'homme du métier telles que par exemple :
a) une réaction d'estérification de fonction acide,
b) une réaction de saponification de fonction ester en fonction acide,
c) une réaction de réduction de la fonction carboxy libre ou estérifié en fonction alcool,
d) une réaction de transformation de fonction alcoxy en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formule (I) ainsi obtenus étant sous toutes les formes isomères possibles racémiques, énantiomères et diastéréoisomères.

Les réactions a) à g) peuvent être réalisées dans les conditions usuelles connues de l'homme du métier telles que, par exemple, celles indiquées ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
   La réaction de saponification peut être réalisée selon les méthodes usuelles connues de l'homme du métier, telles que par exemple dans un solvant tel que le méthanol ou l'éthanol, le dioxanne ou le diméthoxyéthane, en présence de soude ou de potasse.
c) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxanne ou l'éther éthylique.
   Les éventuelles fonctions carboxy libre des produits décrits ci-dessus peuvent être, si désiré, réduites en fonction alcool notamment par de l'hydrure de bore.
d) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro-acétique au reflux.
e) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
f) Les produits décrits ci-dessus peuvent, si désiré, faire l'objet de réactions de salification par exemple par un acide minéral ou organique ou par une base minérale ou organique selon les méthodes usuelles connues de l'homme du métier : une telle réaction de salification peut être réalisée par exemple en présence d'acide chlorhydrique par exemple ou encore d'acide tartrique, citrique ou méthane sulfonique, dans un alcool tel que par exemple l'éthanol ou le méthanol .
g) Les éventuelles formes optiquement actives des produits décrits ci-dessus peuvent être préparées par dédoublement des racémiques selon les méthodes usuelles connues de l'homme du métier.

Les produits de formule (I) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.

Les produits de la présente invention sont notamment utiles pour la thérapie de tumeurs.

Les produits de l'invention peuvent également ainsi augmenter les effets thérapeutiques d'agents anti-tumoraux couramment utilisés.

Ces propriétés justifient leur application en thérapeutique et l'invention a particulièrement pour objet à titre de médicaments, les produits de formule (I) telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a tout particulièrement pour objet, à titre de médicaments, les produits répondant aux formules suivantes :
- 2-[(5-fluoro-1H-benzimidazol-2-yl)méthyl]-6-(morpholin-4-yl)pyrimidin-4(3H)-one
- 2-(1,3-benzoxazol-2-ylméthyl)-6-(morpholin-4-yl)pyrimidin-4(3H)-one
- 2-[(6-bromo-1H-benzimidazol-2-yl)méthyl]-6-(morpholin-4-yl)pyrimidin-4(3H)-one
- 2-[(6-méthoxy-1 H-benzimidazol-2-yl)méthyl]-6-(morpholin-4-yl)pyrimidin-4(3H)-one
- 2-[(5,6-difluoro-1 H-benzimidazol-2-yl)méthyl]-6-(morpholin-4-yl)pyrimidin-4(3H)-one
- 2-[(6-fluoro-1,3-benzoxazol-2-yl)méthyl]-6-(morpholin-4-yl)pyrimidin-4(3H)-one
- 2-[(5-fluoro-1,3-benzoxazol-2-yl)méthyl]-6-(morpholin-4-yl)pyrimidin-4(3H)-one
- 2-[(6-méthyl-1,3-benzoxazol-2-yl)méthyl]-6-(morpholin-4-yl)pyrimidin-4(3H)-one
- 2-[(6-bromo-1,3-benzoxazol-2-yl)méthyl]-6-(morpholin-4-yl)pyrimidin-4(3H)-one
- 2-[(1-méthyl-1H-benzimidazol-2-yl)méthyl]-6-(morpholin-4-yl)pyrimidin-4(3H)-one
- 2-[(5-fluoro-1-méthyl-1H-benzimidazol-2-yl)méthyl]-6-(morpholin-4-yl)pyrimidin-4(3H)-one
- 2-[(7-fluoro-1,3-benzoxazol-2-yl)méthyl]-6-(morpholin-4-yl)pyrimidin-4(3H)-one
- 6-(morpholin-4-yl)-2-[(1-phényl-1H-benzimidazol-2-yl)méthyl]pyrimidin-4(3H)-one
- 2-[(1-benzyl-1H-benzimidazol-2-yl)méthyl]-6-(morpholin-4-yl)pyrimidin-4(3H)-one
- 2-[(1-éthyl-1H-benzimidazol-2-yl)méthyl]-6-(morpholin-4-yl)pyrimidin-4(3H)-one
- 2-[(5,6-difluoro-1,3-benzoxazol-2-yl)méthyl]-6-(morpholin-4-yl)pyrimidin-4(3H)-one
- 2-[(6-chloro-1 H-benzimidazol-2-yl)méthyl]-6-(morpholin-4-yl)pyrimidin-4(3H)-one
- 2-[(1-cyclopropyl-5-fluoro-1H-benzimidazol-2-yl)méthyl]-6-(morpholin-4-yl)pyrimidin-4(3H)-one
- 2-{[5-fluoro-1-(2-méthoxyéthyl)-1H-benzimidazol-2-yl]méthyl}-6-(morpholin-4-yl)pyrimidin-4(3H)-one
- 6-(morpholin-4-yl)-2-(naphtho[2,1-d][1,3]oxazol-2-ylmethyl)pyrimidin-4(3H)-one
- 2-[(6-methoxy-1,3-benzoxazol-2-yl)methyl]-6-(morpholin-4-yl)pyrimidin-4(3H)-one
- 2-[(6,7-difluoro-1,3-benzoxazol-2-yl)methyl]-6-(morpholin-4-yl)pyrimidin-4(3H)-one
- 2-[(5,6-dichloro-1 H-benzimidazol-2-yl)methyl]-6-(morpholin-4-yl)pyrimidin-4(3H)-one
- 2-[(6-methyl-1 H-benzimidazol-2-yl)methyl]-6-(morpholin-4-yl)pyrimidin-4(3H)-one
- 2-{[4-chloro-6-(trifluoromethyl)-1H-benzimidazol-2-yl]methyl}-6-(morpholin-4-yl)pyrimidin-4(3H)-one
- 6-(morpholin-4-yl)-2-{[6-(trifluoromethyl)-1H-benzimidazol-2-yl]methyl}pyrimidin-4(3H)-one
- 2-[(5-chloro-6-fluoro-1 H-benzimidazol-2-yl)methyl]-6-(morpholin-4-yl)pyrimidin-4(3H)-one
- 2-[(6,7-difluoro-1H-benzimidazol-2-yl)methyl]-6-(morpholin-4-yl)pyrimidin-4(3H)-one
- 2-[(5-chloro-6-methyl-1 H-benzimidazol-2-yl)methyl]-6-(morpholin-4-yl)pyrimidin-4(3H)-one
- (2-{[4-(morpholin-4-yl)-6-oxo-1,6-dihydropyrimidin-2-yl]methyl}-1H-benzimidazol-1-yl)acetonitrile
- 2-[(5,7-difluoro-1 H-benzimidazol-2-yl)methyl]-6-(morpholin-4-yl)pyrimidin-4(3H)-one
- 2-{[5-chloro-1-(1-phenylethyl)-1H-benzimidazol-2-yl]methyl}-6-(morpholin-4-yl)pyrimidin-4(3H)-one
- 2-[(1-cyclohexyl-1H-benzimidazol-2-yl)methyl]-6-(morpholin-4-yl)pyrimidin-4(3H)-one
- 6-(morpholin-4-yl)-2-[(5,6,7-trifluoro-1 H-benzimidazol-2-yl)methyl]pyrimidin-4(3H)-one
- 2-[(4-bromo-6-fluoro-1H-benzimidazol-2-yl)methyl]-6-(morpholin-4-yl)pyrimidin-4(3H)-one
- 2-[(6-bromo-1-methyl-1H-benzimidazol-2-yl)methyl]-6-(morpholin-4-yl)pyrimidin-4(3H)-one
- 2-[(6,7-difluoro-1-methyl-1H-benzimidazol-2-yl)methyl]-6-(morpholin-4-yl)pyrimidin-4(3H)-one
- 2-[(5,6-dichloro-1-methyl-1H-benzimidazol-2-yl)methyl]-6-(morpholin-4-yl)pyrimidin-4(3H)-one
- 6-(morpholin-4-yl)-2-[(5,6,7-trifluoro-1-methyl-1H-benzimidazol-2-yl)methyl]pyrimidin-4(3H)-one
- 2-[(4-hydroxy-1,3-benzoxazol-2-yl)methyl]-6-(morpholin-4-yl)pyrimidin-4(3H)-one
- 2-[(7-bromo-1-methyl-1H-benzimidazol-2-yl)methyl]-6-(morpholin-4-yl)pyrimidin-4(3H)-one
ainsi que les sels d'addition avec les acides minéraux et organiques ou avec les bases minérales et organiques pharmaceutiquement acceptables desdits produits de formule (I).

L'invention concerne aussi des compositions pharmaceutiques contenant à titre de principe actif l'un au moins des produits de formule (I) tels que définis ci-dessus ou un sel pharmaceutiquement acceptable de ce produit et, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés parafflniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

Un tel médicament peut notamment être destiné au traitement ou à la prévention d'une maladie chez un mammifère.

Est également décrit l'utilisation d'un produit de formule (I) tel que défini ci-dessus pour la préparation d'un médicament destiné à la prévention ou au traitement de maladies liées à une prolifération non contrôlée.

La présente invention a ainsi tout particulièrement pour objet l'utilisation d'un produit de formule (I) tel que défini ci-dessus pour la préparation d'un médicament destiné au traitement ou à la prévention de maladies en oncologie et notamment destiné au traitement de cancers.

Parmi ces cancers, on s'intéresse au traitement de tumeurs solides ou liquides, au traitement de cancers résistant à des agents cytotoxiques Les produits de la présente invention cités peuvent notamment être utilisés pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

La présente invention a aussi pour objet l'utilisation des produits de formule (I) telle que définie ci-dessus pour la préparation de médicaments destinés à la chimiothérapie de cancers.

La présente invention a ainsi pour objet les produits de formule (I) tels que définis ci-dessus pour leur utilisation pour le traitement de cancers.

La présente invention a pour objet les produits de formule (I) tels que définis ci-dessus pour leur utilisation pour le traitement de tumeurs solides ou liquides.

La présente invention a donc pour objet les produits de formule (I) tels que définis ci-dessus pour leur utilisation pour le traitement de cancers résistant à des agents cytotoxiques.

La présente invention a donc pour objet les produits de formule (I) tels que définis ci-dessus pour leur utilisation pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

La présente invention a donc pour objet les produits de formule (I) tels que définis ci-dessus pour leur utilisation pour la chimiothérapie de cancers.

De tels médicaments destinés à la chimiothérapie de cancers peuvent être utilisés seuls ou en en association.

La présente invention a donc pour objet les produits de formule (I) tels que définis ci-dessus pour leur utilisation pour la chimiothérapie de cancers, seuls ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

La présente invention a encore pour objet à titre de produits industriels nouveaux, les intermédiaires de synthèse de formules C, D, F, J et K tels que définis ci-dessus et rappelés ci-après : 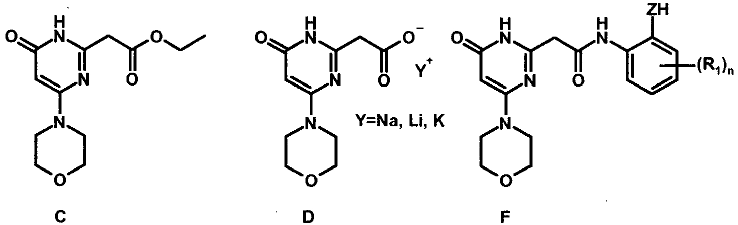 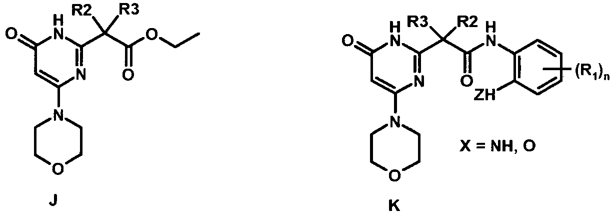 dans lesquels R1, R2, R3 et Z ont la définition indiquée à l'une quelconque des revendications 1 à 2.

Les exemples suivants qui sont des produits de formule (I) illustrent l'invention sans toutefois la limiter.

### Partie expérimentale

La nomenclature des composés de cette présente invention a été effectuée avec le logiciel ACDLABS version 10.0.

Le four à microondes utilisé est un appareil Biotage, Initiator ^{™} 2.0, 400W max, 2450 MHz.

Les spectres de RMN ¹H à 400 MHz et ¹H à 500 MHz ont été effectués sur spectromètre BRUKER AVANCE DRX-400 ou BRUKER AVANCE DPX-500 avec les déplacements chimiques (δ en ppm) dans le solvant diméthylsulfoxide-d₆ (DMSO-d₆) référencé à 2,5 ppm à la température de 303K.

Les spectres de masse (SM) ont été obtenus soit par la méthode A, la méthode B, ou la méthode C :

### Méthode A :

Appareil WATERS UPLC-SQD ; Ionisation : électrospray en mode positif et/ou négatif (ES+/-) ; Conditions chromatographiques : Colonne : ACQUITY BEH C₁₈ 1,7 µm - 2,1 x 50 mm ; Solvants : A : H₂O (0,1 % acide formique) B : CH₃CN (0,1 % acide formique) ; Température de colonne : 50 °C ; Débit : 1 ml/min ; Gradient (2 min) : de 5 à 50 % de B en 0,8 min ; 1,2 min : 100 % de B ; 1,85 min : 100 % de B ; 1,95 : 5 % de B ; Temps de rétention = Tr (min).

### Méthode B :

Appareil WATERS ZQ ; Ionisation : électrospray en mode positif et/ou négatif (ES+/-) ; Conditions chromatographiques : Colonne : XBridge C18 2,5 pm - 3 x 50 mm ; Solvants : A : H2O (0,1 % acide formique) B : CH3CN (0,1 % acide formique) ; Température de colonne : 70°C ; Débit : 0,9 ml/min ; Gradient (7 min) : de 5 à 100 % de B en 5,3 min ; 5,5 min : 100 % de B ; 6,3 min : 5 % de B ; Temps de rétention = Tr (min).

### Méthode C :

Appareil Waters UPLC, Colonne BEH C18 2,1*50 mm; 1.7u, H2O+0.1%TFA : AcN+0.08% TFA 95:5 (0min) to 5:95 (1.1min) to 5:95 (1.7min) to 95:5 (1.8min) to 95:5 (2min), 0.9 ml/min 55°C ; Waters SQD Single Quadrupol, 0.5s scantime for mass 120-1200

### Exemple 1 : Synthèse du 2-[4-(morpholin-4-yl)-6-oxo-1,6-dihydropyrimidin-2-yl]-N-phénylacétamide

### Etape 1 :

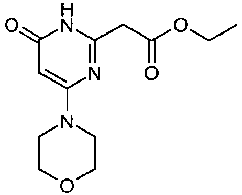

A une solution de 25 g de morpholine dans 400 mL d'éthanol chauffée à 95 °C sont ajoutés 168.5 mL de chlorhydrate de 3-éthoxy-3-iminopropanoate d'éthyle, puis 155 mL de N,N-diisopropyléthylamine dans 200 mL d'éthanol. Le mélange réactionnel est chauffé à 95 °C pendant 30 heures puis laissé revenir à température ambiante. Le précipité formé est filtré sur verre fritté puis lavé avec 100 mL d'éthanol, 2 fois 500 mL d'eau et enfin 500 mL d'éther éthylique. Le solide est séché sous vide pour donner 35 g de [4-(morpholin-4-yl)-6-oxo-1,6-dihydropyrimidin-2-yl]acétate d'éthyle sous forme de solide blanc dont les caractéristiques sont les suivantes :
Spectre RMN 1H (400MHz, δ en ppm, DMSO-d6) : 1,19 (t, J=7,1 Hz, 3 H); 3,38 à 3,44 (m, 4 H); 3,56 (s, 2H); 3,61 (dd, J=4,0 et 5,7 Hz, 4 H); 4,12 (q, J=7,1 Hz, 2 H); 5,20 (s, 1 H); 11,69 (s large, 1 H)
Spectrométrie de Masse : méthode A
Temps de rétention Tr (min) = 0.48;
[M+H]+ : m/z 268 ; [M-H]- : m/z 266
Pureté : 98 %

### Etape 2:

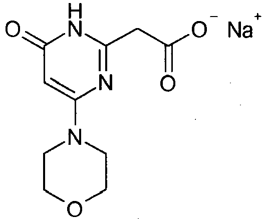

A une solution de 10 g de [4-(morpholin-4-yl)-6-oxo-1,6-dihydropyrimidin-2-yl]acétate d'éthyle dans 300 mL de tétrahydrofuranne, est ajouté 18.7 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif pour donner 8.7 g de [4-(morpholin-4-yl)-6-oxo-1,6-dihydropyrimidin-2-yl]acétate de sodium sous forme de solide blanc dont les caractéristiques sont les suivantes:
Spectre RMN 1 H (400MHz, d en ppm, DMSO-d6) : 3,08 (s, 2 H); 3,38 (t, J=4,6 Hz, 4 H); 3,61 (t, J=4,6Hz, 4 H); 5,08 (s, 1 H); 13,16 (s large, 1 H)
Spectrométrie de Masse : méthode A
Temps de rétention Tr (min) = 0.29;
[M+H]+ : m/z 240 ; [M-H]- : m/z 238
Pureté : 98 %

### Etape 3 :

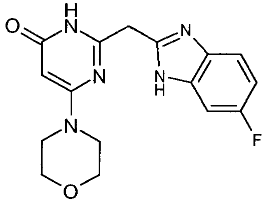

A une solution de 261 mg de [4-(morpholin-4-yl)-6-oxo-1,6-dihydropyrimidin-2-yl]acétate de sodium dans 8 mL de méthanol sont ajoutés 151 mg de 1,2-diamino-4-fluorobenzène. On agite à température ambiante pendant 5 minutes puis on ajoute 354 mg chlorure de 4-(4,6-diméthoxy-1,3,5-triazin-2-yl)-4-méthylmorpholin-4-ium hydrate. On agite ainsi pendant 30 minutes à température ambiante. Le mélange réactionnel est ensuite concentré à sec sous pression réduite, puis le résidu est repris dans 30 mL d'acide acétique. Le mélange réactionnel est porté au reflux pendant 1 heure, puis concentré sous pression réduite. On ajoute alors 30 mL d'eau et une solution aqueuse saturée de bicarbonate de sodium jusqu'à obtention d'un pH voisin de 7. On ajoute alors 20 mL d'acétate d'éthyle et on agite pendant 1 heure. On filtre le précipité formé que l'on purifie ensuite par chromatographie sur colonne de gel de silice en éluant avec un gradient de l'éluant CH2Cl2/MeOH : 80/20 dans le dichlorométhane de 0% à 100% en 25 minutes. On obtient 140 mg de 2-[(5-fluoro-1H-benzimidazol-2-yl)méthyl]-6-(morpholin-4-yl)pyrimidin-4(3H)-one sous forme de solide blanc cassé dont les caractéristiques sont les suivantes:
Spectre RMN 1 H (400MHz, d en ppm, DMSO-d6) : 3,37 (t, J=4,9 Hz, 4 H); 3,58 (t, J=4,9 Hz, 4 H); 4,08(s, 2 H); 5,22 (s, 1 H); 7,00 (t, J=8,2 Hz, 1 H); 7,31 (dd, J=0,7 et 9,8 Hz, 1 H); 7,49 (s large, 1 H); 12,15 (s large, 2 H)
Spectrométrie de Masse : méthode A
Temps de rétention Tr (min) = 0.41;
[M+H]+ : m/z 330 ; [M-H]- : m/z 328
Pureté : 98 %

### Exemple 2 : Synthèse du 2-(1,3-benzoxazol-2-ylméthyl)-6-(morpholin-4-yl)pyrimidin-4(3H)-one

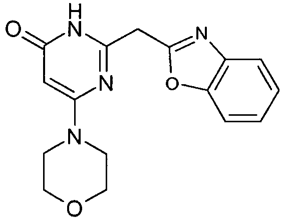

### Etape 1 :

A une solution de 500 mg de [4-(morpholin-4-yl)-6-oxo-1,6-dihydropyrimidin-2-yl]acétate de sodium dans 4 mL de diméthylformamide sont ajoutés 4 mL de pyridine, 550 mg de N-[3-(diméthylamino)propyl]-N'-éthylcarbodiimide chlorhydrate et 700 mg de 2-aminophénol. On agite à température ambiante pendant 1 nuit, puis le mélange réactionnel est concentré sous pression réduite. On ajoute de l'eau et de l'acétate d'éthyle et agite ainsi pendant 30 minutes. Le précipité formé est filtré, rincé à l'eau, à l'éther éthylique et à l'éther de pétrole. Le solide obtenu est séché sous vide. On obtient ainsi 370 mg de N-(2-hydroxyphényl)-2-[4-(morpholin-4-yl)-6-oxo-1,6-dihydropyrimidin-2-yl]acétamide sous forme de solide beige dont les caractéristiques sont les suivantes:
Spectrométrie de Masse : méthode B
Temps de rétention Tr (min) = 2.58;
[M+H]+ : m/z 331 ; [M-H]- : m/z 329
Pureté : 98 %

### Etape 2 :

A une solution de 200 mg de N-(2-hydroxyphényl)-2-[4-(morpholin-4-yl)-6-oxo-1,6-dihydropyrimidin-2-yl]acétamide dans 40 mL de toluène sont ajoutés 20 mg d'hydrate d'acide 4-méthylbenzènesulfonique. Le mélange réactionnel est porté au reflux pendant 1 nuit. Après refroidissement à 0°C, l'insoluble formé est filtré. Le filtrat est concentré à sec sous pression réduite puis le résidu est chromatographié sur colonne de gel de silice, éluant : CH2Cl2/MeOH : 98/02 puis 90/10. On obtient ainsi 16 mg de 2-(1,3-benzoxazol-2-ylméthyl)-6-(morpholin-4-yl)pyrimidin-4(3H)-one sous forme de solide blanc dont les caractéristiques sont les suivantes:
Spectre RMN 1 H (400MHz, d en ppm, DMSO-d6) : 3,35 (t, J=5,0 Hz, 4 H); 3,56 (t, J=4,9 Hz, 4 H); 4,25 (s, 2 H); 5,25 (s large, 1 H); 7,31 à 7,40 (m, 2 H); 7,62 à 7,74 (m, 2 H); 11,92 (s large, 1 H)
Spectrométrie de Masse : méthode A
Temps de rétention Tr (min) = 0.60
[M+H]+ : m/z 313 ; [M-H]- : m/z 311
Pureté : 98 %
Point de fusion (Köfler) = 224°C

### Exemple 3 : Synthèse du 2-[(5-bromo-1H-benzimidazol-2-yl)méthyl]-6-(morpholin-4-yl)pyrimidin-4(3H)-one

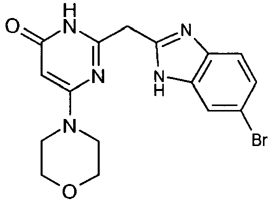

Dans un ballon, on introduit 1.3 g de [4-(morpholin-4-yl)-6-oxo-1,6-dihydropyrimidin-2-yl]acétate de sodium dans 10 mL de diméthylformamide puis on ajoute 10 mL de pyridine, 1.44 g de N-[3-(diméthylamino)propyl]-N'-éthylcarbodiimide chlorhydrate et 1.87 g de 4-bromobenzène-1,2-diamine. On agite à température ambiante pendant 1 nuit, puis le mélange réactionnel est concentré à sec sous pression réduite. Le résidu est repris dans 40 mL d'acide acétique et porté au reflux pendant 4 heures, puis concentré sous pression réduite. On ajoute alors 100 mL d'eau et une solution aqueuse saturée de bicarbonate de sodium jusqu'à obtention d'un pH voisin de 7. On ajoute ensuite 200 mL d'acétate d'éthyle et on agite pendant 1 heure. Le précipité formé est filtré puis purifié par chromatographie sur colonne de gel de silice en éluant avec un gradient de l'éluant CH2Cl2/MeOH : 80/20 dans le dichlorométhane de 0% à 100% en 25 minutes. On obtient 560 mg de 2-[(5-bromo-1H-benzimidazol-2-yl)méthyl]-6-(morpholin-4-yl)pyrimidin-4(3H)-one sous forme de solide blanc cassé dont les caractéristiques sont les suivantes:
Spectre RMN 1 H (400MHz): 3,34 à 3,39 (m, 4 H) ; 3,55 à 3,59 (m, 4 H) ; 4,09 (s,2 H) ; 5,21 (s, 1 H) ; 7,28 (dd, J=2,0 et 8,6 Hz, 1 H) ; 7,47 (d, J=8,6 Hz, 1 H) ; 7,71 (d,J=2,0 Hz, 1 H) ;12,20 (m étalé, 2 H)
Spectrométrie de Masse : méthode B
Temps de rétention Tr (min) = 2,71 ;
[M+H]+ : m/z 390 ; [M-H]- : m/z 388
Pureté : 98 %

### Exemple 4 : Synthèse du 2-[(6-méthoxy-1H-benzimidazol-2-yl)méthyl]-6-(morpholin-4-yl)pyrimidin-4(3H)-one

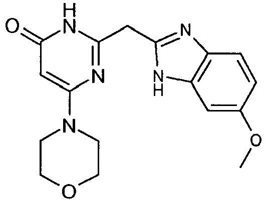

Le produit est préparé en suivant le mode opératoire décrit à l'étape 3 de l'exemple 1 à partir de 261 mg de [4-(morpholin-4-yl)-6-oxo-1,6-dihydropyrimidin-2-yl]acétate de sodium et 166 mg de 1,2-diamino-4-méthoxybenzène au lieu du 1,2-diamino-4-fluorobenzène. Après purification par chromatographie sur colonne de gel de silice en éluant avec un gradient de CH2Cl2 pur à CH2Cl2/MeOH : 85/15, on obtient 8 mg de 2-[(6-méthoxy-1H-benzimidazol-2-yl)méthyl]-6-(morpholin-4-yl)pyrimidin-4(3H)-one sous forme de solide blanc cassé dont les caractéristiques sont les suivantes:
Spectre RMN 1 H (400MHz): 3,38 (m, 4 H) ; 3,58 (m, 4 H) ; 3,76 (s, 3 H) ; 4,04 (s,2 H) ; 5,21 (s, 1 H) ; 6,72 à 6,82 (m, 1 H) ; 6,89 à 7,15 (m étalé, 1 H) ; 7,27 à 7,52 (métalé, 1 H) ; 11,82 (métalé, 1 H) ; 12,11 (m étalé, 1 H)
Spectrométrie de Masse : méthode A
Temps de rétention Tr (min) = 0,40 ;
[M+H]+ : m/z 342
Pureté : 90 %

### Exemple 5 : Synthèse du 2-[(5,6-difluoro-1H-benzimidazol-2-yl)méthyl]-6-(morpholin-4-yl)pyrimidin-4(3H)-one

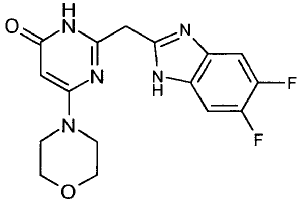

Le produit est préparé en suivant le mode opératoire décrit à l'étape 3 de l'exemple 1 à partir de 261 mg de [4-(morpholin-4-yl)-6-oxo-1,6-dihydropyrimidin-2-yl]acétate de sodium et 173 mg de 1,2-diamino-4,5-difluorobenzène au lieu du 1,2-diamino-4-fluorobenzène. Après purification par chromatographie sur colonne de gel de silice en éluant avec un gradient de CH2Cl2 pur à CH2Cl2/MeOH : 85/15, on obtient 150 mg de 2-[(5,6-difluoro-1 H-benzimidazol-2-yl)méthyl]-6-(morpholin-4-yl)pyrimidin-4(3H)-one sous forme de solide rose clair dont les caractéristiques sont les suivantes:
Spectre RMN 1 H (400MHz): 3,34 à 3,39 (m, 4 H) ; 3,55 à 3,60 (m, 4 H) ; 4,08 (s,2 H) ; 5,21 (s, 1 H) ; 7,57 (dd, J=7,8 et 10,8 Hz, 2 H) ; 12,16 (s large, 2 H)
Spectrométrie de Masse : méthode A
Temps de rétention Tr (min) = 0,51 ;
[M+H]+ : m/z 348 ; [M-H]- : m/z 346
Pureté : 98 %

### Exemple 6 : Synthèse du 2-[(6-fluoro-1,3-benzoxazol-2-yl)méthyl]-6-(morpholin-4-yl)pyrimidin-4(3H)-one

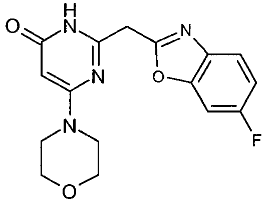

### Etape 1 :

Le produit est préparé en suivant le mode opératoire décrit à l'étape 1 de l'exemple 2 à partir de 550 mg de [4-(morpholin-4-yl)-6-oxo-1,6-dihydropyrimidin-2-yl]acétate de sodium et 815 mg de 2-amino-5-fluorophnénol au lieu de 2-aminophénol. Après purification par chromatographie sur colonne de gel de silice en éluant avec CH2Cl2 pur puis, CH2Cl2/MeOH : 95/05, on obtient 149 mg de N-(4-fluoro-2-hydroxyphényl)-2-[4-(morpholin-4-yl)-6-oxo-1,6-dihydropyrimidin-2-yl]acétamide sous forme de solide gris dont les caractéristiques sont les suivantes:
Spectrométrie de Masse : méthode B
Temps de rétention Tr (min) = 2,74
[M+H]+ : m/z 349 ; [M-H]- : m/z 347

### Etape 2 :

Le produit est préparé en suivant le mode opératoire décrit à l'étape 2 de l'exemple 2 à partir de 120 mg N-(4-fluoro-2-hydroxyphényl)-2-[4-(morpholin-4-yl)-6-oxo-1,6-dihydropyrimidin-2-yl]acétamide, 11 mg de acide 4-méthylbenzènesulfonique hydrate et en remplaçant le toluène par le xylène. Après purification par chromatographie sur colonne de gel de silice, éluant : CH2Cl2/MeOH : 98/02, on obtient 15 mg de 2-[(6-fluoro-1,3-benzoxazol-2-yl)méthyl]-6-(morpholin-4-yl)pyrimidin-4(3H)-one sous forme de solide blanc dont les caractéristiques sont les suivantes:
Spectre RMN 1 H (400MHz): 3,35 (m, 4 H) ; 3,56 (m, 4 H) ; 4,25 (s, 2 H) ; 5,25 (s large, 1 H) ; 7,24 (ddd, J=2,6 et 8,7 et 10,0 Hz, 1 H) ; 7,70 à 7,77 (m, 2 H) ; 11,93 (s large, 1 H)
Spectrométrie de Masse : méthode A
Temps de rétention Tr (min) = 0,65
[M+H]+ : m/z 329 ; [M-H]- : m/z 331

### Exemple 7 : Synthèse du 2-[(5-fluoro-1,3-benzoxazol-2-yl)méthyl]-6-(morpholin-4-yl)pyrimidin-4(3H)-one

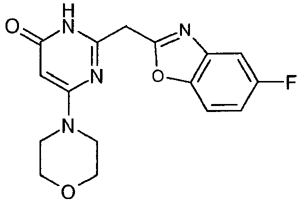

### Etape 1 :

Le produit est préparé en suivant le mode opératoire décrit à l'étape 1 de l'exemple 2 à partir de 1 g de [4-(morpholin-4-yl)-6-oxo-1,6-dihydropyrimidin-2-yl]acétate de sodium et 466 mg de 2-amino-4-fluorophénol au lieu de la 2,4-difluoroaniline. On obtient 795 mg de 2 N-(5-fluoro-2-hydroxyphényl)-2-[4-(morpholin-4-yl)-6-oxo-1,6-dihydropyrimidin-2-yl]acétamide sous forme de solide marron dont les caractéristiques sont les suivantes :
Spectrométrie de Masse : méthode A
Temps de rétention Tr (min) = 0,55
[M+H]+ : m/z 349 ; [M-H]- : m/z 347

### Etape 2 :

Le produit est préparé en suivant le mode opératoire décrit à l'étape 2 de l'exemple 2 à partir de 700 mg N-(5-fluoro-2-hydroxyphényl)-2-[4-(morpholin-4-yl)-6-oxo-1,6-dihydropyrimidin-2-yl]acétamide, 292 mg de acide 4-méthylbenzènesulfonique hydrate et en remplaçant le toluène par le xylène. Après purification par chromatographie sur colonne de gel de silice en éluant avec un mélange CH2Cl2/MeOH : 98/02 puis, CH2Cl2/MeOH : 95/05,suivie d'une recristallisation dans le méthanol, on obtient 188 mg de 2-[(5-fluoro-1,3-benzoxazol-2-yl)méthyl]-6-(morpholin-4-yl)pyrimidin-4(3H)-one sous forme de solide marron dont les caractéristiques sont les suivantes: Spectre RMN 1 H (400MHz): pour ce lot, tous les signaux sont larges avec : 3,33 à 3,39 (m, 4 H) ; 3,54 à 3,62 (m, 4 H) ; 4,26 (s, 2 H) ; 5,25 (s, 1 H) ; 7,25 (t, *J*=9,0 Hz, 1 H) ; 7,61 (d, *J*=9,0 Hz, 1 H) ; 7,76 (dd, *J*=4,5 et 9,0 Hz, 1 H) ; 11,83 à 11,96 (m étalé, 1 H)
Spectrométrie de Masse : méthode A
Temps de rétention Tr (min) = 0,65
[M+H]+ : m/z 331 ; [M-H]- : m/z 329

### Exemple 8 : Synthèse de 2-[(6-méthyl-1,3-benzoxazol-2-yl)méthyl]-6-(morpholin-4-yl)pyrimidin-4(3H)-one

### Etape 1:

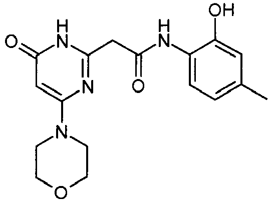

Le produit est préparé en suivant le mode opératoire décrit à l'exemple 2 à partir de 500 mg de [4-(morpholin-4-yl)-6-oxo-1,6-dihydropyrimidin-2-yl]acétate de sodium et 466 mg de 2-amino-5-méthylphénol au lieu du 2,4-difluoroaniline. On obtient 515 mg de N-(2-hydroxy-4-méthylphényl)-2-[4-(morpholin-4-yl)-6-oxo-1,6-dihydropyrimidin-2-yl]acétamide sous forme de solide violacé dont les caractéristiques sont les suivantes :
Spectrométrie de Masse : méthode A
Temps de rétention Tr (min) = 0,57
[M+H]+ : m/z 345 ; [M-H]- : m/z 343

### Etape 2:

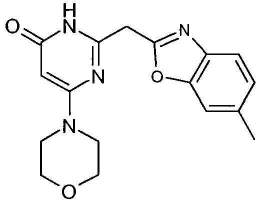

Le produit est préparé en suivant le mode opératoire décrit à l'exemple 2 à partir de 390 mg N-(2-hydroxy-4-méthylphényl)-2-[4-(morpholin-4-yl)-6-oxo-1,6-dihydropyrimidin-2-yl]acétamide, 108 mg de acide 4-méthylbenzènesulfonique hydrate et en remplaçant le toluène par le xylène. Après recristallisation dans 20 mL d'éthanol et 5 mL de méthanol, on obtient 105 mg de 2-[(6-méthyl-1,3-benzoxazol-2-yl)méthyl]-6-(morpholin-4-yl)pyrimidin-4(3H)-one sous forme de solide blanc dont les caractéristiques sont les suivantes:
Spectre RMN 1 H (400MHz): 2,43 (s, 3 H) ; 3,36 (m, 4 H) ; 3,56 (m, 4 H) ; 4,21 (s, 2 H) ; 5,24 (m étalé, 1 H) ; 7,18 (d large, J=8,3 Hz, 1 H) ; 7,51 (s large, 1 H) ; 7,57 (d, J=8,3 Hz, 1 H) ; 11,91 (m étalé, 1 H)
Spectrométrie de Masse : méthode A
Temps de rétention Tr (min) = 0,69
[M+H]+ : m/z 327 ; [M-H]- : m/z 325

### Exemple 9 : Synthèse de 2-[(6-bromo-1,3-benzoxazol-2-yl)méthyl]-6-(morpholin-4-yl)pyrimidin-4(3H)-one

### Etape 1 :

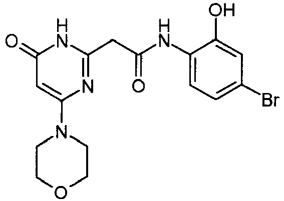

Le produit est préparé en suivant le mode opératoire décrit à l'exemple 2 à partir de 500 mg de [4-(morpholin-4-yl)-6-oxo-1,6-dihydropyrimidin-2-yl]acétate de sodium et 520 mg de 2-amino-4-bromophénol au lieu du 2,4-difluoroaniline. On obtient 350 mg de N-(4-bromo-2-hydroxyphényl)-2-[4-(morpholin-4-yl)-6-oxo-1,6-dihydropyrimidin-2-yl]acétamide sous forme de solide violacé dont les caractéristiques sont les suivantes :
Spectrométrie de Masse : méthode A
Temps de rétention Tr (min) = 0,65
[M+H]+ : m/z 409 ; [M-H]- : m/z 407

### Etape 2 :

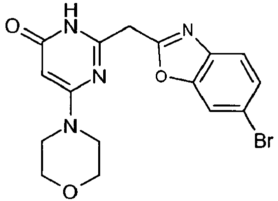

Le produit est préparé en suivant le mode opératoire décrit à l'exemple 2 à partir de 316 mg N-(4-bromo-2-hydroxyphényl)-2-[4-(morpholin-4-yl)-6-oxo-1,6-dihydropyrimidin-2-yl]acétamide, 73 mg de acide 4-méthylbenzènesulfonique hydrate et en remplaçant le toluène par le xylène. Après recristallisation dans 30 mL d'éthanol, 5 mL de méthanol et 5 mL de dichlorométhane puis purification du précipité formé par chromatographie sur colonne de silice, éluant : CH₂Cl₂/MeOH : 90/10, on obtient 70 mg de 2-[(6-bromo-1,3-benzoxazol-2-yl)méthyl]-6-(morpholin-4-yl)pyrimidin-4(3H)-one sous forme de solide blanc cassé dont les caractéristiques sont les suivantes:
Spectre RMN 1 H (400MHz): 3,35 (m, 4 H) ; 3,56 (m, 4 H) ; 4,26 (s, 2 H) ; 5,25 (m étalé, 1 H) ; 7,54 (dd, J=1,8 et 8,4 Hz, 1 H) ; 7,69 (d, J=8,4 Hz, 1 H) ; 8,07 (d, J=1,8 Hz, 1 H) ; 11,92 (m étalé, 1 H)
Spectrométrie de Masse : méthode B
Temps de rétention Tr (min) = 3,40
[M+H]+ : m/z 391 ; [M-H]- : m/z 389

### Exemple 10 : Synthèse de 2-[(1-méthyl-1H-benzimidazol-2-yl)méthyl]-6-(morpholin-4-yl)pyrimidin-4(3H)-one

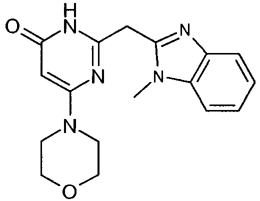

Le produit est préparé en suivant le mode opératoire décrit à l'exemple 3 à partir de 300 mg de [4-(morpholin-4-yl)-6-oxo-1,6-dihydropyrimidin-2-yl]acétate de sodium et 280 mg de N-méthyl-1,2-phenylenediamine au lieu du 4-bromobenzène-1,2-diamine. Après purification par chromatographie sur colonne de silice, éluant: un gradient de l'éluant CH₂Cl₂ pur à CH₂Cl₂/MeOH : 90/10, on obtient 225 mg de 2-[(1-méthyl-1H-benzimidazol-2-yl)méthyl]-6-(morpholin-4-yl)pyrimidin-4(3H)-one sous forme de solide blanc dont les caractéristiques sont les suivantes:
Spectre RMN 1 H (400MHz): 3,34 (m, 4 H) ; 3,56 (m, 4 H) ; 3,80 (s, 3 H) ; 4,20 (s, 2 H) ; 5,21 (s, 1 H) ; 7,17 (t, J=7,8 Hz, 1 H) ; 7,23 (t, J=7,8 Hz, 1 H) ; 7,52 (d, J=7,8 Hz, 1 H) ; 7,56 (d, J=7,8 Hz, 1 H) ; 11,85 (s large, 1 H)
Spectrométrie de Masse : méthode A
Temps de rétention Tr (min) = 0,36
[M+H]+ : m/z 326 ; [M-H]- : m/z 324

### Exemple 11 : Synthèse de 2-[(5-fluoro-1-méthyl-1H-benzimidazol-2-yl)méthyl]-6-(morpholin-4-yl)pyrimidin-4(3H)-one

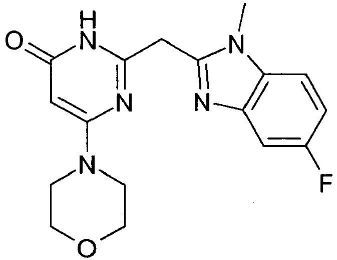

### Etape 1

A une solution de 329 mg de 2-[(6-fluoro-1H-benzimidazol-2-yl)méthyl]-6-(morpholin-4-yl)pyrimidin-4(3H)-one (exemple 1, étape 3) dans 15 mL d'acétone est ajouté 0.75 mL d'hydroxyde de sodium (2N). Après dix minutes d'agitation à une température voisine de 20°C, est ajouté 0.93 mL de iodométhane. Après une nuit, évaporer l'acétone, diluer le résidu dans 20 mL d'eau puis amener le milieu à pH 6 à l'aide d'acide chlorhydrique (2N). Après filtration de l'insoluble, le filtrat est évaporé à sec sous pression réduite et purifié par chromatographie sur colonne de silice, éluant: CH₂Cl₂/MeOH : 95/05, on obtient 145 mg d'un mélange d'isomères contenant 50% de la 2-[(5-fluoro-1-méthyl-1H-benzimidazol-2-yl)méthyl]-6-(morpholin-4-yl)pyrimidin-4(3H)-one qui est purifié dans l'étape suivante :
Spectrométrie de masse : Méthode A
Temps de rétention Tr (min) = 0,43 et 0,45 : mélange 50-50 d'isomères ; [M+H]+ : m/z 344 ; [M-H]- : m/z 342

### Etape 2

Séparation du mélange d'isomères de position du méthyl par chromatographie chirale sur Chiralpak AD 20 µm (1000 g, diamètre 80mm)
Injection : 60ml EtOH + 40ml Heptane
Phase mobile : 60% EtOH 40% Heptane 0.1 % TEA
Débit de phase mobile : 150 mL/mn
Déection U.V. : 240 nm

Après séparation des chémio-isomères, on obtient 63 mg de 2-[(5-fluoro-1-méthyl-1 H-benzimidazol-2-yl)méthyl]-6-(morpholin-4-yl)pyrimidin-4(3H)-one sous forme de cristaux beiges dont les caractéristiques sont les suivantes : Spectre RMN 1 H (400MHz): 3,31 à 3,36 (m, 4 H) ; 3,53 à 3,58 (m, 4 H) ; 3,80 (s, 3 H) ; 4,19 (s, 2 H) ; 5,21 (s, 1 H) ; 7,09 (ddd, J=2,5 et 8,8 et 9,8 Hz, 1 H) ; 7,37 (dd, J=2,5 et 9,8 Hz, 1 H) ; 7,54 (dd, J=4,8 et 8,8 Hz, 1 H) ; 11,87 (m étalé, 1 H)
Spectrométrie de masse : méthode A
Temps de rétention Tr (min) = 0,44
[M+H]+ : m/z 344 ; [M-H]- : m/z 342

### Exemple 12 : Synthèse de 2-[(7-fluoro-1,3-benzoxazol-2-yl)méthyl]-6-(morpholin-4-yl)pyrimidin-4(3H)-one

### Etape 1 :

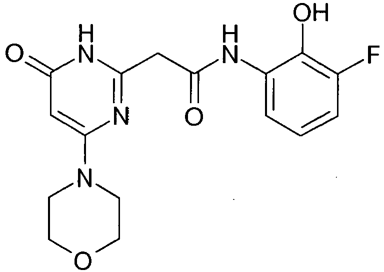

Le produit est préparé en suivant le mode opératoire décrit à l'exemple 2 étape 2 à partir de 500 mg de [4-(morpholin-4-yl)-6-oxo-1,6-dihydropyrimidin-2-yl]acétate de sodium (exemple 1 étape 2), de 357 mg de 2-amino-6-fluorophénol, de 600 mg de N-[3-(diméthylamino)propyl]-N'-éthylcarbodiimide chlorhydrate dans un mélange de 4 mL de pyridine et de 4 mL de diméthylformamide . On obtient 335 mg de N-(3-fluoro-2-hydroxyphényl)-2-[4-(morpholin-4-yl)-6-oxo-1,6-dihydropyrimidin-2-yl]acétamide sous forme d'un solide beige dont les caractéristiques sont les suivantes:
Spectre RMN 1 H (400MHz, d en ppm , DMSO-d6) : 3,44 (m, 4 H) ; 3,62 (m, 4 H) ; 3,71 (s, 2 H) ; 5,21 (s, 1 H) ; 6,78 (dt, J=6,0 et 8,1 Hz, 1 H) ; 6,94 (t large, J=8,1 Hz, 1 H) ; 7,64 (d large, J=8,1 Hz, 1 H) ; 9,55 à 10,10 (m étalé, 2 H) ; 11,69 (m étalé, 1 H)
Spectrométrie de Masse : méthode B
Temps de rétention Tr (min) = 2,66
[M+H]+ : m/z 349 ; [M-H]- : m/z 347
Point de fusion (Kofler) : supérieur à 260°C

### Etape 2 :

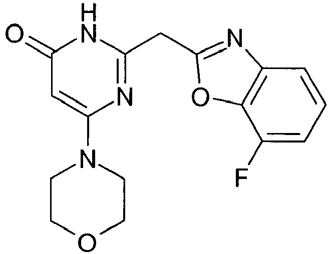

A une solution de 300 mg de N-(3-fluoro-2-hydroxyphényl)-2-[4-(morpholin-4-yl)-6-oxo-1,6-dihydropyrimidin-2-yl]acétamide dans 20 mL de xylène sont ajoutés 42 mg d'acide 4-méthylbenzènesulfonique hydrate. Après six heures de reflux (montage avec Dean-Stark), le milieu est concentré à sec sous pression réduite. Après purification par chromatographie sur colonne de silice du résidu solide, éluant : gradient de CH₂Cl₂ pur à CH₂Cl₂/MeOH : 95/05, le produit obtenu est repris par deux fois 20 mL d'oxyde de diéthyle puis essoré et séché sous vide. On obtient 110 mg de 2-[(7-fluoro-1,3-benzoxazol-2-yl)méthyl]-6-(morpholin-4-yl)pyrimidin-4(3H)-one sous forme d'une poudre blanche dont les caractéristiques sont les suivantes :
Spectre RMN 1 H (400 MHz, d en ppm , DMSO-d6): 3,34 à 3,38 (m, 4 H) ; 3,53 à 3,59 (m, 4 H) ; 4,31 (s, 2 H) ; 5,26 (s, 1 H) ; 7,30 à 7,42 (m, 2 H) ; 7,59 (dd, J=1,4 et 7,7 Hz, 1 H) ; 11,93 (m étalé, 1 H)
Spectrométrie de masse : méthode B
Temps de rétention Tr (min) = 3,11
[M+H]+ : m/z 331 ; [M-H]- : m/z 329

### Exemple 13 : Synthèse de 6-(morpholin-4-yl)-2-[(1-phényl-1H-benzimidazol-2-yl)méthyl]pyrimidin-4(3H)-one

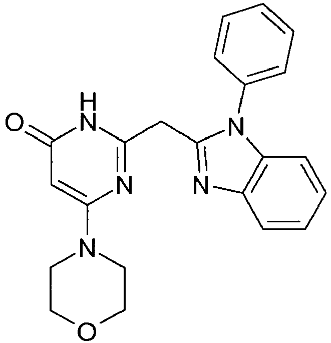

Le produit est préparé en suivant le mode opératoire décrit à l'exemple 3 à partir de 300 mg de [4-(morpholin-4-yl)-6-oxo-1,6-dihydropyrimidin-2-yl]acétate de sodium, de 423 mg de N-phénylbenzène-1,2-diamine, et de 330 mg de N-[3-(diméthylamino)propyl]-N'-éthylcarbodiimide chlorhydrate dans un mélange de 2.5 mL de pyridine et de 2.5 mL de diméthylformamide puis dans 5 mL d'acide acétique. Après purification par chromatographie sur colonne de silice, éluant: CH₂Cl₂/MeOH : 95/05, on obtient 230 mg de 6-(morpholin-4-yl)-2-[(1-phényl-1 H-benzimidazol-2-yl)méthyl]pyrimidin-4(3H)-one sous forme d'une poudre blanche dont les caractéristiques sont les suivantes :
Spectre RMN 1 H (400 MHz, d en ppm , DMSO-d6): 3,32 (m, partiellement étalé, 4 H) ; 3,56 (m, 4 H) ; 4,05 (s, 2 H) ; 5,16 (s, 1 H) ; 7,11 à 7,17 (m, 1 H) ; 7,19 à 7,30 (m, 2 H) ; 7,49 à 7,72 (m, 6 H) ; 11,74 (m étalé, 1 H) Spectrométrie de masse : méthode A
Temps de rétention Tr (min) = 0,66
[M+H]+ : m/z 388 ; [M-H]- : m/z 386

### Exemple 14 : Synthèse de 2-[(1-benzyl-1H-benzimidazol-2-yl)méthyl]-6-(morpholin-4-yl)pyrimidin-4(3H)-one

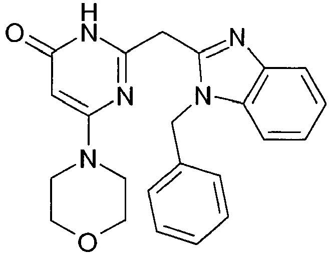

Le produit est préparé en suivant le mode opératoire décrit à l'exemple 3 à partir de 300 mg de [4-(morpholin-4-yl)-6-oxo-1,6-dihydropyrimidin-2-yl]acétate de sodium, de 455 mg de N-benzylbenzène-1,2-diamine, et de 330 mg de N-[3-(diméthylamino)propyl]-N'-éthylcarbodiimide chlorhydrate dans un mélange de 2.5 mL de pyridine et de 2.5 mL de diméthylformamide puis dans 5 mL d'acide acétique. Après quarante cinq minutes de reflux et purification par chromatographie sur colonne de silice, éluant: CH₂Cl₂/MeOH : 95/05, on obtient 250 mg de 2-[(1-benzyl-1 H-benzimidazol-2-yl)méthyl]-6-(morpholin-4-yl)pyrimidin-4(3H)-one sous forme d'une poudre crème dont les caractéristiques sont les suivantes :
Spectre RMN 1 H (400 MHz, d en ppm , DMSO-d6): 3,25 à 3,35 (m partiellement masqué, 4 H) ; 3,52 à 3,58 (m, 4 H) ; 4,18 (s, 2 H) ; 5,18 (s, 1 H) ; 5,56 (s, 2 H) ; 7,13 à 7,20 (m, 4 H) ; 7,23 à 7,35 (m, 3 H) ; 7,39 à 7,47 (m, 1 H) ; 7,55 à 7,64 (m, 1 H) ; 11,88 (m étalé, 1)
Spectrométrie de masse : méthode A
Temps de rétention Tr (min) = 0,58
[M+H]+ : m/z 402 ; [M-H]- : m/z 400

### Exemple 15 : Sythèse de 2-((1-éthyl-1H-benzimidazol-2-yl)méthyl]-6-(morpholin-4-yl)pyrimidin-4(3H)-one

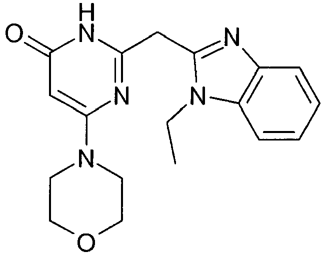

### Etape 1 :

Le produit est préparé en suivant le mode opératoire décrit à l'exemple 3 à partir de 300 mg de [4-(morpholin-4-yl)-6-oxo-1,6-dihydropyrimidin-2-yl]acétate de sodium, de 248 mg de benzène-1,2-diamine, et de 330 mg de N-[3-(diméthylamino)propyl]-N'-éthylcarbodiimide chlorhydrate dans un mélange de 2.5 mL de pyridine et de 2.5 mL de diméthylformamide puis dans 5 mL d'acide acétique. Après une heure de reflux et purification par chromatographie sur colonne de silice, éluant: CH2Cl2/MeOH : gradient de 100/0 à 90/10, on obtient 75 mg de 2-(1 H-benzimidazol-2-ylméthyl)-6-(morpholin-4-yl)pyrimidin-4(3H)-one sous forme d'une poudre beige dont les caractéristiques sont les suivantes :
Spectrométrie de masse : méthode A
Temps de rétention Tr (min) = 0,35
[M+H]+ : m/z 312 ; [M-H]- : m/z 310

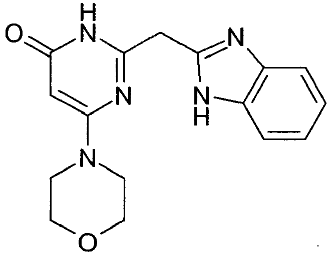

### Etape 2 :

Le produit peut être préparé comme à l'étape 1 de l'exemple 11 mais à partir de 155 mg de 2-(1 H-benzimidazol-2-ylméthyl)-6-(morpholin-4-yl)pyrimidin-4(3H)-one dans 15 mL d'acétone,de 0.375 mL d'hydroxyde de sodium (2N) et de 0.6 mL d' iodoéthane. Après purification par chromatographie sur colonne de silice, éluant: CH₂Cl₂/MeOH : 95/05, on obtient 30 mg de 2-[(1-éthyl-1H-benzimidazol-2-yl)méthyl]-6-(morpholin-4-yl)pyrimidin-4(3H)-one sous forme d'une poudre beige dont les caractéristiques sont les suivantes :
Spectre RMN 1 H (400 MHz, d en ppm , DMSO-d6): 1,31 (t, J=7,1 Hz, 3 H) ; 3,34 (m, 4 H) ; 3,57 (m, 4 H) ; 4,18 (s, 2 H) ; 4,31 (q, J=7,1 Hz, 2 H) ; 5,21 (s, 1 H) ; 7,13 à 7,19 (m, 1 H) ; 7,20 à 7,25 (m, 1 H) ; 7,51 à 7,59 (m, 2 H) ; 11,88 (m étalé, 1H)
Spectrométrie de masse : méthode A
Temps de rétention Tr (min) = 0,42
[M+H]+ : m/z 340 ; [M-H]- : m/z 338

### Exemple 16 : Synthèse de 2-[(5,6-difluoro-1,3-benzoxazol-2-yl)méthyl]-6-(morpholin-4-yl)pyrimidin-4(3H)-one

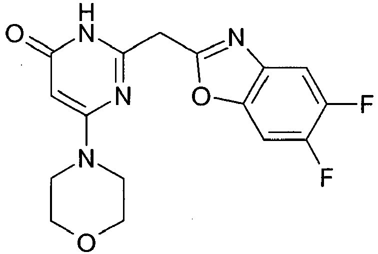

### Etape 1 :

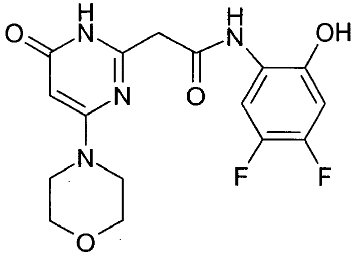

Le produit est préparé en suivant le mode opératoire décrit à l'étape 1 de l'exemple 2 mais à partir de 500 mg de [4-(morpholin-4-yl)-6-oxo-1,6-dihydropyrimidin-2-yl]acétate de sodium (exemple 1 étape 2), de 425 mg de 2-amino-4,5-difluorophénol, de 600 mg de N-[3-(diméthylamino)propyl]-N'-éthylcarbodiimide chlorhydrate dans un mélange de 4 mL de pyridine et de 4 mL de diméthylformamide . On obtient 500 mg de N-(4,5-difluoro-2-hydroxyphényl)-2-[4-(morpholin-4-yl)-6-oxo-1,6-dihydropyrimidin-2-yl]acétamide sous forme d'un solide marron dont les caractéristiques sont les suivantes:
Spectrométrie de masse : méthode A
Temps de rétention Tr (min) = 0,68
[M+H]+ : m/z 367 ; [M-H]- : m/z 365

### Etape 2 :

Le produit peut être préparé comme décrit à l'étape 2 de l'exemple 12 mais à partir de 465 mg de N-(4,5-difluoro-2-hydroxyphényl)-2-[4-(morpholin-4-yl)-6-oxo-1 ,6-dihydropyrimidin-2-yl]acétamide dans 30 mL de xylène et de 77 mg d'acide 4-méthylbenzènesulfonique hydrate. Après six heures de reflux, ajout de 100 mg d'acide 4-méthylbenzènesulfonique hydrate et purification par chromatographie sur colonne de silice, éluant : gradient de CH₂Cl₂ pur à CH₂Cl₂/MeOH : 96/04, le produit obtenu est repris par 20 mL d'oxyde de diéthyle puis essoré et séché sous vide. On obtient 63 mg de 2-[(5,6-difluoro-1,3-benzoxazol-2-yl)méthyl]-6-(morpholin-4-yl)pyrimidin-4(3H)-one sous forme d'un solide blanc dont les caractéristiques sont les suivantes :
Spectre RMN 1 H (400 MHz, d en ppm , DMSO-d6): 3,35 (m, 4 H) ; 3,56 (m, 4 H) ; 4,26 (s, 2 H) ; 5,24 (m étalé, 1 H) ; 7,91 (dd, J=7,6 et 10,3 Hz, 1 H) ; 8,04 (dd, J=7,0 et 9,9 Hz, 1 H); 11,88 (m étalé, 1 H)
Spectrométrie de masse : méthode A
Temps de rétention Tr (min) = 0,68
[M+H]+ : m/z 349 ; [M-H]- : m/z 347

### Exemple 17 : Synthèse de 2-[(6-chloro-1H-benzimidazol-2-yl)méthyl]-6-(morpholin-4-yl)pyrimidin-4(3H)-one

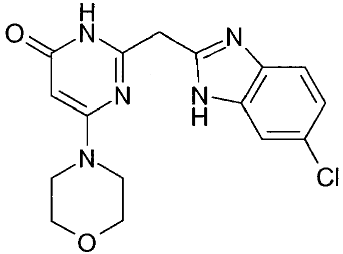

Le produit est préparé en suivant le mode opératoire décrit à l'exemple 3 à partir de 400 mg de [4-(morpholin-4-yl)-6-oxo-1,6-dihydropyrimidin-2-yl]acétate de sodium, de 436 mg de 4-chlorobenzène-1,2-diamine, et de 440 mg de N-[3-(diméthylamino)propyl]-N'-éthylcarbodiimide chlorhydrate dans un mélange de 3.5 mL de pyridine et de 3.5 mL de diméthylformamide puis dans 7 mL d'acide acétique. Après trente minutes de reflux et purification par chromatographie sur colonne de silice, éluant: CH₂Cl₂/MeOH : gradient de 100/0 à 90/10, on obtient 260 mg de 2-[(6-chloro-1 H-benzimidazol-2-yl)méthyl]-6-(morpholin-4-yl)pyrimidin-4(3H)-one sous forme d'une poudre blanche dont les caractéristiques sont les suivantes :
Spectre RMN 1 H (400 MHz, d en ppm , DMSO-d6): 3,37 (m, 4 H); 3,57 (m, 4 H) ; 4,10 (s, 2 H) ; 5,22 (s, 1 H) ; 7,17 (dd, J=1,9 et 8,6 Hz, 1 H) ; 7,51 (d, J=8,6 Hz, 1 H) ; 7,57 (d, J=1,9 Hz, 1 H) ; 12,18 (m étalé, 2 H)
Spectrométrie de masse : méthode A
Temps de rétention Tr (min) = 0,51
[M+H]+ : m/z 346 ; [M-H]- : m/z 344

### Exemple 18 : Synthèse de 2-[(1-cyclopropyl-5-fluoro-1H-benzimidazol-2-yl)méthyl]-6-(morpholin-4-yl)pyrimidin-4(3H)-one

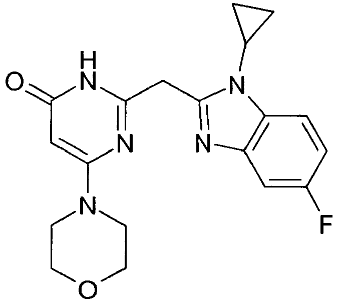

### Etape 1 :

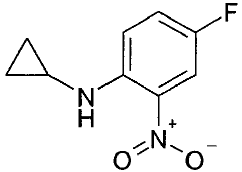

Dans un réacteur pour four micro-ondes sont introduits 795 mg de 1,4-difluoro-2-nitrobenzène dans 10 mL de tétrahydrofurane. Sont ajoutés 1 mL de N-éthyl-N-(propan-2-yl)propan-2-amine et 0.416 mL de cyclopropanamine. Après trente minutes à une température 110°C sous irradiation micro-ondes, puis une heure vingt à une température de 120°C, est ajouté 0.1 mL de cyclopropanamine puis le milieu est placé à nouveau trente minutes à une température de 130°C sous irradiation micro-ondes. Après concentration à sec sous pression réduite on obtient 960 mg de N-cyclopropyl-4-fluoro-2-nitroaniline sous forme d'une poudre orange qui sera utilisé tel que dans l'étape suivante.

### Etape 2 :

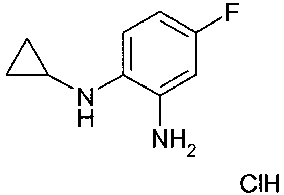

A une solution de 950 mg de N-cyclopropyl-4-fluoro-2-nitroaniline dans 15 mL d'acide acétique sont ajoutés lentement 1.58 g de zinc. Après une heure trente d'agitation à une température voisine de 20°C, on ajoute 25 mL d'eau au milieu et ajuste le pH à 7 à l'aide d'une solution d'ammoniaque aqueuse à 28%. La phase aqueuse est extraite par trois fois 30 mL d'acétate d'éthyle, les phases organiques réunies sont séchées sur sulfate de magnésium anhydre, filtrées et concentrées à sec sous pression réduite. Le résidu huileux est repris dans 100 mL d'oxyde de diéthyle et ajoute une solution aqueuse d'acide chlorhydrique (2N) jusqu'à formation d'un précipité. Le solide est essoré puis séché sous cloche à vide et on obtient 960 mg de N∼1∼cyclopropyl-4-fluorobenzène-1,2-diamine chlorhydrate sous forme d'une poudre violette dont les caractéristiques sont les suivantes :
Spectrométrie de masse : méthode A
Temps de rétention Tr (min) = 0,62
[M+H]+ : m/z 167

### Etape 3 :

Le produit est préparé en suivant le mode opératoire décrit à l'exemple 3 à partir de 300 mg de [4-(morpholin-4-yl)-6-oxo-1,6-dihydropyrimidin-2-yl]acétate de sodium, de 645 mg de N∼1∼-cyclopropyl-4-fluorobenzène-1,2-diamine chlorhydrate, et de 330 mg de N-[3-(diméthylamino)propyl]-N'-éthylcarbodiimide chlorhydrate dans un mélange de 3 mL de pyridine et de 3 mL de diméthylformamide puis dans 5 mL d'acide acétique. Après deux heures de reflux et purification par chromatographie sur colonne de silice, éluant: CH₂Cl₂/MeOH : 95/05, le résidu obtenu est purifié par HPLC / MS préparative (Méthode 1). Après évaporation de l'acétonitrile le pH est ammené à 8 à l'aide de bicarbonate de sodium (solide) puis la phase aqueuse est extraite par deux fois 25 mL d'acétate d'éthyle, les phases organiques réunies sont séchées sur sulfate de magnésium anhydre, filtrées et concentrées à sec sous pression réduite. On obtient 15 mg de 2-[(1-cyclopropyl-5-fluoro-1 H-benzimidazol-2-yl)méthyl]-6-(morpholin-4-yl)pyrimidin-4(3H)-one sous forme d'une poudre blanche dont les caractéristiques sont les suivantes :
Spectre RMN 1 H (400 MHz, d en ppm , DMSO-d6): 1,04 à 1,22 (m, 4 H) ; 3,33 à 3,43 (m, 5 H) ; 3,54 à 3,60 (m, 4 H) ; 4,24 (s, 2 H) ; 5,22 (s, 1 H) ; 7,09 (dt, J=2,4 et 9,3 Hz, 1 H) ; 7,39 (dd, J=2,4 et 9,8 Hz, 1 H) ; 7,56 (dd, J=4,8 et 9,3 Hz, 1 H) ; 11,80 (m étalé, 1 H)
Spectrométrie de masse : méthode A
Temps de rétention Tr (min) = 0,57
[M+H]+ : m/z 370 ; [M-H]- : m/z 368

### Exemple 19 : Synthèse de 2-{[5-fluoro-1-(2-méthoxyéthyl)-1H-benzimidazol-2-yl]méthyl}-6-(morpholin-4-yl)pyrimidin-4(3H)-one

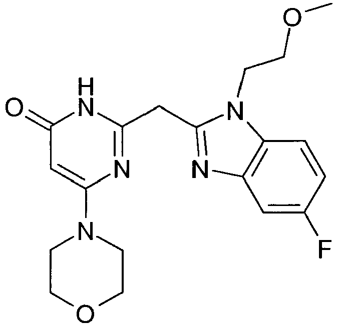

### Etape 1:

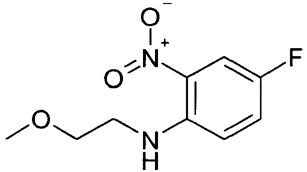

Le prosuit peut être préparé comme décrit à l'étape 1 de l'exemple 18 mais à partir de 795 mg de 1,4-difluoro-2-nitrobenzène dans 10 mL de tétrahydrofurane, de 1 mL de N-éthyl-N-(propan-2-yl)propan-2-amine et de 0.655 mL de 2-méthoxyéthanamine. Après une heure à une température 130°C sous irradiation micro-ondes on obtient 900 mg de 4-fluoro-N-(2-méthoxyéthyl)-2-nitroaniline sous forme d'une huile rouge qui sera utilisée telle que dans l'étape suivante.

### Etape 2 :

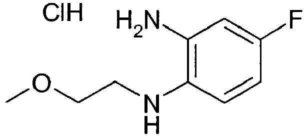

Le produit peut être préparé comme décrit à l'étape 2 de l'exemple 18 mais à partir de 880 mg de 4-fluoro-N-(2-méthoxyéthyl)-2-nitroaniline dans 11 mL d'acide acétique et de 1.34 g de zinc. Après trois heures d'agitation à une température voisine de 20°C, sont ajoutés 536 mg de zinc. On obtient 850 mg de 4-fluoro-N∼1∼-(2-méthoxyéthyl)benzène-1,2-diamine chlorhydrate sous forme d'une huile rouge qui sera utilisée telle que dans l'étape suivante.

### Etape 3 :

Le produit est préparé en suivant le mode opératoire décrit à l'exemple 3 à partir de 300 mg de [4-(morpholin-4-yl)-6-oxo-1,6-dihydropyrimidin-2-yl]acétate de sodium, de 760 mg de 4-fluoro-N∼1∼-(2-méthoxyéthyl)benzène-1,2-diamine chlorhydrate, et de 330 mg de N-[3-(diméthylamino)propyl]-N'-éthylcarbodiimide chlorhydrate dans un mélange de 5 mL de pyridine et de 5 mL de diméthylformamide puis dans 5 mL d'acide acétique. Après une heure de reflux et purification par chromatographie sur colonne de silice, éluant: CH₂Cl₂/MeOH : 95/05, le résidu obtenu est purifié par HPLC / MS préparative (Méthode 1). Après évaporation de l'acétonitrile, le pH est ammené à 8 à l'aide de bicarbonate de sodium (solide) puis la phase aqueuse est extraite par deux fois 25 mL d'acétate d'éthyle, les phases organiques réunies sont séchées sur sulfate de magnésium anhydre, filtrées et concentrées à sec sous pression réduite. On obtient 120 mg de 2-{[5-fluoro-1-(2-méthoxyéthyl)-1 H-benzimidazol-2-yl]méthyl}-6-(morpholin-4-yl)pyrimidin-4(3H)-one sous forme d'une poudre blanche dont les caractéristiques sont les suivantes :
Spectre RMN 1 H (400 MHz, d en ppm , DMSO-d6): 3,20 (s, 3 H) ; 3,33 à 3,40 (m, 4 H) ; 3,54 à 3,59 (m, 4 H) ; 3,63 (t, J=5,3 Hz, 2 H) ; 4,19 (s, 2 H) ; 4,47 (t, J=5,3 Hz, 2 H) ; 5,21 (s, 1 H) ; 7,08 (dt, J=2,4 et 9,3 Hz, 1 H) ; 7,37 (dd, J=2,4 et 9,8 Hz, 1 H) ; 7,57 (dd, J=4,8 et 9,3 Hz, 1 H) ; 11,80 (m étalé, 1 H)
Spectrométrie de masse : méthode A
Temps de rétention Tr (min) = 0,51
[M+H]+ : m/z 388 ; [M-H]- : m/z 386

### Exemple 20 : Synthèse du 6-(morpholin-4-yl)-2-(naphtho[2,1-d][1,3]oxazol-2-ylméthyl)pyrimidin-4(3H)-one

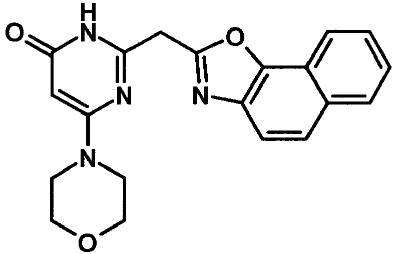

### Etape 1

A une solution de 40 mg de 3-amino-2-naphtol dans 2 mL de diméthylformamide, sont ajoutés 65 mg de [4-(morpholin-4-yl)-6-oxo-1,6-dihydropyrimidin-2-yl]acétate de sodium, 104 mg de 2-(1 H-Benzotriazole-1-yl)-1,1,3,3-Tetramethyluronium hexafluorophosphate (HBTU) et 82 µL de N-méthylmorpholine. Le mélange réactionnel est chauffé pendant 1 heure à 110°C puis après refroidissement, 0.1 mL de TFA est ajouté et le précipité est purifié par chromatographie. Le N-(2-naphtol)-2-[4-(morpholin-4-yl)-6-oxo-1,6-dihydropyrimidin-2-yl]acétamide obtenu est utilisé tel quel dans l'étape suivante.

### Etape 2 :

Le N-(2-naphtol)-2-[4-(morpholin-4-yl)-6-oxo-1,6-dihydropyrimidin-2-yl]acétamide obtenu à l'étape précédente est dissous dans 2 mL d'acide trifluoroacétique et irradié aux micro-ondes pendant 30 minutes à 120°C. 8 mL de toluène sont ajoutés et la solution est concentrée à sec. Après purification par chromatographie, on obtient 4 mg de 6-(morpholin-4-yl)-2-(naphtho[2,1-d][1,3]oxazol-2-ylméthyl)pyrimidin-4(3H)-one dont les caractéristiques sont les suivantes :
Spectre RMN 1 H (400MHz, d en ppm, DMSO-d6) : 3,31 à 3,48 (m partiellement masqué, 4 H) ; 3,54 à 3,64 (m, 4 H) ; 4,39 (s, 2 H) ; 5,26 (m étalé, 1 H) ; 7,63 (t, J=8,0 Hz, 1 H) ; 7,73 (t, J=8.0 Hz, 1 H) ; 7,87 (d, J=9,0 Hz, 1 H) ; 7,94 (d, J=9,0 Hz, 1 H) ; 8,14 (d, J=8,0 Hz, 1 H) ; 8,21 (d, J=8,0 Hz, 1 H) ; 12,00 (m étalé, 1 H)
Spectrométrie de masse : méthode A
Temps de rétention Tr (min) = 1,02
[M+H]+ : m/z 363 ; [M-H]- : m/z 361

### Exemple 21 : Synthèse du 2-[(6-methoxy-1,3-benzoxazol-2-yl)méthyl]-6-(morpholin-4-yl)pyrimidin-4(3H)-one

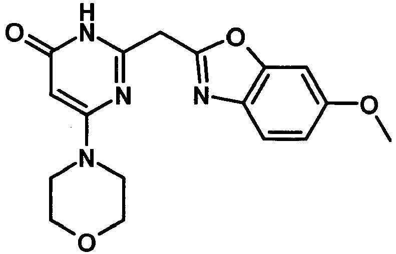

### Etape 1 :

A une solution de 1 g de [4-(morpholin-4-yl)-6-oxo-1,6-dihydropyrimidin-2-yl]acétate de sodium dans 6 mL de diméthylformamide sont ajoutés 4 mL de pyridine, 1,2 g de N-[3-(diméthylamino)propyl]-N'-éthylcarbodiimide chlorhydrate et 940 mg de 2-hydroxy-4-méthoxyaniline. Le mélange réactionnel est agité à température ambiante pendant 2 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 1,2g du N-(4-fluoro-2-hydroxyphényl)-2-[4-(morpholin-4-yl)-6-oxo-1,6-dihydropyrimidin-2-yl]acétamide sous forme de solide marron qui est utilisé tel quel dans l'étape suivante.
Spectrométrie de Masse : méthode B
Temps de rétention Tr (min) = 2,55
[M+H]+ : m/z 361 ; [M-H]- : m/z 359

### Etape 2 :

On mélange 500 mg de du N-(4-fluoro-2-hydroxyphényl)-2-[4-(morpholin-4-yl)-6-oxo-1,6-dihydropyrimidin-2-yl]acétamide dans 40 mL de xylène et on ajoute 155 mg d'hydrate d'acide 4-méthylbenzènesulfonique. Le mélange réactionnel est porté au reflux pendant 3 heures. Après refroidissement à 20°C, le mélange réactionnel est concentré à sec sous pression réduite puis le résidu est chromatographié sur colonne de gel de silice, éluant : CH2Cl2/MeOH : 95/05. On obtient ainsi 220 mg de 2-[(6-méthoxy-1,3-benzoxazol-2-yl)méthyl]-6-(morpholin-4-yl)pyrimidin-4(3H)-one sous forme de solide blanc dont les caractéristiques sont les suivantes :
Spectre RMN 1 H (400MHz): 3,36 (m, 4 H) ; 3,57 (m, 4 H) ; 3,81 (s, 3 H) ; 4,19 (s, 2 H) ; 5,24 (s, 1 H) ; 6,95 (dd, J=2,2 et 8,8 Hz, 1 H) ; 7,33 (d, J=2,2 Hz, 1 H) ; 7,58 (d, J=8,8 Hz, 1 H) ; 11,89 (m étalé, 1 H)
Spectrométrie de Masse : méthode A
Temps de rétention Tr (min) = 0,64
[M+H]+ : m/z 343 ; [M-H]- : m/z 341

### Exemple 22 : Synthèse du 2-((6,7-difluoro-1,3-benzoxazol-2-yl)méthyl]-6-(morpholin-4-yl)pyrimidin-4(3H)-one

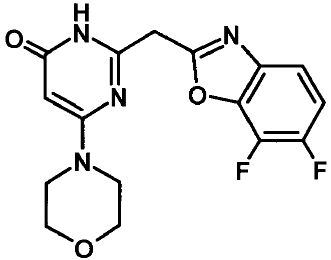

### Etape 1

A une solution de 32 mg de 6-amino-2,3-difluorophénol dans 2 mL de diméthylformamide, sont ajoutés 65 mg de [4-(morpholin-4-yl)-6-oxo-1,6-dihydropyrimidin-2-yl]acétate de sodium, 284 mg de 2-(1 H-Benzotriazole-1-yl)-1,1,3,3-Tetramethyluronium hexafluorophosphate (HBTU) et 82 µL de N-méthylmorpholine. Le mélange réactionnel est chauffé pendant 1 heure à 110°C puis après refroidissement, 0.1 mL de TFA est ajouté et le précipité est purifié par chromatographie. Le N-(3,4-fluoro-2-hydroxyphényl)-2-[4-(morpholin-4-yl)-6-oxo-1,6-dihydropyrimidin-2-yl]acétamide obtenu est utilisé tel quel dans l'étape suivante.

### Etape 2 :

Le N-(3,4-fluoro-2-hydroxyphényl)-2-[4-(morpholin-4-yl)-6-oxo-1,6-dihydropyrimidin-2-yl]acétamide obtenu à l'étape précédente est dissous dans 2 mL d'acide trifluoroacétique et irradié aux micro-ondes pendant 30 minutes à 120°C. 8 mL de toluène sont ajoutés et la solution est concentrée à sec. Après purification par chromatographie, on obtient 4 mg de 2-[(6,7-difluoro-1,3-benzoxazol-2-yl)méthyl]-6-(morpholin-4-yl)pyrimidin-4(3H)-one dont les caractéristiques sont les suivantes :
Spectre RMN 1 H (400MHz, d en ppm, DMSO-d6) : 3,26 à 3,47 (m partiellement masqué, 4 H) ; 3,54 à 3,71 (m, 4 H) ; 4,32 (s, 2 H) ; 5,25 (m étalé, 1 H) ; 7,50 (m, 1 H) ; 7,63 (m, 1 H) ; 11,96 (m étalé, 1 H)
Spectrométrie de Masse : méthode A
Temps de rétention Tr (min) = 0,97
[M+H]+ : m/z 349 ; [M-H]- : m/z 347

### Procédure générique utilisée pour les exemples 23 à 35

Couplage : 1 équivalent de diamine est pesé et placé dans un réacteur. 1 ml d'une suspension contenant 1 équivalent du sel de sodium de l'acide (4-Morpholine-4-yl-6-oxo-1,6-dihydro-pyrimidin-2-yl)-acétique, obtenu à l'étape 2 de l'exemple 1 3 équivalents de N-méthyl morpholine (109-02-4) dans le diméthyleformamide est alors ajouté, suivi par 1.1 équivalent de HBTU (94790-37-1) en solution dans le N,N-diméthyleformamide. Le réacteur est alors fermé et agité à 110°C pendant une heure. Apres refroidissement à température ambiante, 0.1 ml d'acide trifluoroacétique est ajouté au milieu réactionnel qui est alors filtré. Le filtrat ainsi obtenu est purifié par HPLC préparative.

Cyclisation : Le composé purifié obtenu précédemment est dissout dans 2 ml d'acide acétique glacial, puis chauffé à 100°C pendant 3 heures. La solution ainsi obtenue est évaporée ; le résidu est repris dans 2 ml d'un mélange de diméthyleformamide et d'acide trifluoroacétique (19/1) puis filtré ; le filtrat est purifié par HPLC préparative.

Le protocole en deux étapes décrit ci-dessus est appliqué sur une échelle de 0.25mmol à la série de diamines suivantes (colonne diamine) conduisant aux composés ci-dessous (exemples 23 à 35).

Les composés sont caractérisés par analyse LCMS méthode C

| exemple | Diamine | composé obtenu | MH⁺ | Temps de ¹ rétention (min.) | Pureté (%) |
|---|---|---|---|---|---|
| 23 | 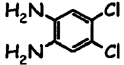 | 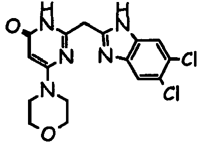 | 380.01 | 0.96 | 98 |
| | CAS 95-54-5 | 2-[(5,6-dichloro-1H-benzimidazol-2-yl)méthyle]-6-(morpholin-4-yl)pyrimidin-4(3H)-one | | | |
| 24 | 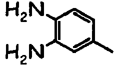 | 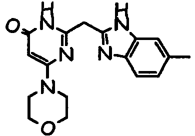 | 326.13 | 0.74 | 100 |
| | CAS : 496-72-0 MFCD00007728, 3-4-diamino toluene | 2-[(6-méthyle-1H-benzimidazol-2-yl)méthyle]-6-(morpholin-4-yl)pyrimidin-4(3H)-one | | | |
| 25 | 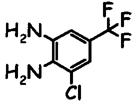 | 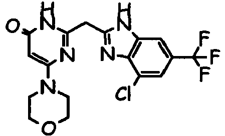 | 141.04 | 1.02 | 100 |
| | 132915-80-1 MFCD00042198 3-chloro-5-trifluorométhyle-1,2-phenylenediamine | 2-{[4-chloro-6-(trifluorométhyle)-1H-benzimidazol-2-yl]méthyle}-6-(morpholin-4-yl)pyrimidin-4(3H)-one | | | |
| 26 | 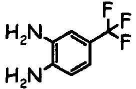 | 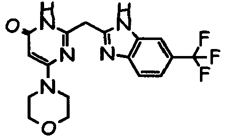 | 380.05 | 0.93 | 100 |
| | 368-71-8 MFCD00042456 4-(trifluorométhyle)-1,2-phenylenediamine | 6-(morpholin-4-yl)-2-{[6-(trifluorométhyle)-1H-benzimidazol-2-yl]méthyle}pyrimidin-4(3H)-one | | | |
| 27 | 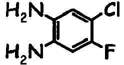 | 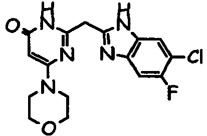 | 364.01 | 0.91 | 100 |
| | MFCD00042485 4-chloro-5-fluoro-o-phenylenediamine | 2-[(5-chloro-6-fluoro-1 H-benzimidazol-2-yl)méthyle]-6-(morpholin-4-yl)pyrimidin-4(3H)-one | | | |
| 28 | 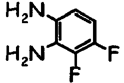 | 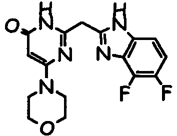 | 348.08 | 0.88 | 100 |
| | MFCD00153128 1,2-diamino-3,4-difluorobenzène | 2-[(6,7-difluoro-1H-benzimidazol-2-yl)méthyle]-6-(morpholin-4-yl)pyrimidin-4(3H)-one | | | |
| 29 | 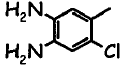 | 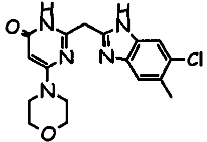 | 360.06 | 0.88 | 100 |
| | MFCD00221471 63155-04-4 4-chloro-5-méthylebenzène-1,2-diamine | 2-[(5-chloro-6-méthyle-1H-benzimidazol-2-yl)méthyle]-6-(morpholin-4-yl)pyrimidin-4(3H)-one | | | |
| 30 | 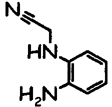 | 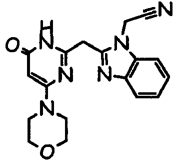 | 351.1 | 0.85 | 100 |
| | MFCD00234427 [(2-aminophenyl)amino]-acetonitrile | (2-{[4-(morpholin-4-yl)-6-oxo-1,6-dihydropyrimidin-2-yl]méthyle}-1H-benzimidazol-1-yl)acetonitrile | | | |
| 31 | 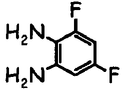 | 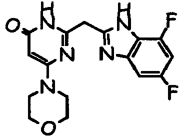 | 348.06 | 0.88 | 100 |
| | MFCD00973899 1,2-diamino-3,5-difluorobenzène | 2-[(5,7-difluoro-1H-benzimidazol-2-yl)méthyle]-6-(morpholin-4-yl)pyrimidin-4(3H)-one | | | |
| 32 | 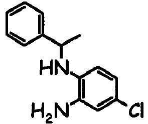 | 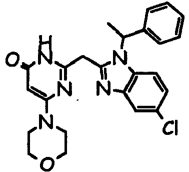 | 450.15 | 1.08 | 98 |
| | MFCD02029653 4-chloro-1-n-(1-phenylethyl)benzène-1,2-diamine | 2-{[5-chloro-1-(1-phenylethyl)-1H-benzimidazol-2-yl]méthyle}-6-(morpholin-4-yl)pyrimidin-4(3H)-one | | | |
| 33 | 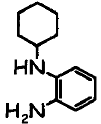 | 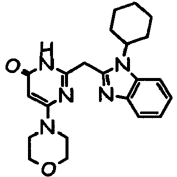 | 394.19 | 0.92 | 100 |
| | MFCD01120262 nl-cyclohexyl-1,2-benzènediamine | 2-[(1-cyclohexyl-1H-benzimidazol-2-yl)méthyle]-6-(morpholin-4-yl)pyrimidin-4(3H)-one | | | |
| 34 | 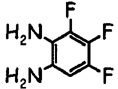 | 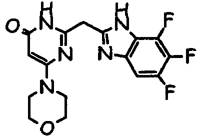 | 366.03 | 0.93 | 100 |
| | MFCD01569524 1,2-diamino-3,4,5-trifluorobenzène | 6-(morpholin-4-yl)-2-[(5,6,7-trifluoro-1H-benzimidazol-2-yl)méthyle]pyrimidin-4(3H)-one | | | |
| 35 | 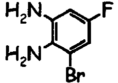 | 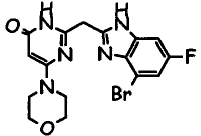 | 407.97 | 0.93 | 100 |
| | MFCD08741398 3-bromo-1,2-diamino-5-fluorobenzène | 2-[(4-bromo-6-fluoro-1H-benzimidazol-2-yl)méthyle]-6-(morpholin-4-yl)pyrimidin-4(3H)-one | | | |

### Exemple 36 : 2-[(6-bromo-1-méthyle-1H-benzimidazol-2-yl)méthyle]-6-(morpholin-4-yl)pyrimidin-4(3H)-one

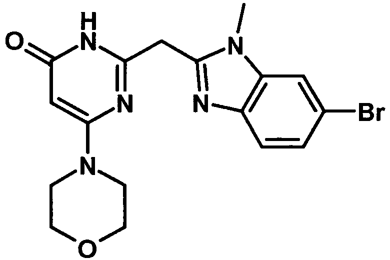

### Etape 1 :

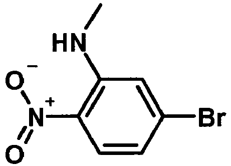

une solution de 4-bromo-2-fluoronitro benzène (1.2g) dans le THF (10 ml) est chauffée en présence de N,N-diisopropyle éthyle amine (690µl) et de méthylamine (2M dans le THF, 3 ml) dans un réacteur microondes (10°C, 20 min). Apres 20 min, on ajoute 100µl de Methyle amine THF (2M) puis on chauffe à 105°C pendant 5 minutes additionelles. Le milieu réactionnel est concentré puis repris dans l'acétate d'éthyle, lavé à l'eau puis à la saumure. Les extraits organiques sont réunis, séchés sur sulfate de magnésium, filtrés et évaporés sous pression réduite. On isole la (5-Bromo-2-nitro-phenyl)-methyl-amine (1.2g), solide orange que l'on utilise tel quel dans l'étape suivante.

### Etape 2 :

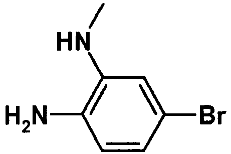

Une solution de (5-Bromo-2-nitro-phenyl)-methyl-amine préparée comme ci dessus (1.2g) dans l'acide acétique (20 ml) est traitée portion par portion par de la poudre de Zinc (1.72 mg). Le milieu réactionnel est agité à température ambiante (20°C) pendant 90min. On ajoute de l'eau, de l'ammoniac 28% jusqu'à pH 7 puis on épuise le milieu réactionnel par l'acétate d'éthyle. Les extraits organiques sont réunis, lavés à l'eau et à la saumure, séchés sur sulfate de magnésium, filtrés et évaporés sous pression réduite. L'huile obtenue est reprise dans l'éther puis ajoutée par HCl 1N. Le solide formé après évaporation est isolé et séché. On obtient le chlorhydrate de 4-b romo-N2-méthyl-benzene-1,2-diamine (1.2g), que l'on utilise tel quel dans l'étape suivante.

### Etape 3 :

Dans un ballon on place le chlorhydrate de 4-bromo-N2-méthyl-benzene-1,2-diamine préparée ci-dessus (1.2g) dans un mélange de DMF (10 ml) et de pyridine (10 ml) sous agitation magnétique puis on ajoute le chlorhydrate de N-(3-diméthyle aminopropyl)-N'-ethylcarbodiimide (968mg) puis le sel de sodium de l'acide (4-Morpholine-4-yl-6-oxo-1,6-dihydro-pyrimidin-2-yl)-acétique, obtenu à l'étape 2 de l'exemple 1 (1.2g). Le mélange ainsi obtenu est agité à température ambiante (20°C) pendant une nuit. Après évaporation du milieu réactionnel, on ajoute de l'acide acétique glacial (20ml) et on porte au reflux (90min). Le milieu réactionnel est concentré, repris dans le dichlorométhane et de l'eau à pH8 (soude 2N), pour former un solide qui est filtré et lavé au dichlorométhane. Le solide obtenu est dissout dans du méthanol, adsorbé sur silice (7g) et purifié par chromatographie. Purification sur colonne de silice (50g) en éluant avec un mélange de dichlorométhane et de dichlorométhane/MeOH 9/1.(avec CV=75 ml ; Gradient : 4 CV DCM ; 4 CV de 0 à 16% DCM/MeOH 9/1 ; 8 CV de 16 à 20% DCM/MeOH 9/1; 1 CV de 20 à 30% DCM/MeOH 9/1.) Les fractions contenant le composé attendu sont réunies et évaporées sous pression réduite. On isole 900mg de 2-[(6-bromo-1-méthyle-1 H-benzimidazol-2-yl)méthyle]-6-(morpholin-4-yl)pyrimidin-4(3H)-one dont les caractéristiques sont les suivantes :
Spectre RMN 1 H (400MHz): 3,33 (m, 4 H) ; 3,57 (m, 4 H) ; 3,80 (s, 3 H) ; 4,20 (s,2 H) ; 5,21 (s, 1 H) ; 7,31 (dd, J=1,7 et 8,6 Hz, 1 H) ; 7,52 (d, J=8,6 Hz, 1 H) ; 7,83 (d, J=1,7 Hz, 1 H) ;11,81 (m étalé, 1 H)
Spectrométrie de Masse : méthode B
Temps de rétention Tr (min) = 2,91;
[M+H]+ : m/z 404 ; [M-H]- : m/z 402

### Exemple 37 : 2-[(6,7-difluoro-1-méthyle-1H-benzimidazol-2-yl)méthyle]-6-(morpholin-4-yl)pyrimidin-4(3H)-one ;

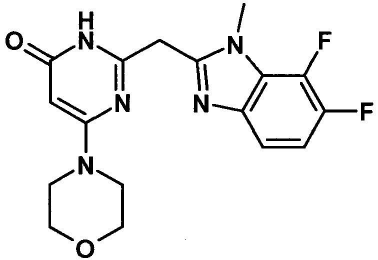

### Etape 1 :

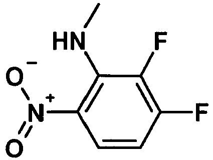

Une solution de 2,3-difluoro-6-nitro aniline (3g) dans le toluène (9.2 ml) est traitée par du sulfate de diméthyle (2.6g) et de la soude 2N (17.3 ml) en présence de bromure de tétrabutyle ammonium (0.33g) sous agitation à température ambiante (20°C) pendant 24heures. Le milieu réactionnel est versé sur de l'HCl 2N (150 ml) puis on l'épuise avec du dichlorométhane (300ml). Les extraits organiques sont réunis, séchés sur sulfate de magnésium, filtrés et évaporés sous pression réduite. Le composé obtenu est purifié par chromatographie sur gel de silice (300g g silice,) en éluant avec un mélange de heptane / acétate d'éthyle 85/15. Les fractions contenant le composé attendu sont réunies et évaporées sous pression réduite. On isole la (2,3-Difluoro-6-nitro-phényle)-méthyle-amine désirée que l'on utilise dans l'étape suivante (1,7g).

### Etape 2 :

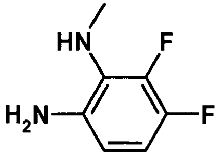

Une solution de (2,3-Difluoro-6-nitro-phényle)-méthyle-amine (500mg) dans l'acide acétique est traitée portion par portion par de la poudre de Zinc (869mg). Le milieu réactionnel est agité à température ambiante (20°C) jusqu'à réduction complète. On ajoute de l'eau (13ml), de l'ammoniac 28% jusqu'à pH 7 puis on épuise le milieu réactionnel par l'acétate d'éthyle (3x50). Les extraits organiques sont réunis, séchés sur sulfate de magnésium, filtrés et évaporés sous pression réduite. L'huile obtenue est reprise dans l'éther (50ml) puis traitée avec HCl 2N. Le solide formé est filtré. On isole la 3,4-difluoro-N1-méthyle-benzène-1,2-diamine sous sa forme chlorhydrate (510mg), que l'on utilise dans l'étape suivante.

### Etape 3 :

A une solution de 242.3 mg de l'amine préparée ci dessus dans un mélange de DMF (1.75 ml) et de pyridine (1.75 ml) sous agitation magnétique sont ajoutés le chlorhydrate de N-(3-diméthyleaminopropyl)-N'-ethylcarbodiimide (220.3 mg) puis le sel de sodium de l'acide (4-Morpholine-4-yl-6-oxo-1,6-dihydro-pyrimidin-2-yl)-acétique, obtenu à l'étape 2 de l'exemple 1 (200mg). Le mélange ainsi obtenu est agité à température ambiante (20°C) pendant une nuit. Après évaporation du milieu réactionnel, on ajoute 3.5 ml d'acide acétique glacial et on porte au reflux jusqu'à obtention du composé de cyclisation. Le milieu réactionnel est alors dilué à l'eau (20 ml) puis ajouté de soude 2N jusqu'à pH 7 ; le milieu réactionnel est extrait à l'acétate d'éthyle (3 x 20 ml). Les extraits organiques sont réunis, séchés sur sulfate de magnésium, filtrés et évaporés sous pression réduite. Le composé obtenu est lavé au dichlorométhane puis séché. On isole 20.8mg de 2-[(6,7-difluoro-1-méthyle-1H-benzimidazol-2-yl)méthyle]-6-(morpholin-4-yl)pyrimidin-4(3H)-one dont les caractéristiques sont les suivantes :
Spectre RMN 1 H (400MHz): 3,33 (m, 4 H) ; 3,57 (m, 4 H) ; 3,94 (s, 3 H) ; 4,19 (s,2 H) ; 5,21 (s, 1 H) ; 7,20 (ddd, J=7,5 et 8,8 et 11,7 Hz, 1 H) ; 7,38 (dd, J=3,3 et 8,8 Hz, 1 H) ; 11,81 (métalé, 1 H)
Spectrométrie de Masse : méthode A
Temps de rétention Tr (min) = 0,65 ;
[M+H]+ : m/z 362 ; [M-H]- : m/z 360

### Exemple 38 : 2-[(5,6-dichloro-1-méthyle-1H-benzimidazol-2-yl)méthyle]-6-(morpholin-4-yl)pyrimidin-4(3H)-one

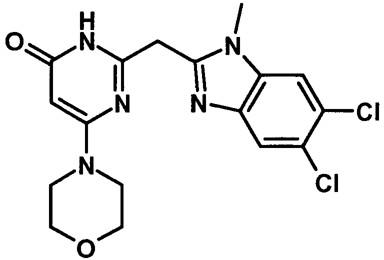

### Etape 1 :

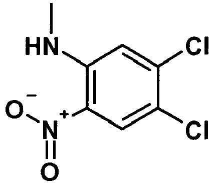

Une solution de 4,5-dichloro-2-nitro aniline (3g) dans le toluène (9.2 ml) est traitée par du sulfate de diméthyle (2.2g) et de la soude 2N (14.5 ml) en présence de bromure de tétrabutyle ammonium (0.28g) sous agitation à température ambiante (20°C) pendant 24heures. Le milieu réactionnel est versé sur de l'HCl 2N (150 ml) puis on l'épuise avec du dichlorométhane (3x100ml). Les extraits organiques sont réunis, séchés sur sulfate de magnésium, filtrés et évaporés sous pression réduite. Le composé obtenu est purifié par chromatographie sur gel de silice (300g g silice,) en éluant avec un mélange de heptane / acétate d'éthyle 85/15. Les fractions contenant le composé attendu sont réunies et évaporées sous pression réduite. On isole la (4,5-Dichloro-2-nitro-phenyl)-méthyle-amine désirée que l'on utilise dans l'étape suivante (0.57g).

### Etape 2 :

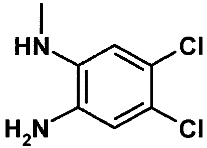

une solution de (4,5-Dichloro-2-nitro-phenyl)-méthyle-amine (500mg) dans l'acide acétique (7.5 ml) est traitée portion par portion par de la poudre de Zinc (740 mg). Le milieu réactionnel est agité à température ambiante (20°C) jusqu'à réduction complète. On ajoute de l'eau (13ml), de l'ammoniac 28% jusqu'à pH 7 puis on épuise le milieu réactionnel par l'acétate d'éthyle (3x50). Les extraits organiques sont réunis, séchés sur sulfate de magnésium, filtrés et évaporés sous pression réduite. L'huile obtenue est reprise dans l'éther (50ml) puis ajoutée par HCl 2N. Le solide formé est filtré. On isole la 4,5-Dichloro-N-méthyle-benzène-1,2-diamine sous sa forme chlorhydrate (510mg), que l'on utilise dans l'étape suivante.

### Etape 3 :

A une solution de chlorhydrate de 4,5-Dichloro-N-méthyle-benzène-1,2-diamine préparée ci-dessus (292.7mg) dans un mélange de DMF (1.75 ml) et de pyridine (1.75 ml) sous agitation magnétique sont ajoutés le chlorhydrate de N-(3-diméthyle aminopropyl)-N'-ethylcarbodiimide (220.3 mg) puis le sel de sodium de l'acide (4-Morpholine-4-yl-6-oxo-1,6-dihydro-pyrimidin-2-yl)-acétique, obtenu à l'étape 2 de l'exemple 1 (200mg). Le mélange ainsi obtenu est agité à température ambiante (20°C) pendant une nuit. Après évaporation du milieu réactionnel, on ajoute 3.5 ml d'acide acétique glacial et on porte au reflux jusqu'à obtention du composé de cyclisation. Le milieu réactionnel est alors dilué à l'eau (20 ml) puis ajouté de soude 2N jusqu'à pH 7 ; le milieu réactionnel est extrait à l'acétate d'éthyle (3 x 20 ml). Les extraits organiques sont réunis, séchés sur sulfate de magnésium, filtrés et évaporés sous pression réduite. Le composé obtenu est lavé au dichlorométhane puis séché. Le composé obtenu est purifié par chromatographie sur gel de silice (60 g silice,) en éluant avec un mélange de dichlorométhane / méthanol 80/20. Les fractions contenant le composé attendu sont réunies et évaporées sous pression réduite. On isole 121 mg de 2-[(5,6-dichloro-1-méthyle-1H-benzimidazol-2-yl)méthyle]-6-(morpholin-4-yl)pyrimidin-4(3H)-one dont les caractéristiques sont les suivantes :
Spectre RMN 1 H (400MHz): 3,32 (m, 4 H) ; 3,56 (m, 4 H) ; 3,80 (s, 3 H) ; 4,21 (s,2 H) ; 5,21 (s, 1 H) ; 7,85 (s, 1 H) ; 7,94 (s, 1 H) ; 11,82 (m étalé, 1 H)
Spectrométrie de Masse : méthode A
Temps de rétention Tr (min) = 0,75 ;
[M+H]+ : m/z 394 ; [M-H]- : m/z 392

### Exemple 39 : 6-(morpholin-4-yl)-2-[(5,6,7-trifluoro-1-méthyle-1H-benzimidazol-2-yl)méthyle]pyrimidin-4(3H)-one ;

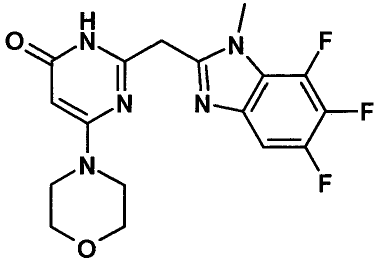

### Etape 1 :

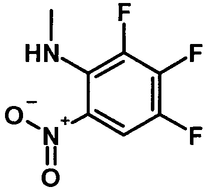

une solution de 2,3,4-trifluoro-6-nitro aniline (3g) dans le toluène (8.3 ml) est traitée par du sulfate de diméthyle (2.4g) et de la soude 2N (15.6 ml) en présence de bromure de tétrabutyle ammonium (0.3g) sous agitation à température ambiante (20°C) pendant 48 heures. Le milieu réactionnel est versé sur de l'HCl 2N (150 ml) puis on l'épuise avec du dichlorométhane (3x300ml). Les extraits organiques sont réunis, séchés sur sulfate de magnésium, filtrés et évaporés sous pression réduite. Le composé obtenu est purifié par chromatographie sur gel de silice (300g g silice,) en éluant avec un mélange de heptane / acétate d'éthyle 85/15. Les fractions contenant le composé attendu sont réunies et évaporées sous pression réduite. On isole la Méthyle-(3,4,5-trifluoro-2-nitro-phényle)-amine (2g) que l'on utilise dans l'étape suivante.

### Etape 2 :

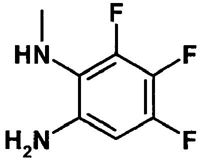

une solution de Méthyle-(3,4,5-trifluoro-2-nitro-phényle)-amine (0.5g) dans l'acide acétique (7.5 ml) est traitée portion par portion par de la poudre de Zinc (793 mg). Le milieu réactionnel est agité à température ambiante (20°C) jusqu'à réduction complète. On ajoute de l'eau (13ml), de l'ammoniac 28% jusqu'à pH 7 puis on épuise le milieu réactionnel par l'acétate d'éthyle (3x50). Les extraits organiques sont réunis, séchés sur sulfate de magnésium, filtrés et évaporés sous pression réduite. L'huile obtenue est reprise dans l'éther (50ml) puis ajoutée par HCl 2N. Le solide formé est filtré. On isole la 3,4,5-Trifluoro-N*1*-méthyle-benzène-1,2-diamine sous sa forme chlorhydrate (508mg), que l'on utilise dans l'étape suivante.

### Etape 3 :

A une solution de 3,4,5-trifluoro-N1-méthyle-benzène-1,2-diamine préparée ci-dessus (270 mg) dans un mélange de DMF (1.75 ml) et de pyridine (1.75 ml) sous agitation magnétique sont ajoutés le chlorhydrate de N-(3-diméthyle aminopropyl)-N'-ethylcarbodiimide (220.3 mg) puis le sel de sodium de l'acide (4-Morpholine-4-yl-6-oxo-1,6-dihydro-pyrimidin-2-yl)-acétique, obtenu à l'étape 2 de l'exemple 1 (200mg). Le mélange ainsi obtenu est agité à température ambiante (20°C) pendant une nuit. Après évaporation du milieu réactionnel, on ajoute 3.5 ml d'acide acétique glacial et on porte au reflux jusqu'à obtention du composé de cyclisation. Le milieu réactionnel est alors dilué à l'eau (20 ml) à pH 7 ; le milieu réactionnel est extrait à l'acétate d'éthyle (3 x 20 ml). Les extraits organiques sont réunis, séchés sur sulfate de magnésium, filtrés et évaporés sous pression réduite. Le composé obtenu est lavé au dichlorométhane puis séché. Le composé obtenu est purifié par chromatographie sur gel de silice (100 g silice,) en éluant avec un mélange de dichlorométhane / méthanol 80/20. Les fractions contenant le composé attendu sont réunies et évaporées sous pression réduite. On isole 44 mg de 6-(morpholin-4-yl)-2-[(5,6,7-trifluoro-1-méthyle-1 H-benzimidazol-2-yl)méthyle]pyrimidin-4(3H)-one dont les caractéristiques sont les suivantes :
Spectre RMN 1 H (400MHz): 3,33 (m, 4 H) ; 3,57 (m, 4 H) ; 3,94 (s, 3 H) ; 4,20 (s,2 H) ; 5,21 (s large, 1 H) ; 7,55 (ddd, J=1,5 et 6,4 et 10,5 Hz, 1 H) ; 11,82 (m étalé, 1 H)
Spectrométrie de Masse : méthode A
Temps de rétention Tr (min) = 0,72 ;
[M+H]+ : m/z 380 ; [M-H]- : m/z 378

### Exemple 40 : Synthèse du 2-[(4-hydroxy-1,3-benzoxazol-2-yl)méhyl]-6-(morpholin-4-yl)pyrimidin-4(3H)-one

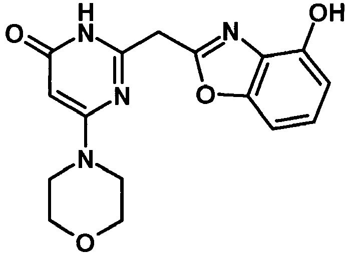

### Etape 1

A une solution de 1 g de [4-(morpholin-4-yl)-6-oxo-1,6-dihydropyrimidin-2-yl]acétate de sodium dans 8 mL de pyridine sont ajoutés 1,19 g de N-[3-(diméthylamino)propyl]-N'-éthylcarbodiimide chlorhydrate et 946 mg de 2-amino-résorcinol. Le mélange réactionnel est agité à température ambiante pendant 18 heures, puis concentré sous pression réduite. On ajoute de l'eau et de l'acétate d'éthyle et agite ainsi pendant 30 minutes. Le précipité formé est filtré, rincé à l'eau, l'éther éthylique et l'éther de pétrole. Le solide obtenu est séché sous vide. On obtient 904 mg du N-(2,6-dihydroxyphényl)-2-[4-(morpholin-4-yl)-6-oxo-1,6-dihydropyrimidin-2-yl]-acétamide sous forme de solide rouge utilisé tel quel dans l'étape suivante.
Spectrométrie de Masse : méthode B
Temps de rétention Tr (min) = 2,15
[M+H]+ : m/z 347 ; [M-H]- : m/z 345

### Etape 2

A une solution de 870 mg de N-(2,6-dihydroxyphényl)-2-[4-(morpholin-4-yl)-6-oxo-1,6-dihydropyrimidin-2-yl]-acétamide dans 60 mL d'ortho xylène sont ajoutés 239 mg d'hydrate d'acide 4-méthylbenzènesulfonique. Le mélange réactionnel est porté au reflux pendant 4 heures. Après refroidissement à 0°C, l'insoluble formé est filtré. Le filtrat est concentré à sec sous pression réduite puis le résidu est chromatographié sur colonne de gel de silice, en éluant avec CH2Cl2/MeOH 90/10. On obtient ainsi 654 mg de 2-[(4-hydroxy-1,3-benzoxazol-2-yl)méhyl]-6-(morpholin-4-yl)pyrimidin-4(3H)-one sous forme de solide blanc dont les caractéristiques sont les suivantes:
Spectre RMN 1 H (400MHz, d en ppm, DMSO-d6) : : 3,37 (m, 4 H) ; 3,57 (m, 4 H) ; 4,20 (s, 2 H) ; 5,25 (s, 1 H) ; 6,73 (d, J=7,9 Hz, 1 H) ; 7,09 (d, J=7,9 Hz, 1 H) ; 7,16 (t, J=7,9 Hz, 1 H) ; 10,23 (s large, 1 H) ; 11,92 (m étalé, 1 H)
Spectrométrie de Masse : méthode A
Temps de rétention Tr (min) = 0,56
[M+H]+ : m/z 329 ; [M-H]- : m/z 327

### Exemple 41 : 2-[(7-bromo-1-méthyle-1H-benzimidazol-2-yl)méthyle]-6-(morpholin-4-yl)pyrimidin-4(3H)-one

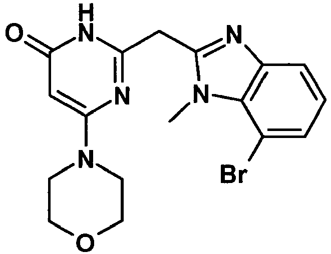

### Etape 1 :

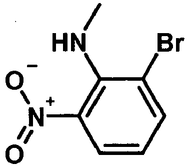

Une solution de 3-bromo-2-fluoronitro benzène (1g) dans le THF (8.3 ml) est chauffée en présence de N,N-diisopropyle ethyle amine (690µl) et de méthylamine (2.54 ml) dans un réacteur microondes (105°C, 30 min). Le milieu réactionnel est concentré puis repris dans l'acétate d'éthyle,lavé à l'eau puis à la saumure. Les extraits organiques sont réunis, séchés sur sulfate de magnésium, filtrés et évaporés sous pression réduite. On isole la (2-Bromo-6-nitro-phenyl)-methyl-amine (1g), solide orange que l'on utilise tel quel dans l'étape suivante.

### Etape 2 :

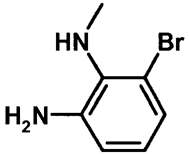

Une solution de (2-Bromo-6-nitro-phenyl)-methyl-amine (1g) dans l'acide acétique (20 ml) est traitée portion par portion par de la poudre de Zinc (1.41 mg). Le milieu réactionnel est agité à température ambiante (20°C) jusqu'à réduction complète. On ajoute de l'eau, de l'ammoniac 28% jusqu'à pH 7 puis on épuise le milieu réactionnel par l'acétate d'éthyle (3x50). Les extraits organiques sont réunis, lavés à l'eau et à la saumure, séchés sur sulfate de magnésium, filtrés et évaporés sous pression réduite. L'huile obtenue est reprise dans l'éther puis ajoutée par HCl 1 N. Le solide formé après évaporation est isolé et séché. On obtiens le chlorhydrate de 3-Bromo-N2-methyl-benzene-1,2-diamine (1g), que l'on utilise tel quel dans l'étape suivante.

### Etape 3 :

A une solution de chlorhydrate de 3-bromo-N2-methyl-benzene-1,2-diamine préparée ci-dessus (1 g) dans un mélange de DMF (8.3 ml) et de pyridine (8.3 ml) sous agitation magnétique puis on ajoute le chlorhydrate de N-(3-diméthyle aminopropyl)-N'-ethylcarbodümide (807mg) puis le sel de sodium de l'acide (4-Morpholine-4-yl-6-oxo-1,6-dihydro-pyrimidin-2-yl)-acétique, obtenu à l'étape 2 de l'exemple 1 (1g). Le mélange ainsi obtenu est agité à température ambiante (20°C) pendant une nuit. Après évaporation du milieu réactionnel, on ajoute de l'acide acétique glacial (20ml) et on porte au reflux (90min). Le milieu réactionnel est concentré, repris dans le dichlorométhane et de l'eau à pH 8 (soude 2N), il se forme un solide qui est filtré et lavé au dichlorométhane. Le solide obtenu est dissout dans du méthanol, adsorbé sur silice (7g) et purifié par chromatographie. Purification sur colonne de silice (50g) en éluant avec un mélange de dichlorométhane et de dichlorométhane/MeOH 9/1.(avec CV=75 ml ; Gradient : 4 CV DCM ; 4 CV de 0 à 16% DCM/MeOH 9/1; 8 CV de 16 à 20% DCM/MeOH 9/1; 1 CV de 20 à 30% DCM/MeOH 9/1.) Les fractions contenant le composé attendu sont réunies et évaporées sous pression réduite. On isole 100mg de 2-[(7-bromo-1-méthyle-1 H-benzimidazol-2-yl)méthyle]-6-(morpholin-4-yl)pyrimidin-4(3H)-one dont les caractéristiques sont les suivantes:
Spectre RMN 1 H (400MHz): 3,34 (m, 4 H) ; 3,57 (m, 4 H) ; 4,05 (s, 3 H) ; 4,21 (s,2 H) ; 5,21 (s, 1 H) ; 7,09 (t, J=7,8 Hz, 1 H) ; 7,40 (d, J=7,8 Hz, 1 H) ; 7,58 (d, J=7,8 Hz, 1 H) ; 11,82 (m
étalé, 1 H)
Spectrométrie de Masse : méthode B
Temps de rétention Tr (min) = 3,02 ;
[M+H]+ : m/z 404 ; [M-H]- : m/z 402

### Exemple 14 : Composition pharmaceutique

On a préparé des comprimés répondant à la formule suivante :
Produit de l'exemple 1 0,2 g
Excipient pour un comprimé terminé à 1 g
(détail de l'excipient : lactose, talc, amidon, stéarate de magnésium).

L'exemple 1 est pris à titre d'exemple de préparation pharmaceutique, cette préparation pouvant être réalisée si désiré avec d'autres produits en exemples dans la présente demande.

### Partie pharmacologique :

### Protocoles expérimentaux

### Procédures expérimentales in vitro

L'activité inhibitrice des molécules sur la phosphorylation d'AKT est mesurée soit par western blotting par la technique décrite ci-dessous, soit par la technique MSD Multi-spot Biomarker détection de Meso Scale Discovery également décrite ci-dessous. Il a été démontré sur un set de molécules que les 2 techniques donnent des résultats compatibles.

### Etude de l'expression de pAKT dans les cellules humaines PC3 de carcinome de prostate mesurée par western blotting (Test A):

Cet essai est basé sur la mesure de l'expression de la protéine AKT phosphorylée sur la serine 473. La phosphorylation d'AKT (pAKT) est mesurée par western blotting dans la lignée de carcinome de prostate humaine PC3 (ATCC CRL-1435), en utilisant un anticorps reconnaissant spécifiquement pAKT-S473.

Le jour 1, les cellules PC3 sont ensemencées en plaques 6 puits (TPP, # 92006) à la concentration de 0.8x106 cellules/puits dans 1800 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2 heures à 37°C en présence de 5% CO2. Les molécules diluées dans du diméthylsulfoxyde (DMSO Sigma #D2650), sont ajoutées à partir d'une solution mère concentrée 10 fois, le pourcentage final de DMSO étant de 0.1%. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Après aspiration du milieu de culture, les cellules sont rincées par 1ml de PBS (DPBS Gibco, #14190-094), récupérées par grattage dans 200µl de tampon HNTG complet et transfert en plaque 96 puits (Greiner #651201), et lysées pendant 1 H sur glace. Le tampon HNTG est composé du mélange suivant :Hepes 50 mM, NaCl 150 mM, Triton 1%, Glycerol 10%, avec ajoût extemporané d'une pastille de Protease Inhibitor Cocktail Mini (Roche 1836153) et d'une pastille de Phosphatase Inhibitor Cocktail (Roche104906837001) pour 10ml de tampon.

Le lysat est centrifugé 10min à 6000RPM. 155µl de surnageant sont récupérés. 150 µl sont incubés pour dénaturation pendant 5min à 95°C en présence de tampon NuPAGE LDS Sample Buffer 4X dilué 4 fois (Ref InVitrogen NP0007) et de NuPAGE Sample Reducing Agent 10X dilué 10 fois (Ref InVitrogen NP0009). Ces échantillons sont ensuite congelés à -20°C. 5 µl sont dosées par la technique microBCA selon la fiche technique du MicroBCA Proteine Assay Kit (Pierce #23235).

Pour la séparation des protéines, 20µg de protéines sont déposées sur gel NU-PAGE 4-12% Bis Tris Gel 12 puits (Ref InVitrogen NP0322BOX) et la migration est effectuée pendant 1 h30 en tampon de migration NU-PAGE MOPS SDS Running Buffer 20X dilué 20 fois (Ref InVitrogen NP0001), à 150 Volts.

Le gel est ensuite transféré sur une membrane Invitrolon PVDF (Invitrogen #LC2007) préalablement perméabilisée quelques secondes dans de l'éthanol (Ethanol Fischer Scientific #E/0600DF/15).

Le transfert s'effectue dans une cuve Biorad à 30 Volts pendant la nuit ou à 60 volts pendant 3 heures, en présence de tampon de transfert NUPAGE Transfer Buffer 20X dilué 20 fois (Ref InVitrogen NP0006).

La membrane est ensuite saturée en solution de saturation composé de TBS (Tris Buffer Saline 10x, Sigma #T5912 Sigma, dilué 10 fois), Tween 20 0.1% (#P5927 Sigma) et BSA 3% (Bovine Albumin Serum Fraction V, Sigma #A4503) pendant 6h après un transfert d'une durée de une nuit ou bien pendant 1 h après un transfert d'une durée de 3h.

Les anticorps primaires sont dilués au 1/1000e pour l'anticorps anti-phospho AKT-Ser473 (193H2, monoclonal de lapin, cat#4058 de chez Cell Signaling Technology) Abcam), en solution de saturation composée de PBS, Tween 20 0.1%, BSA 3%, puis mis sous agitation pendant la nuit à 4°C.

Deux rinçages de 5 min en solution de lavage composée de TBS, Tween 20 0.1% sont effectués avant l'hybridation des anticorps secondaires.

Les anticorps secondaires sont dilués au 1/10000e pour l'anticorps Rabbit anti-Mouse IgG HRP (W402 Promega) et au 1/10000e pour l'anticorps Goat anti-Rabbit IgG HRP (W401 Promega) en solution de saturation puis mis sous agitation pendant 1 h à température ambiante.

Deux rinçages de 30 min en solution de lavage sont effectués puis un rinçage de 5 min à l'H2O est effectué pour éliminer le Tween 20 restant.

La solution de révélation est préparée volume à volume selon la fiche technique du Western Lightning Chemiluminescence Reagent Plus (Western Lightning Chemiluminescence Reagent Plus Perkin Elmer #NEL104).

La membrane est placée pendant 1 min dans la solution de révélation, égouttée, insérée entre deux transparents puis placée dans l'appareil de mesure pour la lecture de la luminescence et la quantification du signal. La lecture de la luminescence est effectuée avec l'appareil FujiFilm (Ray Test).

L'appareil FUJI mesure le signal total de luminescence obtenu (AU) pour chaque bande sélectionnée. Puis il soustrait le bruit de fond (BG) proportionnel à la taille de la bande sélectionnée (Area), bruit de fond calculé à partir d'une bande de bruit de fond spécifique, en vue d'obtenir le signal spécifique (AU-BG) pour chaque bande. La bande obtenue en absence de produit et en présence de 0.1% DMSO est considérée comme le 100 % de signal. Le logiciel calcule le % d'activité spécifique (Ratio) obtenu pour chaque bande sélectionnée en fonction de ce 100 % de signal. Le calcul du pourcentage d'inhibition est fait pour chaque concentration selon la formule (100% - Ratio).

2 expériences indépendantes permettent de calculer la moyenne des pourcentages d'inhibition obtenus à une concentration donnée pour les produits testés uniquement à une concentration.

Le cas échéant, l'activité des produits est traduite en CI50 approchée, obtenue à partir d'une courbe dose-réponse de différentes concentrations testées et représentant la dose donnant 50 % d'inhibition spécifique (CI50 absolue). 2 expériences indépendantes permettent de calculer la moyenne des CI50s.

### Etude de l'expression de pAKT dans les cellules humaines PC3 de carcinome de prostate mesurée par la technique MSD Multi-spot Biomarker Detection de Meso Scale Discovery (Test B):

Cet essai est basé sur la mesure de l'expression de la protéine AKT phosphorylée sur la sérine 473 (P-AKT-S473), dans la lignée de carcinome de prostate humaine PC3, par la technique basée sur un immuno-essai sandwich utilisant le kit MSD Multi-spot Biomarker Detection de Meso Scale Discovery: kits phospho-Akt (Ser473) whole cell lysate (#K151CAD) ou phospho-Akt (Ser473)/Total Akt whole cell lysate (#K151OOD). L'anticorps primaire spécifique de P-AKT-S473 (Kit #K151CAD) est coaté sur une électrode dans chaque puits des plaques 96 puits du kit MSD : après ajoût d'un lysat de protéines dans chaque puits, la révélation du signal se fait par l'addition d'un anticorps secondaire de détection marqué avec un composé électrochimioluminescent. La procédure suivie est celle décrite dans le kit.

Le jour 1, les cellules PC3 sont ensemencées en plaques 96 puits (TPP, #92096) à la concentration de 35000 cellules/puits dans 200 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2h à 37°C en présence de 5% CO2. Les molécules diluées dans du diméthylsulfoxyde (DMSO Sigma #D2650), sont ajoutées à partir d'une solution mère concentrée 20 fois, le pourcentage final de DMSO étant de 0.1 %. Les molécules sont testées soit à une seule concentration inférieure ou égale à 10µM, soit à des concentrations croissantes dans une gamme pouvant s'étendre de moins de 1 nM à 10µM.

Après cette incubation, les cellules sont lysées pour la préparation des protéines. Pour cela, après aspiration du milieu de culture, 50µl de tampon de lyse Tris Lysis Buffer complet du kit MSD contenant les solutions d'inhibiteurs de protéases et phosphatases sont ajoutés dans les puits et les cellules sont lysées pendant 1 H à 4°C sous agitation. A ce stade les plaques contenant les lysats peuvent être congelées à -20°C ou à -80°C.

Les puits des plaques 96 puits du kit MSD sont saturés pendant 1h à température ambiante avec la solution bloquante du kit MSD. Quatre lavages sont effectués avec 150µl de solution de lavage Tris Wash Buffer du kit MSD. Les lysats préparés précédemment sont transférés dans les plaques Multi-spot 96 puits du kit MSD et incubés pendant 1h à température ambiante, sous agitation. Quatre lavages sont effectués avec 150µl de solution de lavage Tris Wash Buffer du kit MSD. 25µl de la solution MSD sulfo-tag détection antibody sont ajoutés dans les puits et incubés pendant 1h à température ambiante, sous agitation. Quatre lavages sont effectués avec 150µl de solution de lavage Tris Wash Buffer du kit MSD. 150µl de tampon de révélation Read Buffer du kit MSD sont ajoutés dans les puits et les plaques sont lues immédiatement sur l'instrument S12400 de Meso Scale Discovery.

L'appareil mesure un signal pour chaque puits. Des puits sans cellules et contenant le tampon de lyse servent à déterminer le bruit de fond qui sera soustrait à toutes les mesures (min). Les puits contenant des cellules en absence de produit et en présence de 0.1% DMSO sont considérés comme le 100 % de signal (max). Le calcul du pourcentage d'inhibition est fait pour chaque concentration de produit testé selon la formule suivante : (1- ((essai-min)/(max-min)))x100.

L'activité du produit est traduite en CI₅₀, obtenue à partir d'une courbe dose-réponse de différentes concentrations testées et représentant la dose donnant 50 % d'inhibition spécifique (CI₅₀ absolue). 2 expériences indépendantes permettent de calculer la moyenne des CI₅₀ₛ.

Les résultats obtenus pour les produits en exemples dans la partie expérimentale sont donnés dans le tableau de résultats pharmacologiques ci-après:

**Tableau de résultats pharmacologiques :**

| exemple | Test A* | Test B* |
|---|---|---|
| Exemple 1 | 298 | 41 |
| Exemple 2 | 388 | |
| Exemple 3 | 1343 | 85 |
| Exemple 4 | | 475 |
| Exemple 5 | 1014 | |
| Exemple 6 | 380 | 49 |
| Exemple 7 | 903 | 26 |
| Exemple8 | | 143 |
| Exemple9 | | 175 |
| Exemple 10 | 713 | 27 |
| Exemple 11 | | 16 |
| Exemple 12 | | 88 |
| Exemple 13 | | 8 |
| Exemple 14 | | 105 |
| Exemple 15 | | 26 |
| Exemple 16 | | 187 |
| Exemple 17 | | 63 |
| Exemple 18 | | 9 |
| Exemple 19 | | 109 |
| Exemple 20 | | **1000** |
| Exemple 21 | | **18** |
| Exemple 22 | | **28** |
| Exemple 23 | | **193** |
| Exemple 24 | | **140** |
| Exemple 25 | | **3000** |
| Exemple 26 | | **961** |
| Exemple 27 | | **256** |
| Exemple 28 | | **585** |
| Exemple 29 | | **506** |
| Exemple 30 | | **1340** |
| Exemple 31 | | **144** |
| Exemple 32 | | **206** |
| Exemple 33 | | **142** |
| Exemple 34 | | **208** |
| Exemple 35 | | **115** |
| Exemple 36 | | **28** |
| Exemple 37 | | **15** |
| Exemple 38 | | **65** |
| Exemple 39 | | **100** |
| Exemple 40 | | **44** |
| Exemple 41 | | **32** |

| | | |
|---|---|---|
| * Tests A et B : CI₅₀ (nM) | | |

## Revendications

1. Produits de formule (I): 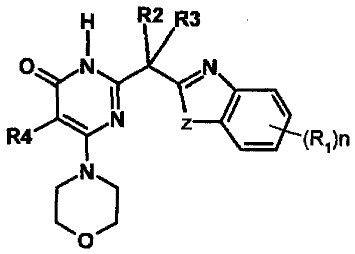 dans laquelle :
Z représente -O-, -NH, Ncycloalkyle, Nalk, ou N-phényl, avec alk et phényle éventuellement substitués par un ou plusieurs radicaux choisis parmi les atomes d'halogène, et les radicaux alkyle, alcoxy, hydroxyle, cyano et phényle lui-même éventuellement substitué par un ou plusieurs radicaux choisis parmi les atomes d'halogène et les radicaux hydroxyle, alcoxy et alkyle ;
n représente un entier de 0 à 4 ;
R1 représente un atome d'hydrogène, d'halogène ou un radical hydroxyle, alkyle ou alcoxy ; les radicaux alkyle et alcoxy étant éventuellement substitués par un groupe NRxRy ou par un ou plusieurs atomes d'halogène ;
étant entendu que R1 peut représenter un reste phényle fusionné avec le phényl qui porte R1 pour former un radical naphtyle;
R2 et R3 identiques ou différents représentent un atome d'hydrogène, un atome d'halogène ou un radical alkyle éventuellement substitué par un ou plusieurs atomes d'halogène;
R4 représente un atome d'hydrogène;
NRxRy étant tel que Rx représente un atome d'hydrogène ou un radical alkyle et Ry représente un atome d'hydrogène ou un radical alkyle; soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué par un ou plusieurs radicaux identiques ou différents choisis parmi les atomes d'halogène et les radicaux alkyle, hydroxyle, oxo, alcoxy, NH2; NHalk et N(alk)2 ;
tous les radicaux alkyle ou alk et alcoxy ci-dessus étant linéaires ou ramifiés et renfermant de 1 à 6 atomes de carbone,
lesdits produits de formule (I) étant sous toutes les formes stéréoisomères possibles racémiques, énantiomères et diastéréoisomères, ainsi que les sels d'addition avec les acides minéraux et organiques ou avec les bases minérales et organiques desdits produits de formule (I).

2. Produits de formule (I) tels que définis à la revendication 1 dans laquelle :
Z représente -O-, -NH, Ncycloalkyle, Nalk ou N-phényl, avec alk éventuellement substitué par un ou plusieurs radicaux choisis parmi alcoxy, hydroxyle, cyano et phényle;
n représente un entier de 0 à 4 ;
R1 représente un atome d'hydrogène, d'halogène ou un radical alkyle ou alcoxy ; les radicaux alkyle et alcoxy étant éventuellement substitués par un groupe NRxRy ou par un ou plusieurs atomes d'halogène ;
étant entendu que R1 peut représenter un reste phényle fusionné avec le phényl qui porte R1 pour former un radical naphtyle;
R2 et R3 représentent un atome d'hydrogène ;
R4 représente un atome d'hydrogène;
NRxRy étant tel que Rx représente un atome d'hydrogène ou un radical alkyle et Ry représente un atome d'hydrogène ou un radical alkyle; soit soit Rx et Ry forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
tous les radicaux alkyle ou alk et alcoxy ci-dessus étant linéaires ou ramifiés et renfermant de 1 à 6 atomes de carbone,
lesdits produits de formule (I) étant sous toutes les formes stéréoisomères possibles racémiques, énantiomères et diastéréoisomères, ainsi que les sels d'addition avec les acides minéraux et organiques ou avec les bases minérales et organiques desdits produits de formule (I).

3. Produits de formule (I) tels que définis à l'une quelconque des revendications 1 ou 2, répondant aux formules suivantes :
- 2-[(5-fluoro-1H-benzimidazol-2-yl)méthy)]-6-(morpholin-4-yl)pyrimidin-4(3H)-one
- 2-(1,3-benzoxazol-2-ylméthyl)-6-(morpholin-4-yl)pyrimidin-4(3H)-one
- 2-[(6-bromo-1H-benzimidazol-2-yl)méthyl]-6-(morpholin-4-yl)pyrimidin-4(3H)-one
- 2-[(6-méthoxy-1H-benzimidazol-2-yl)méthyl]-6-(morpholin-4-yl)pyrimidin-4(3H)-one
- 2-[(5,6-difluoro-1H-benzimidazol-2-yl)méthyl]-6-{morpholin-4-yl)pyrimidin-4(3H)-one
- 2-[(6-fluoro-1,3-benzoxazol)-2-yl)méthyl]-6-(morpholin-4-yl)pyrimidin-4(3H)-one
- 2-((5-fluoro-1,3-benzoxazol-2-yl}méthyl]-6-(morpholin-4-yl)pyrimidin-4(3H)-one
- 2-[(6-méthyl-1,3-benzoxazol-2-yl)méthyl]-6-{morpholin-4-yl)pyrimidin-4(3H)-one
- 2-[(6-bromo-1,3-benzoxazol-2-yl)méthyl]-6-(morpholin-4-yl)pyrimidin-4(3H)-one
- 2-[(1-méthyl-1H-benzimidazol-2-yl))méthyl]-6-(morpholin-4-yl)pyrimidin-4(3H)-one
- 2-[(5-fluoro-1-méthyl-1H-benzimidazol-2-yl)méthyl]-6-(morpholin-4-yl)pyrimidin-4(3H)-one
- 2-[(7-fluoro-1,3-benzoxazol-2-yl)méthyl]-fi-(morpholin-4-yl)pyrimidin-4(3H)-one
- 6-(morpholin-4-yl)-2-[(1-phényl-1H-benzimidazol-2-yl)méthyl]pyrimidin-4(3H)-one
- 2-[(1-benzol-1H-benzimidazol-2-yl)méthyl]-6-(morpholin-4-yl)pyrimidin-4(3H)-one
- 2-[(1-éthyl-1H-benzimidazol-2-yl)méthyl]-6-(morpholin-4-yl)pyrimidin-4(3H)-one
- 2-[(5,6-difluoro-1,3-benzoxazol-2-yl)méthyl]-6-(morpholin-4-yl)pyrimidin-4(3H)-one
- 2-[(6-chloro-1H-benzimidazol-2-yl)méthyl]-6-(morpholin-4-yl)pyrimidin-4(3H)-one
- 2-[(1-cyclopropyl-5-fluoro-1H-benzimidazol-2-yl)méthyl]-6-(morpholin-4-yl)pyrimidin-4(3H)-one
- 2-{[5-fluoro-1-(2-méthoxyéthyl)-1H-benzimidazol-2-yl]méthyl}-6-(morpholin-4-yl)pyrimidin-4(3H)-one
- 6-(morpholin-4-yl)-2-(naphtho[2,1-d][1,3]oxazol-2-ylmethyl)pyrimidin-4(3H)-one
- 2-[(6-methoxy-1,3-benzoxazol-2-yi)methyl]-6-(morpholin-4-yl)pyrimidin-4(3H)-one
- 2-[(6,7-difluoro-1,3-benzoxazol-2-yl)methyl]-6-(morpholin-4-yl)pyrimidin-4(3H)-one
- 2-[(5,6-dichloro-1H-benzimidazol-2-yl)methyl]-6-(morpholin-4-yl)pyrimidin-4(3H)-one
- 2-[(6-methyl-1 H-benzimidazol-2-yl)methyl]-6-(morpholin-4-yl)pyrimidin-4(3H)-one
- 2-{[4-chloro-6-(trifluoromethyl)-1H-benzimidazol-2-yl]methyl}-6-(morpholin-4-yl)pyrimidin-4(3H)-one
- 6-(morpholin-4-yl)-2-{[6-(trifluoromethyl)-1H-benzimidazol-2-yl]methyl}pyrimidin-4(3H)-one
- 2-[(5-chloro-6-fluoro-1H-benzimidazol-2-yl)methyl]-6-(morpholin-4-yl)pyrimidin-4(3H)-one
- 2-[(6,7-difluoro-1H-benzimidazol-2-yl)methyl]-6-(morpholin-4-yl)pyrimidin-4(3H)-one
- 2-[(5-chloro-6-methyl-1H-benzimidazol-2-yl)methyl]-6-(morpholin-4-yl)pyrimidin-4(3H)-one
- (2-{[4-(morpholin-4-yl)-6-oxo-1,6-dihydropyrimidin-2-yl]methyl}-1H-benzimidazol-1-yl)acetonitrile
- 2-[(5,7-difluoro-1H-benzimidazol-2-yl)methyl]-6-(morpholin-4-yl)pyrimidin-4(3H)-one
- 2-{[5-chloro-1-(1-phenylethyl)-1H-benzimidazol-2-yl]methyl}-6-(morpholin-4-yl)pyrimidin-4(3H)-one
- 2-[(1-cyclohexyl-1H-benzimidazol-2-yl)methyl]-6-(morpholin-4-yl)pyrimidin-4(3H)-one
- 6-(morpholin-4-yl)-2-[(5,6,7-trifluoro-1 H-benzimidazol-2-yl)methyl]pyrimidin-4(3H)-one
- 2-[(4-bromo-6-fluoro-1 H-benzimidazol-2-yl)methyl]-6-(morpholin-4-yl)pyrimidin-4(3H)-one
- 2-[(6-bromo-1-methyl-1H-benzimidazol-2-yl)methyl]-6-(morpholin-4-yl)pyrimidin-4(3H)-one
- 2-[(6,7-difluoro-1-methyl-1H-benzimidazol-2-yl)methyl]-6-(morpholin-4-yl)pyrimidin-4(3H)-one
- 2-[(5,6-dichloro-1-methyl-1H-benzimidazol-2-yl)methyl]-6-(morpholin-4-yl)pyrimidin-4(3H)-one
- 6-(morpholin-4-yl)-2-[(5,6,7-trifluoro-1-methyl-1H-benzimidazol-2-yl)methyl]pyrimidin-4(3H)-one
- 2-[(4-hydroxy-1,3-benzoxazol-2-yl)methyl]-6-(morpholin-4-yl)pyrimidin-4(3H)-one
- 2-[(7-bromo-1-methyl-1H-benzimidazol-2-yl)methyl]-6-(morpholin-4-yl)pyrimidin-4(3H)-one
ainsi que les sels d'addition avec les acides minéraux et organiques ou avec les bases minérales et organiques desdits produits de formule (I).

4. Procédé de préparation des produits de formule (I) tels que définis à l'une quelconque des revendications 1 à 3 selon le schéma 1A tel que défini ci-après. 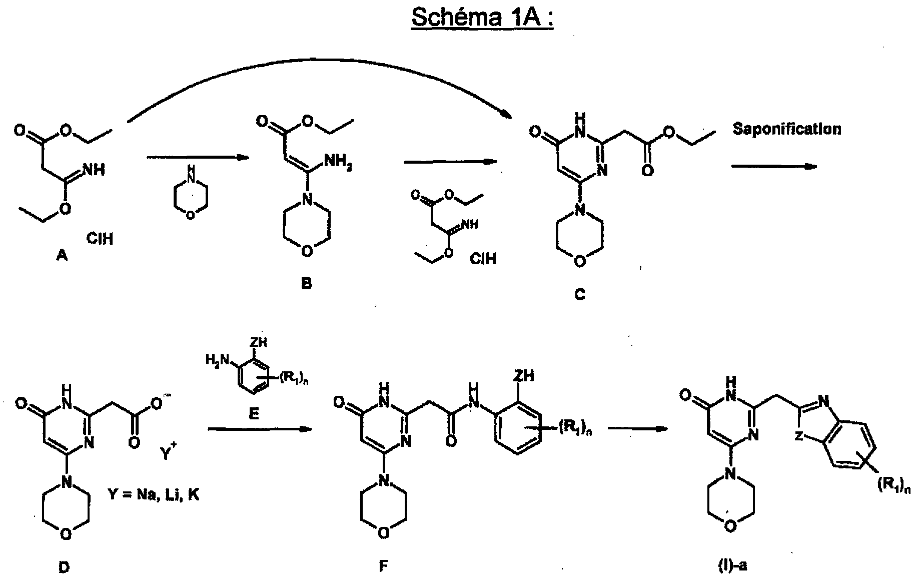 dans lequel les substituants R1 et Z ont les significations indiquées à l'une quelconques revendications 1 ou 2.

5. Procédé de préparation des produits de formule (I) tels que définis à l'une quelconque des revendications 1 à 3 selon le schéma 1B tel que défini ci-après. 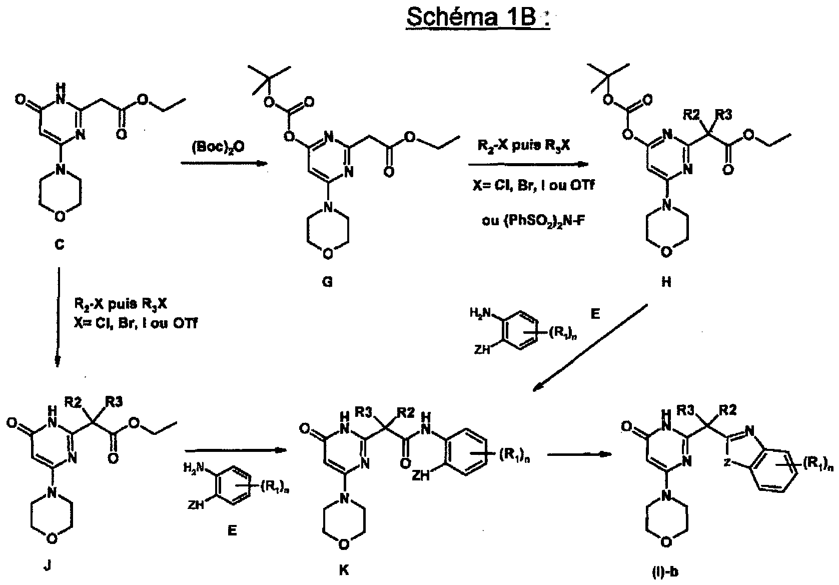 dans lequel les substituants R1, R2, R3 et Z ont les significations indiquées à l'une quelconque des revendications 1 ou 2.

6. Produits de formule (I) telle que définie à l'une quelconque des revendications 1 à 3, ainsi que les sels d'addition avec les acides minéraux et organiques ou avec les bases minérales et organiques pharmaceutiquement acceptables desdits produits de formule (I), pour leur utilisation en tant que médicaments.

7. Produits de formule (I) telle que définie à la revendication 3, ainsi que les sels d'addition avec les acides minéraux et organiques ou avec les bases minérales et organiques pharmaceutiquement acceptables desdits produits de formule (I), pour leur utilisation en tant que médicaments.

8. Compositions pharmaceutiques contenant à titre de principe actif l'un au moins des produits de formule (I) tel que défini à l'une quelconque des revendications 1 à 3, ou un sel pharmaceutiquement acceptable de ce produit et un support pharmaceutiquement acceptable.

9. Produits de formule (I) tels que définis à l'une quelconque des revendications 1 à 3 pour leur utilisation pour le traitement de cancers.

10. Produits de formule (I) tels que définis à l'une quelconque des revendications 1 à 3 pour leur utilisation pour le traitement de tumeurs solides ou liquides.

11. Produits de formule (I) tels que définis à l'une quelconque des revendications 1 à 3 pour leur utilisation pour le traitement de cancers résistant à des agents cytotoxiques.

12. Produits de formule (I) tels que définis à l'une quelconque des revendications 1 à 3 pour leur utilisation pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

13. Produits de formule (I) tels que définis à l'une quelconque des revendications 1 à 3 pour leur utilisation pour la chimiothérapie de cancers.

14. Produits de formule (I) tels que définis à l'une quelconque des revendications 1 à 3 pour leur utilisation pour la chimiothérapie de cancers, seuls ou en en association.

15. Intermédiaires de synthèse de formules C, D, F, J et K tels que définis à la revendication 4 ou 5 et rappelés ci-après: 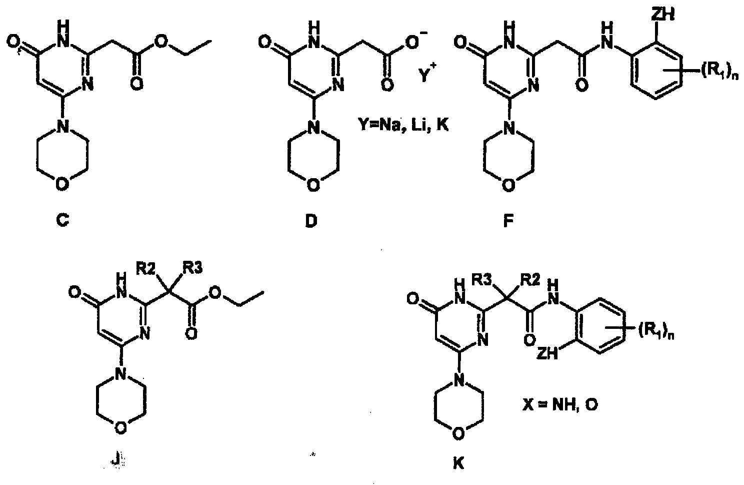 dans lesquels R1, R2, R3 et Z ont la définition indiquée à l'une quelconque des revendications 1 à 2.

## Patentansprüche

1. Produkte der Formel (I): 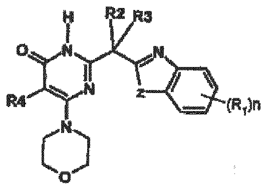 worin:
Z für -O-, -NH, N-Cycloalkyl, Nalk oder N-Phenyl steht, wobei alk und Phenyl gegebenenfalls durch einen oder mehrere Reste, die aus Halogenatomen und Alkyl-, Alkoxy-, Hydroxyd-, Cyano- und Phenylresten ausgewählt sind, substituiert sind, wobei der Phenylrest gegebenenfalls selbst durch einen oder mehrere Reste, die aus Halogenatomen und Hydroxyl-, Alkoxy- und Alkylresten ausgewählt sind, substituiert ist;
n für eine ganze Zahl von 0 bis 4 steht;
R1 für ein Wasserstoffatom, ein Halogenatom oder einen Hydroxyl-, Alkyl- oder Alkoxyrest steht; wobei die Alkyl- und Alkoxyreste gegebenenfalls durch eine Gruppe NRxRy oder ein oder mehrere Halogenatome substituiert sind;
mit der Maßgabe, dass R1 für einen Phenylrest, der mit dem Phenyl, das R1 trägt, zu einen Naphtylrest kondensiert ist, stehen kahn;
R2 und R3 gleich oder verschieden sind und für ein Wasserstoffatom, ein Halogenatom oder einen Alkylrest, der gegebenenfalls durch ein oder mehrere Halogenatome substituiert ist, stehen; R4 für ein Wasserstoffatom steht;
NRxRy so beschaffen ist, dass Rx für ein Wasserstoffatom oder einen Alkylrest steht und Ry für ein Wasserstoffatom oder einen Alkylrest steht; oder Rx und Ry mit dem Stickstoffatom, an das sie gebunden sind, einen 3- bis 10-gliedrigen cyclischen Rest bilden, der gegebenenfalls ein oder mehrere aus O, S, NH und N-Alkyl ausgewählte Heteroatome enthält und gegebenenfalls durch einen oder mehrere gleiche oder verschiedene Reste, die aus Halogenatomen und Alkyl-, Hydroxyl-, Oxo-, Alkoxy-, NH₂-, NHalk- und N(alk)₂-Resten ausgewählt sind, substituiert ist;
wobei alle obigen Alkyl- oder alk- und Alkoxyreste linear oder verzweigt sind und 1 bis 6 Kohlenstoffatome enthalten,
wobei die Produkte der Formel (I) in allen möglichen racemischen, enantiomeren und diastereoisomeren Stereoisomerformen vorliegen, sowie die Säureadditionssalze der Produkte der Formel (I) met anorganischen und organischen Säuren oder mit anorganischen und organischen Basen.

2. Produkte der Formel (I) gemäß Anspruch 1, worin:
Z für -O-, -NH, -cycloalkyl, Nalk oder N-phenyl steht, wobei alk gegebenenfalls durch einen oder mehrere Reste, die aus Alkoxy-, Hydroxyl-, Cyano-und Phenylresten ausgewählt sind, substituiert ist;
n für eine ganze Zahl von 0 bis 4 steht;
R1 für ein Wasserstoffatom, ein Halogenatom oder einen Alkyl- oder Alkoxyrest steht; wobei die Alkyl- und Alkoxyreste gegebenenfalls durch eine Gruppe NRxRy oder ein oder mehrere Halogenatome substituiert sind;
mit der Maßgabe, dass R1 für einen Phenylrest, der mit dem Phenol, das R1 trägt, zu einem Naphthylrest kondensiert ist, stehen kann;
R2 und R3 für ein Wasserstoffatom stehen;
R4 für ein Wasserstoffatom steht;
NRxRy so beschaffen ist, dass Rx für ein Wasserstoffatom oder einen Alkylrest steht und Ry für ein Wasserstoffatom oder einen Alkylrest steht; oder Rx und Ry mit dem Stickstoffatom, an das sie gebunden sind, einen 3- bis 10-gliedrigen cyclischen Rest bildern, der gegebenenfalls ein oder mehrere aus O, S, NH und N-Alkyl ausgewählte Heteroatome enthält und der gegebenenfalls substituiert ist;
wobei alle obigen Alkyl- oder alk- und Alkoxyreste linear oder verzweigt sind und 1 bis 6 Kohlenstoffatome enthalten,
wobei die Produkte der Formel (I) in allen möglichen racemischen, enantiomeren und diastereoisomeren Stereoisomerformen vorliegen, sowie die Säureadditionssalze der Produkte der Formel (I) mit anorganischen und organischem Säuren oder mit anorganischen und organischen Basen.

3. Produkte der Formel (I) gemäß einem der Ansprüche 1 oder 2, die den folgenden Formeln entsprechen:
- 2-[(5-Fluor-1H-benzimidazol-2-yl)methyl]-6-(morpholin-4-yl)pyrimidin-4(3H)-on
- 2-(1,3-Benzoxazol-2-ylmethyl)-6-(morpholin-4-yl)pyrimidin-4(3H)-on
- 2-[(6-Brom-1H-benzimidazol-2-yl)methyl]-6-(morpholin-4-yl)pyrimidin-4(3H)-on
- 2-[(6-Methoxy-1H-benzimidazol-2-yl)methyl]-6-(morpholin-4-yl)pyrimidin-4(3H)-on
- 2-[(5,6-Difluor-1H-benzimidazol-2-yl)methyl]-6-(morpholin-4-yl)pyrimidin-4(3H)-on
- 2-[(6-Fluor-1,3-benzoxazol-2-yl)methyl]-6-(morpholin-4-yl)pyrimidin-4(3H)-on 2-[(5-Fluor-1,3-benzoxazol-2-yl)methyl]-6-(morpholin-4-yl)pyrimidin-4(3H)-on
- 2-[(6-Methyl-1,3-benzoxazol-2-yl)methyl]-6-(morpholin-4-yl)pyrimidin-4(3H)-on
- 2-[(6-Brom-1,3-benzoxazol-2-yl)methyl]-6-(morpholin-4-yl)pyrimidin-4(3H)-on
- 2-[(1-Methyl-1H-benzimidazol-2-yl)methyl]-6-(morpholin-4-yl)pyrimidin-4(3H)-on
- 2-[(5-Fluor-1-methyl-1H-benzimidazol-2-yl)methyl]-6-(morpholin-4-yl)pyrimidin-4(3H)-on
- 2-[(7-Fluor-1,3-benzoxazol-2-yl)methyl]-6-(morpholin-4-yl)pyrimidin-4(3H)-on
- 6-(Morpholin-4-yl)-2-[(1-phenyl-1H-benzimidazol-2-yl)methyl]pyrimidin-4(3H)-on
- 2-[(1-Benzyl-1H-benzimidazol-2-yl)methyl]-6-(morpholin-4-yl)pyrimidin-4(3H)-on
- 2-[(1-Ethyl-1H-benzimidazol-2-yl)methyl]-6-(morpholin-4-yl)pyrimidin-4(3H)-on
- 2-[(5,6-Difluor-1,3-benzoxazol-2-yl)methyl]-6-(morpholin-4-yl)pyrimidin-4(3H)-on
- 2-[(6-Chlor-1H-benzimidazol-2-yl)methyl]-6-(morpholin-4-yl)pyrimidin-4(3H)-on
- 2-[(1-Cyclopropyl-5-fluor-1H-benzimidazol-2-yl)methyl]-6-(morpholin-4-yl)pyrimidin-4(3H)-on
- 2-{[5-Fluor-1-(2-methoxyethyl)-1H-benzimidazol-2-yl]methyl}-6-(morpholin-4-yl)pyrimidin-4(3H)-on
- 6-(Morpholin-4-yl)-2-(naphtho[2,1-d][1,3]oxazol-2-ylmethyl)pyrimidin-4(3H)-on
- 2-[(6-Methoxy-1,3-benzoxazol-2-yl)methyl]-6-(morpholin-4-yl)pyrimidin-4(3H)-on
- 2-[(6,7-Difluor-1,3-benzoxazol-2-yl)methyl]-6-(morpholin-4-yl)pyrimidin-4(3H)-on
- 2-[(5,6-Dichlor-1H-benzimidazol-2-yl)methyl]-6-(morpholin-4-yl)pyrimidin-4(3H)-on
- 2-[(6-Methyl-1H-benzimidazol-2-yl)methyl]-6-(morpholin-4-yl)pyrimidin-4(3H)-on
- 2-{[4-Chlor-6-(trifluormethyl)-1H-benzimidazol-2-yl]methyl}-6-(morpholin-4-yl)pyrimidin-4(3H)-on
- 6-(Morpholin-4-yl)-2-{[6-(trifluormethyl)-1H-benzimidazol-2-yl]methyl}pyrimidin-4(3H)-on
- 2-[(5-Chlor-6-fluor-1H-benzimidazol-2-yl)methyl]-6-(morpholin-4-yl)pyrimidin-4(3H)-on
- 2-[(6,7-Difluor-1H-benzimidazol-2-yl)methyl]-6-(morpholin-4-yl)pyrimidin-4(3H)-on
- 2-[(5-Chlor-6-methyl-1H-benzimidazol-2-yl)methyl]-6-(morpholin-4-yl)pyrimidin-4(3H)-on
- (2-{[4-(Morpholin-4-yl)-6-oxo-1,6-dihydropyrimidin-2-yl]methyl}-1H-benzimidazol-1-yl)acetonitril
- 2-[(5,7-Difluor-1H-benzimidazol-2-yl)methyl]-6-(morpholin-4-yl)pyrimidin-4(3H)-on
- 2-{[5-Chlor-1-(1-phenylethyl)-1H-benzimidazol-2-yl]methyl}-6-(morpholin-4-yl)pyrimidin-4(3H)-on
- 2-[(1-Cyclohexyl-1H-benzimidazol-2-yl)methyl]-6-(morpholin-4-yl)pyrimidin-4(3H)-on
- 6-(Morpholin-4-yl)-2-[(5,6,7-trifluor-1H-benzimidazol-2-yl)methyl]pyrimidin-4(3H)-on
- 2-[(4-Brom-6-fluor-1H-benzimidazol-2-yl)methyl]-6-(morpholin-4-yl)pyrimidin-4(3H)-on
- 2-[(6-Brom-1-methyl-1H-benzimidazol-2-yl)methyl]-6-(morpholin-4-yl)pyrimidin-4(3H)-on
- 2-[(6,7-Difluor-1-methyl-1H-benzimidazol-2-yl)methyl]-6-(morpholin-4-yl)pyrimidin-4(3H)-on
- 2-[(5,6-Dichlor-1-methyl-1H-benzimidazol-2-yl)methyl]-6-(morpholin-4-yl)pyrimidin-4(3H)-on
- 6-(Morpholin-4-yl)-2-[(5,6,7-trifluor-1-methyl-1H-benzimidazol-2-yl)methyl]pyrimidin-4(3H)-on
- 2-[(4-Hydroxy-1,3-benzoxazol-2-yl)methyl]-6-(morpholin-4-yl)pyrimidin-4(3H)-on
- 2-[(7-Brom-1-methyl-1H-benzimidazol-2-yl)methyl]-6-(morpholin-4-yl)pyrimidin-4(3H)-on,
sowie die Säureadditionssalze der Produkte der Formel (I) mit anorganischen und organischen Säuren oder mit anorganischen und organischen Basen.

4. Verfahren zur Herstellung der Produkte der Formel (I) gemäß einem der Ansprüche 1 bis 3 gemäß nachstehendem Schema 1A 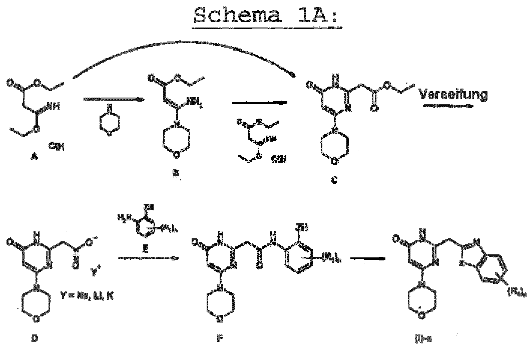 wobei die Substituenten R1 und Z die in einem der Ansprüche 1 oder 2 angegebenen Bedeutungen besitzen.

5. Verfahren zur Herstellung der Produkte der Formel (I) gemäß einem der Ansprüche 1 bis 3 gemäß nachstehendem Schema 1B 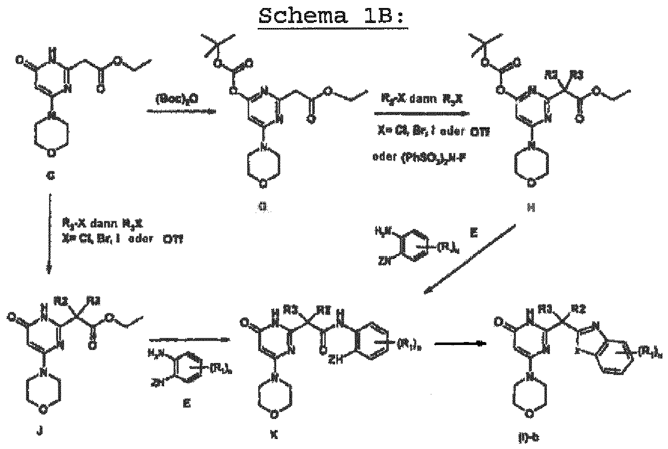 wobei die Substituenten R1, R2, R3 und Z die in einem der Ansprüche 1 oder 2 angegebenen Bedeutungen besitzen.

6. Produkte der Formel (I) gemäß einem der Ansprüche 1 bis 3 sowie die pharmazeurisch unbedenklichen Säureadditionssalze der Produkte der Formel (I) mit anorganischen und organischen Säuren oder mit anorganischen und organischen Basen zur Verwendung als Medikamente.

7. Produkte der Formel (I) gemäß Anspruch 3 sowie die pharmazeutisch unbedenklichen Säureadditionssalze der Produkte der Formel (I) mit anorganischen und organischen Säuren oder mit anorganischen und organischen Basen zur Verwerdung als Medikamente.

8. Pharmazeutisch Zusammensetzungen, die als Wirkstoff mindestens eine der Produkte der Formel (I) gemäß einem der Ansprüche 1 bis 3 oder ein pharmazeurisch unbedenkliches Salz dieses Produkts und einen pharmazeutisch unbedenklichen Träger enthält.

9. Produkte der Formel (I) gemäß einem der Ansprüche 1 bis 3 zur Verwendung für die Behandlung von Krebserkrankungen.

10. Produkte der Formel (I) gemäß einem der Ansprüche 1 bis 3 zur Verwendung für die Behandlung von soliden oder flüssigen Tumoren.

11. Produkte der Formel (I) gemäß einem der Ansprüche 1 bis 3 zur Verwendung für die Behandlung von Krebserkrankungen, die gegenüber Zytotoxika resistent sind.

12. Produkte der Formel (I) gemäß einem der Ansprüche 1 bis 3 zur Verwendung für die Behandlung von primären Tumoren und/oder Metastasen, insbesondere bei Krebserkrankungen des Magens, der Leber, der Niere, der Eierstöcke, des Kolons, der Prostata, des Endometriums, der Lunge (NSCLC und SCLC), Glioblastomen, Krebserkrankungen der Schilddrüse, der Blase, der Brust, bei Melanom, bei lymphoiden oder myeloiden hämatopoetischen Tumoren, bei Sarkomen, bei Krebserkrankungen des Gehirns, des Kehlkopfs, des Lymphsystems, Krebserkrankungen der Knochen und der Bauchspeicheldrüse, und bei Hamartomen.

13. Produkte der Formel (I) gemäß einen der Ansprüche 1 bis 3 zur Verwerdung für die Chemotherapie von Krebserkrankungen.

14. Produkte der Formel (I) gemäß einem der Ansprüche 1 bis 3 zur Verwendung für die Chemotherapie von Krebserkrankungen, allein oder in Kombination.

15. Synthesezwischenprodukte der Formeln C, D, F, J und K gemäß Anspruch 4 oder 5, die nachstehend noch einmal aufgeführt sind: 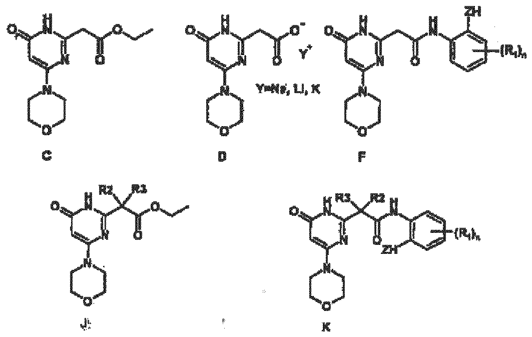 wobei die Substituenten R1, R2, R3 und Z die in einem der Ansprüche 1 oder 2 angegebenen Bedeutungen besitzen.

## Claims

1. Products of formula (I): 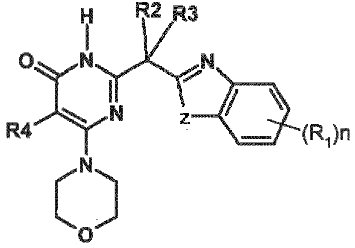 in which:
Z represents -O-, -NH, N-cycloalkyl, Nalk or N-phenyl, with alk and phenyl optionally substituted with one or more radicals chosen from halogen atoms, and alkyl, alkoxy, hydroxyl, cyano and phenyl radicals, said phenyl radical being itself optionally substituted with one or more radicals chosen from halogen atoms and hydroxyl, alkoxy and alkyl radicals;
n represents an integer from 0 to 4;
R1 represents a hydrogen atom, a halogen atom or a hydroxyl, alkyl or alkoxy radical; the alkyl and alkoxy radicals being optionally substituted with an NRxRy group or with one or more halogen atoms;
it being understood that R1 can represent a phenyl residue fused with the phenyl which bears R1 so as to form a naphthyl radical;
R2 and R3, which may be identical or different, represent a hydrogen atom, a halogen atom, or an alkyl radical optionally substituted with one or more halogen atoms;
R4 represents a hydrogen atom;
NRxRy being such that Rx represents a hydrogen atom or an alkyl radical and Ry represents a hydrogen atom or an alkyl radical; or Rx and Ry form, with the nitrogen atom to which they are attached, a cyclic radical containing from 3 to 10 ring members and optionally one or more other heteroatoms chosen from O, S, NH and N-alkyl, this cyclic radical being optionally substituted with one or more radicals, which may be identical or different, chosen from halogen atoms and alkyl, hydroxyl, oxo, alkoxy, NH₂, NHalk and N(alk)₂ radicals; all the above alkyl or alk and alkoxy radicals being linear or blanched and containing from 1 to 6 carbon atoms,
said products of formula (I) being in all the possible racemic, enantiomeric and diastereoisomeric stereoisomer forms, and also the addition salts with inorganic and organic acids or with inorganic and organic bases, of said products of formula (I).

2. Products of formula (I) as defined in Claim 1, in which:
Z represents -O-, -NH, N-cycloalkyl, Nalk or N-phenyl, with alk optionally substituted with one or more radicals chosen from alkoxy, hydroxyl, cyano and phenyl;
n represents an integer from 0 to 4;
R1 represents a hydrogen atom, a halogen atom or an alkyl or alkoxy radical; the alkyl and alkoxy radicals being optionally substituted with an NRxRy group or with one or more halogen atoms;
it being understood that R1 can represent a phenyl residue fused with the phenyl which bears R1 so as to form a naphthyl radical;
R2 and R3 represent a hydrogen atom;
R4 represents a hydrogen atom;
NRxRy being such that Rx represents a hydrogen atom or an alkyl radical and Ry represents a hydrogen atom or an alkyl radical; or Rx and Ry form, with the nitrogen atom to which they are attached, a cyclic radical containing from 3 to 10 ring members and optionally one or more other heteroatoms chosen from O, S, NH and N-alkyl, this cyclic radical being optionally substituted;
all the above alkyl or alk and alkoxy radicals being linear or branched and containing from 1 to 6 carbon atoms,
said products of formula (I) being in all the possible racemic, enantiomeric and diastereoisomeric stereoisomer forms, and also the addition salts with inorganic and organic acids or with inorganic and organic bases, of said products of formula (I).

3. Products of formula (I) as defined in either one of Claims 1 and 2, corresponding to the following formulae:
- 2-[(5-fluoro-1H-benzimidazol-2-yl)methyl]-6-(morpholin-4-yl)pyrimidin-4(3H)-one
- 2-(1,3-benzoxazol-2-ylmethyl)-6-(morpholin-4-yl)pyrimidin-4(3H)-one
- 2-[(6-bromo-1H-benzimidazol-2-yl)methyl]-6-(morpholin-4-yl)pyrimidin-4(3)-one
- 2-[(6-methoxy-1H-benzimidazol-2-yl)methyl]-6-(morpholin-4-yl)pyrimidin-4(3H)-one
- 2-[(5,6-difluoro-1H-benzimidazol-2-yl)methyl]-6-(morpholin-4-yl)pyrimidin-4(3H)-one
- 2-[(6-fluoro-1,3-benzoxazol-2-yl)methyl]-6-(morpholin-4-yl)pyrimidin-4(3H)-one
- 2-[(5-fluoro-1,3-benzoxazol-2-yl)methyl]-6-(morpholin-4-yl)pyrimidin-4(3H)-one
- 2-[(6-methyl-1,3-benzoxazol-2-yl)methyl]-6-(morpholin-4-yl)pyrimidin-4(3H)-one
- 2-[(6-bromo-1,3-benzoxazol-2-yl)methyl]-6-(morpholin-4-yl)pyrimidin-4(3H)-one
- 2-[(1-methyl-1H-benzimidazol-2-yl)methyl]-6-(morpholin-4-yl)pyrimidin-4(3H)-one
- 2-[(5-fluoro-1-methyl-1H-benzimidazol-2-yl)methyl]-6-(morpholin-4-yl)pyrimidin-4(3H)-one
- 2-[(7-fluoro-1,3-benzoxazol-2-yl)methyl]-6-(morpholin-4-yl)pyrimidin-4(3H)-one
- 6-(morpholin-4-yl)-2-[(1-phenyl-1H-benzimidazol-2-yl)methyl]pyrimidin-4(3H)-one
- 2-[(1-benzyl-1H-benzimidazol-2-yl)methyl]-6-(morpholin-4-yl)pyrimidin-4(3H)-one
- 2-[(1-ethyl-1H-benzimidazol-2-yl)methyl]-6-(morpholin-4-yl)pyrimidin-4(3H)-one
- 2-[(5,6-difluoro-1,3-benzoxazol-2-yl)methyl]-6-(morpholin-4-yl)pyrimidin-4(3H)-one
- 2-[(6-chloro-1H-benzimidazol-2-yl)methyl]-6-(morpholin-4-yl)pyrimidin-4(3H)-one
- 2-[(1-cyclopropyl-5-fluoro-1H-benzimidazol-2-yl)methyl]-6-(morpholin-4-yl)pyrimidin-4(3H)-one
- 2-{[5-fluoro-1-(2-methoxyethyl)-1H-benzimidazol-2-yl]methyl}-6-(morpholin-4-yl)pyrimidin-4(3H)-one
- 6-(morpholin-4-yl)-2-(naphtho[2,1-d][1,3]oxazol-2-ylmethyl)pyrimidin-4(3H)-one
- 2-[(6-methoxy-1,3-benzoxazol-2-yl)methyl]-6-(morpholin-4-yl)pyrimidin-4(3H)-one
- 2-[(6,7-difluoro-1,3-benzoxazol-2-yl)methyl]-6-(morpholin-4-yl)pyrimidin-4(3H)-one
- 2-[(5,6-dichloro-1H-benzimidazol-2-yl)methyl]-6-(morpholin-4-yl)pyrimidin-4(3H)-one
- 2-[(6-methyl-1H-benzimidazol-2-yl)methyl]-6-(morpholin-4-yl)pyrimidin-4(3H)-one
- 2-{[4-chloro-6-(trifluoromethyl)-1H-benzimidazol-2-yl]methyl}-6-(morpholin-4-yl)pyrimidin-4(3H)-one
- 6-(morpholin-4-yl)-2-{[6-(trifluoromethyl)-1H-benzimidazol-2-yl]methyl}pyrimidin-4(3H)-one
- 2-[(5-chloro-6-fluoro-1H-benzimidazol-2-yl)methyl]-6-(morpholin-4-yl)pyrimidin-4(3H)-one
- 2-[(6,7-difluoro-1H-benzimidazol-2-yl)methyl]-6-(morpholin-4-yl)pyrimidin-4(3H)-one
- 2-[(5-chloro-6-methyl-1H-benzimidazol-2-yl)methyl]-6-(morpholin-4-yl)pyrimidin-4(3H)-one
- (2-{[4-(morpholin-4-yl)-6-oxo-1,6-dihydropyrimidin-2-yl]methyl}-1H-benzimidazol-1-yl)acetonitrile
- 2-[(5,7-difluoro-1H-benzimidazol-2-yl)methyl]-6-(morpholin-4-yl)pyrimidin-4(3H)-one
- 2-{[5-chloro-1-(1-phenylethyl)-1H-benzimidazol-2-yl]methyl}-6-(morpholin-4-yl)pyrimidin-4(3H)-one
- 2-[(1-cyclohexyl-1H-benzimidazol-2-yl)methyl]-6-(morpholin-4-yl)pyrimidin-4(3H)-one
- 6-(morpholin-4-yl)-2-[(5,6,7-trifluoro-1H-benzimidazol-2-yl)methyl]pyrimidin-4(3H)-one
- 2-[(4-bromo-6-fluoro-1H-benzimidazol-2-yl)methyl]-6-(morpholin-4-yl)pyrimidin-4(3H)-one
- 2-[(6-bromo-1-methyl-1H-benzimidazol-2-yl)methyl]-6-(morpholin-4-yl)pyrimidin-4(3H)-one
- 2-[(6,7-difluoro-1-methyl-1H-benzimidazol-2-yl)methyl]-6-(morpholin-4-yl)pyrimidin-4(3H)-one
- 2-[(5,6-dichloro-1-methyl-1H-benzimidazo-2-yl)methyl]-6-(morpholin-4-yl)pyrimidin-4(3H)-one
- 6-(morpholin-4-yl)-2-[(5,6,7-trifluoro-1-methyl-1H-benzimidazol-2-yl)methyl]pyrimidin-4(3H)-one
- 2-[(4-hydroxy-1,3-benzoxazol-2-yl)methyl]-6-(morpholin-4-yl)pyrimidin-4(3H)-one
- 2-[(7-bromo-1-methyl-1H-benzimidazol-2-yl)methyl]-6-(morpholin-4-yl)pyrimidin-4(3H)-one
and also the addition salts with inorganic and organic acids or with inorganic and organic bases, of said products of formula (I).

4. Process for preparing the products of formula (I) as defined in any one of Claims 1 to 3, according to Scheme 1A as defined hereinafter: 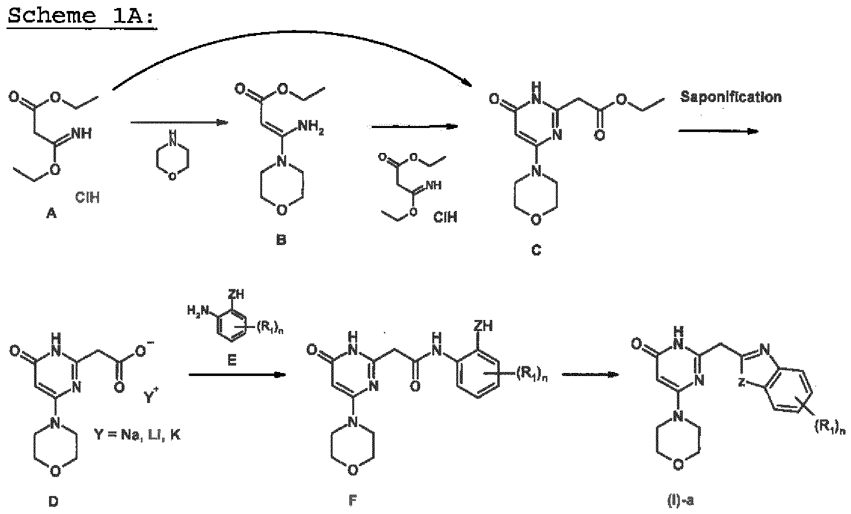 in which the substituents R1 and Z have the meanings indicated in either one of Claims 1 and 2.

5. Process for preparing the products of formula (I) as defined in any one of Claims 1 to 3, according to Scheme 1B as defined hereinafter: 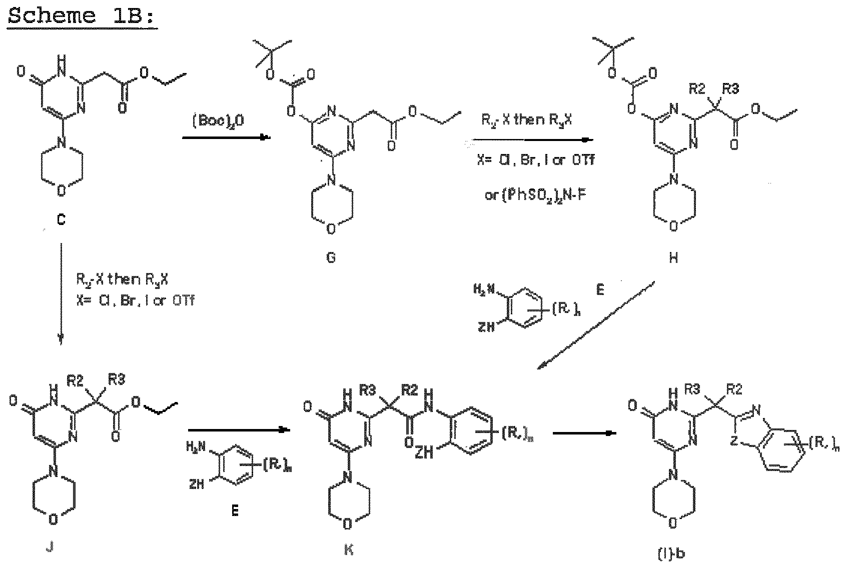 in which the substituents R1, R2, R3 and Z have the meanings indicated in either one of Claims 1 and 2.

6. Products of formula (I) as defined in any one of Claims 1 to 3, and also the pharmaceutically acceptable addition salts with inorganic and organic acids or with inorganic and organic bases, of said products of formula (I), for the use thereof as medicaments.

7. Products of formula (I) as defined in Claim 3, and also the pharmaceutically acceptable addition salts with inorganic and organic acids or with inorganic and organic bases, of said products of formula (I), for the use thereof as medicaments.

8. Pharmaceutical compositions containing, as active ingredient, at least one of the products of formula (I) as defined in any one of Claims 1 to 3, or a pharmaceutically acceptable salt of this product and a pharmaceutically acceptable carrier.

9. Products of formula (I) as defined in any one of Claims 1 to 3, for the use thereof in the treatment of cancers.

10. Products of formula (I) as defined in any one of Claims 1 to 3, for the use thereof in the treatment of solid or liquid tumours.

11. Products of formula (I) as defined in any one of Claims 1 to 3, for the use thereof in the treatment of cancers resistant to cytotoxic agents.

12. Products of formula (I) as defined in any one of Claims 1 to 3, for the use thereof in the treatment of primary tumours and/or of metastases, in particular in gastric, hepatic, renal, ovarian, colon, prostate, endometrial and lung (NSCLC and SCLC) cancers, glioblastomas, thyroid, bladder and breast cancers, in melanoma, in lymphoid or myeloid hematopoietic tumours, in sarcomas, in brain, larynx and lymphatic system cancers, bone and pancreatic cancers, and in hamartomas.

13. Products of formula (I) as defined in any one of Claims 1 to 3, for the use thereof in cancer chemotherapy.

14. Products of formula (I) as defined in any one of Claims 1 to 3, for the use thereof in cancer chemotherapy, alone or in combination.

15. Synthesis intermediates of formulae C, D, F, J and K as defined in Claims 4 or 5 and recalled below: 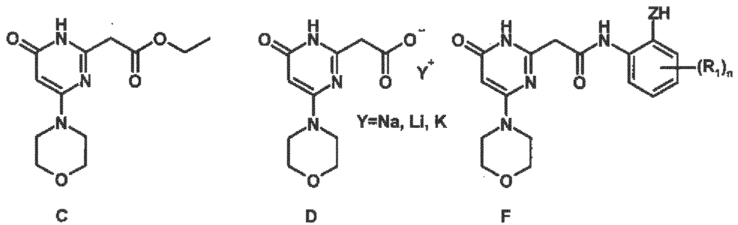 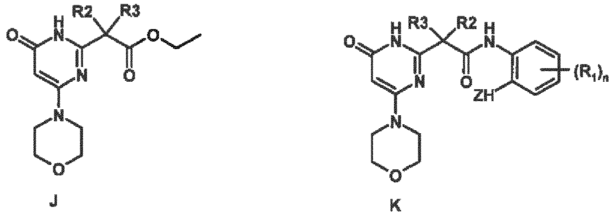 in which R1, R2, R3 and Z have the definition indicated in either one of Claims 1 and 2.
